# EUROPEAN PATENT APPLICATION

(11) **EP 4 768 488 A1**
(43) Date of publication of application: **01.07.2026**
(21) Application number: 25773102.6
(22) Date of filing: 21.03.2025
(51) Int. Cl.: C07D 495/04, C07D 495/14, A61K 31/4162, A61P 35/00

(54) **TRICYCLIC SCAFFOLD COMPOUND AND USE THEREOF IN PREPARATION OF ANTITUMOR DRUGS**

(30) Priority: 22.03.2024 CN 202410333913; 13.12.2024 CN 202411838135
(71) Applicant: Zhongshan Hospital, Fudan University, Shanghai 200032 (CN)
(72) Inventor: FAN, Jia, Shanghai 200032 (CN); KE, Aiwu, Shanghai 200032 (CN); GAO, Chao, Shanghai 200032 (CN)
(74) Representative: Santarelli
(86) International application number: PCT/CN2025/084112
(87) International publication number: WO 2025/195504

(57) **Abstract**

A tricyclic skeleton compound and use thereof in preparation of an antitumor drug are provided. Specifically, a compound of Formula (IA) or a pharmaceutically acceptable salt thereof is provided. The tricyclic skeleton compound can effectively inhibit the proliferation of tumor cells.

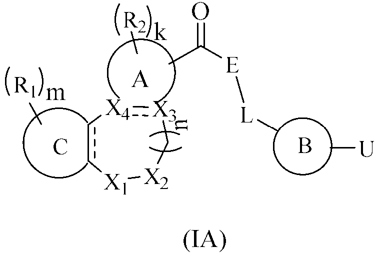

## Description

### Cross-reference To Related Applications

This application is a continuation of International Patent Application No. PCT/CN2025/084112 with a filing date of Mar. 21, 2025, designating the United States, now pending, and further claims priorities to Chinese Patent Application No. 202410333913.9 with a filing date of Mar. 22, 2024, and Chinese Patent Application No. 202411838135.5 with a filing date of Dec. 13, 2024. The contents of the aforementioned applications, including any intervening amendments thereto, are incorporated herein by reference.

### Technical Field

The present disclosure relates to a tricyclic skeleton compound and use thereof in preparation of an antitumor drug.

### Background of the Invention

Cancer remains an ongoing global public health challenge and is the second most common cause of death worldwide. Cancer is anticipated to be the most significant obstacle to increasing life expectancy in the 21st century. The burden of cancer is gradually increasing globally. In China, cancer is the leading cause of death among residents, and the burden of malignant tumors is on the rise. Cancer is also a major disease in China affecting the probability of premature death from key chronic illnesses and overall life expectancy. Therefore, identifying effective diagnostic and therapeutic targets is crucial for achieving breakthroughs in cancer treatment.

With advancements in research, human understanding of cancer has deepened. Since the 21st century, 14 hallmark characteristics of cancer have been summarized. Numerous related signaling pathways or metabolic pathways have been studied, leading to the discovery of relevant targets and the development of novel drugs.

The mitogen-activated protein kinase (MAPK) signaling pathway is a critical pathway in the initiation and progression of tumors. The MAPK pathway transmits signals through a three-tiered kinase phosphorylation model, such as Raf-mitogen extracellular kinase (MEK)-extracellular signal-regulated kinase (ERK). The kinase suppressor of Ras (KSR) family proteins are important scaffold proteins in the MAPK pathway and play a positive regulatory role in this signaling pathway. KSR2, a member of this family, acts as a scaffold protein, binding to RAF, MEK, and ERK to form complexes and participating in the regulation of the MAPK signaling pathway. Crystallographic studies of the KSR2 kinase domain have revealed that KSR2 is a key molecule for the phosphorylation of MEK by Raf (reference: BRENNAN D F, DAR A C, HERTZ N T, et al. A Raf-induced allosteric transition of KSR stimulates phosphorylation of MEK [J]. Nature, 2011, 472(7343): 366-9). KSR2 can directly phosphorylate adenosine 5'-monophosphate-activated protein kinase (AMPK), regulating metabolic processes including AMPK (as an energy receptor)-mediated glucose uptake, fatty acid oxidation, and insulin sensitivity (reference: COSTANZO-GARVEY D L, PFLUGER P T, DOUGHERTY M K, et al. KSR2 is an essential regulator of AMP kinase, energy expenditure, and insulin sensitivity [J]. Cell Metab, 2009, 10(5): 366-78). The cancer-promoting role of KSR2 has been confirmed at the cellular, molecular, and animal levels (reference: FERNANDEZ M R, HENRY M D, LEWIS R E. Kinase suppressor of Ras 2 (KSR2) regulates tumor cell transformation via AMPK [J]. Mol Cell Biol, 2012, 32(18): 3718-31, GAO C, WANG S W, LU J C, et al. KSR2-14-3-3 zeta complex serves as a biomarker and potential therapeutic target in sorafenib-resistant hepatocellular carcinoma [J]. Biomark Res, 2022, 10(1): 25). Key mechanisms are also elucidated in activating AMPK to participate in oxidative phosphorylation and in acquiring stemness to achieve targeted drug resistance. Currently, no KSR2 inhibitors have entered clinical studies. Based on this, small-molecule inhibitors of the KSR2-AMPK pathway are developed for the treatment of tumors associated with KSR2 protein.

### Summary of the Invention

A technical problem the present disclosure aims to solve is the limited range of existing targeted cancer therapies. An objective of the present disclosure is to provide a tricyclic skeleton compound and use thereof in preparation of an antitumor drug. This tricyclic skeleton compound can effectively inhibit the proliferation of tumor cells.

The present disclosure provides a compound of Formula (IA) or a pharmaceutically acceptable salt thereof, 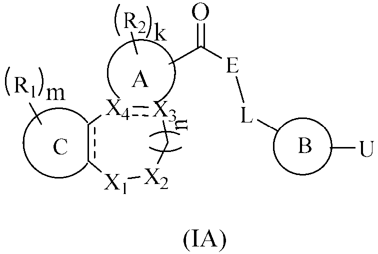
where ring C is selected from the group consisting of a benzene ring and "a 5- to 6-membered heteroaromatic ring having 1, 2, or 3 heteroatoms being 1, 2, or 3 selected from the group consisting of N, O, and S";
R₁ is selected from the group consisting of halogen, cyano, C₁₋₆ alkyl, C₃₋₁₀ cycloalkyl, C₁₋₆ alkoxy, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁;
R₁₋₁ is C₁₋₆ alkyl;
m and k are independently 0, 1, or 2;
X₁ is selected from the group consisting of -CH₂-, -O-, -S-, -NH-, and -S(=O)₂-;
X₂ is selected from the group consisting of -C(RₐR_{b})- and -O-;
Rₐ and R_{b} are independently selected from the group consisting of hydrogen, deuterium, and C₁₋₆ alkyl; alternatively, Rₐ, R_{b}, and a shared carbon atom thereof are joined together to form a 3- to 10-membered saturated carbocyclic ring;
n is 0 or 1;
"-̅ -̅ -̅" represents a single bond or a double bond;
X₃ and X₄ are independently selected from the group consisting of C and N;
ring A is a pyrazole ring;
R₂ is selected from the group consisting of H, halogen, C₁₋₆ alkyl, C₃₋₁₀ cycloalkyl, "3-to 10-membered heterocycloalkyl having 1, 2, or 3 heteroatoms being 1, 2, or 3 selected from the group consisting of N, O, and S", C₆₋₁₀ aryl, C₁₋₆ alkyl substituted with one or more R₂₋₁, and "5- to 10-membered heteroaryl having 1, 2, or 3 heteroatoms being 1, 2, or 3 selected from the group consisting of N, O, and S" substituted with one or more R₂₋₂;
R₂₋₁ and R₂₋₂ are independently selected from the group consisting of halogen, hydroxy, C₁₋₆ alkyl, -N(R₂ₐR_{2b}), C₃₋₁₀ cycloalkyl, C₁₋₆ alkoxy, "3- to 10-membered heterocycloalkyl having 1, 2, or 3 heteroatoms being 1, 2, or 3 selected from the group consisting of N, O, and S", C₆₋₁₀ aryl, and "5- to 10-membered heteroaryl having 1, 2, or 3 heteroatoms being 1, 2, or 3 selected from the group consisting of N, O, and S"; R₂ₐ and R_{2b} are independently selected from the group consisting of H and C₁₋₆ alkyl;
E is selected from the group consisting of -N(R₃)- and 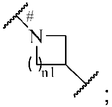 n1 is 1, 2, or 3; # represents an end linked to -C(O)- in Formula (IA);
R₃ is selected from the group consisting of hydrogen, C₁₋₆ alkyl, and C₃₋₁₀ cycloalkyl;
L is selected from the group consisting of C₁₋₆ alkylene, C₁₋₆ alkylene substituted with one or more R_{L}, "-(C₁₋₆ alkylene) -L_{A}-*", and -L_{A}-;
R_{L} is selected from the group consisting of deuterium, C₁₋₆ alkoxy, C₃₋₁₀ cycloalkyl, hydroxy, halogen, and oxo (=O); alternatively, when two R_{L} substituents are on a shared carbon atom, the two R_{L} substituents and the shared carbon atom are joined together to form a 3- to 10-membered saturated carbocyclic ring or "a 3- to 10-membered saturated heterocyclic ring having 1, 2, or 3 heteroatoms being 1, 2, or 3 selected from the group consisting of N, O, and S";
L_{A} is selected from the group consisting of -C(O)NH-, -O-, -N(R_{LA})-, vinylene, ethynylene, a 3- to 10-membered saturated carbocyclic ring, and "a 3- to 10-membered saturated heterocyclic ring having 1, 2, or 3 heteroatoms being 1, 2, or 3 selected from the group consisting of N, O, and S";
R_{LA} is selected from the group consisting of H, C₁₋₆ alkyl, C₃₋₁₀ cycloalkyl, and "3- to 10-membered heterocycloalkyl having 1, 2, or 3 heteroatoms being 1, 2, or 3 selected from the group consisting of N, O, and S";
ring B is selected from the group consisting of a 3- to 10-membered saturated or unsaturated carbocyclic ring, a 3- to 10-membered saturated or unsaturated carbocyclic ring substituted with one or more R_{B-1}, "a 3- to 10-membered saturated or unsaturated heterocyclic ring having 1, 2, or 3 heteroatoms being 1, 2, or 3 selected from the group consisting of N, O, and S", and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-1} and R_{B-2} are independently selected from the group consisting of hydroxy, halogen, oxo (=O), C₁₋₆ alkyl, C₁₋₆ alkoxy, and C₁₋₆ alkyl substituted with one or more hydroxy groups; when U is absent, the ring B is 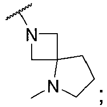
U is absent or selected from the group consisting of -N(R')(R"), C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", "5- to 10-membered heteroaryl having 1, 2, or 3 heteroatoms being 1, 2, or 3 selected from the group consisting of N, O, and S" substituted with one or more R_{U-5}, and
-X-R_{U-6};
X is selected from the group consisting of -C(O)-, -SO₂-, and -O-;
R' and R" are independently selected from the group consisting of hydrogen, C₁₋₆ alkyl,
-C(O)-C₃₋₁₀ cycloalkyl, and C₁₋₆ alkyl substituted with one or more R‴; R‴ is C₆₋₁₀ aryl; R_{U-1} is selected from the group consisting of -N(R')(R"), halogen, C₃₋₁₀ cycloalkyl, C₆₋₁₀ aryl, "5- to 10-membered heteroaryl having 1, 2, or 3 heteroatoms being 1, 2, or 3 selected from the group consisting of N, O, and S", or "5- to 10-membered heteroaryl having 1, 2, or 3 heteroatoms being 1, 2, or 3 selected from the group consisting of N, O, and S" substituted with one or more R_{U-1-1};
R_{U-1-1} is C₁₋₆ alkyl;
R_{U-2}, R_{U-3}, R_{U-4}, and R_{U-5} are independently selected from the group consisting of halogen, cyano, hydroxy, -SO₂-C₁₋₆ alkyl, -C(O)-C₁₋₆ alkyl, -C(O)O-C₁₋₆ alkyl, -C(O)NH-C₁₋₆ alkyl, C₁₋₆ alkyl, C₁₋₆ alkyl substituted with one or more R_{U-3-1}, C₁₋₆ alkoxy, C₁₋₆ alkoxy substituted with one or more R_{U-3-2}, C₃₋₁₀ cycloalkyl, "3- to 10-membered heterocycloalkyl having 1, 2, or 3 heteroatoms being 1, 2, or 3 selected from the group consisting of N, O, and S", and -O-"3- to 10-membered heterocycloalkyl having 1, 2, or 3 heteroatoms being 1, 2, or 3 selected from the group consisting of N, O, and S"; R_{U-3-1} and R_{U-3-2} are independently selected from the group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S";
R_{U-6} is selected from the group consisting of C₆₋₁₀ aryl and "5- to 10-membered heteroaryl having 1, 2, or 3 heteroatoms being 1, 2, or 3 selected from the group consisting of N, O, and S"; and
a compound of Formula (I) is not any one selected from the group consisting of following compounds:

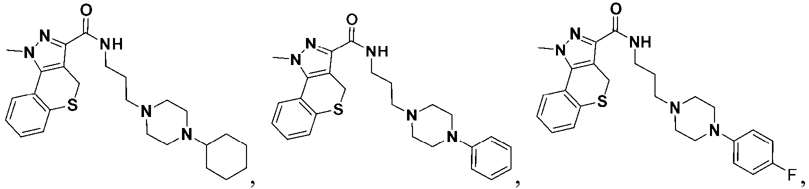

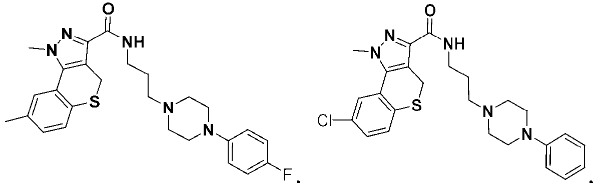

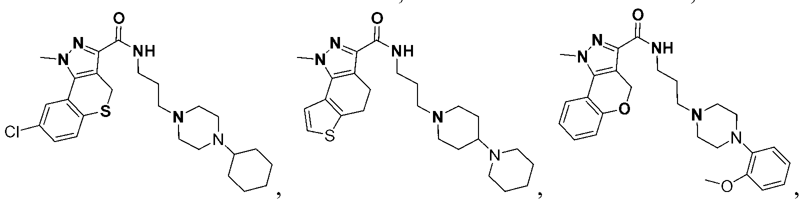

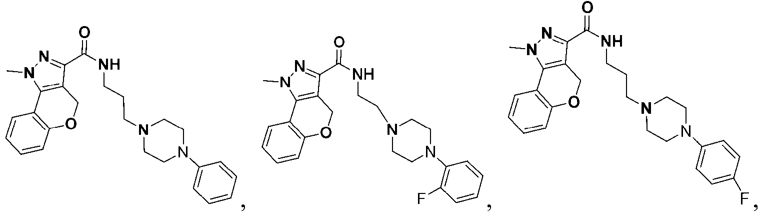

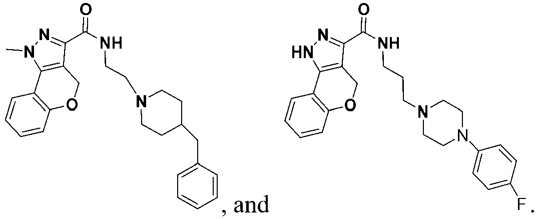

The present disclosure provides a compound of Formula (I) or a pharmaceutically acceptable salt thereof, 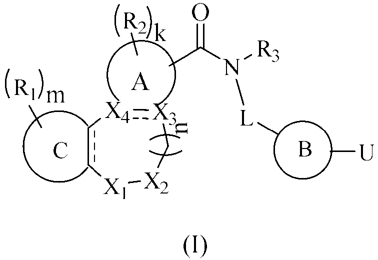
where ring C is selected from the group consisting of a benzene ring and "a 5- to 6-membered heteroaromatic ring having 1, 2, or 3 heteroatoms being 1, 2, or 3 selected from the group consisting of N, O, and S";
R₁ is selected from the group consisting of halogen, cyano, C₁₋₆ alkyl, C₃₋₁₀ cycloalkyl, C₁₋₆ alkoxy, C₁₋₆ alkyl substituted with one or more halogens, and C₁₋₆ alkoxy substituted with one or more halogens;
m and k are independently 0, 1, or 2;
X₁ is selected from the group consisting of -CH₂-, -O-, -S-, -NH-, and -S(=O)₂-;
X₂ is selected from the group consisting of -C(RₐR_{b})- and -O-;
Rₐ and R_{b} are independently selected from the group consisting of hydrogen, deuterium, and C₁₋₆ alkyl; alternatively, Rₐ, R_{b}, and a shared carbon atom thereof are joined together to form a 3- to 10-membered saturated carbocyclic ring;
n is 0 or 1;
"-̅ -̅ -̅" represents a single bond or a double bond;
X₃ and X₄ are independently selected from the group consisting of C and N;
ring A is a pyrazole ring;
R₂ is selected from the group consisting of halogen, C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", C₆₋₁₀ aryl, C₁₋₆ alkyl substituted with one or more R₂₋₁, and "5- to 10-membered heteroaryl having 1, 2, or 3 heteroatoms being 1, 2, or 3 selected from the group consisting of N, O, and S" substituted with one or more R₂₋₂;
R₂₋₁ and R₂₋₂ are independently selected from the group consisting of halogen, hydroxy, C₁₋₆ alkyl, -N(R₂ₐR_{2b}), C₃₋₁₀ cycloalkyl, C₁₋₆ alkoxy, "3- to 10-membered heterocycloalkyl having 1, 2, or 3 heteroatoms being 1, 2, or 3 selected from the group consisting of N, O, and S", C₆₋₁₀ aryl, and "5- to 10-membered heteroaryl having 1, 2, or 3 heteroatoms being 1, 2, or 3 selected from the group consisting of N, O, and S"; R₂ₐ and R_{2b} are independently selected from the group consisting of H and C₁₋₆ alkyl; R₃ is selected from the group consisting of hydrogen, C₁₋₆ alkyl, and C₃₋₁₀ cycloalkyl;

L is selected from the group consisting of C₁₋₆ alkylene, C₁₋₆ alkylene substituted with one or more R_{L}, "-(C₁₋₆ alkylene) -L_{A}-*", and -L_{A}- (where * represents an end linked to the ring B);
R_{L} is selected from the group consisting of deuterium, C₁₋₆ alkoxy, C₃₋₁₀ cycloalkyl, hydroxy, halogen, and oxo (=O); alternatively, when two R_{L} substituents are on a shared carbon atom, the two R_{L} substituents and the shared carbon atom are joined together to form a 3- to 10-membered saturated carbocyclic ring or "a 3- to 10-membered saturated heterocyclic ring having 1, 2, or 3 heteroatoms being 1, 2, or 3 selected from the group consisting of N, O, and S";
L_{A} is selected from the group consisting of -C(O)NH-, -O-, -N(R_{LA})-, vinylene, ethynylene, a 3- to 10-membered saturated carbocyclic ring, and "a 3- to 10-membered saturated heterocyclic ring having 1, 2, or 3 heteroatoms being 1, 2, or 3 selected from the group consisting of N, O, and S";
R_{LA} is selected from the group consisting of H, C₁₋₆ alkyl, C₃₋₁₀ cycloalkyl, and "3- to 10-membered heterocycloalkyl having 1, 2, or 3 heteroatoms being 1, 2, or 3 selected from the group consisting of N, O, and S";
ring B is selected from the group consisting of a 3- to 10-membered saturated or unsaturated carbocyclic ring, a 3- to 10-membered saturated or unsaturated carbocyclic ring substituted with one or more R_{B-1}, "a 3- to 10-membered saturated or unsaturated heterocyclic ring having 1, 2, or 3 heteroatoms being 1, 2, or 3 selected from the group consisting of N, O, and S", and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-1} and R_{B-2} are independently selected from the group consisting of hydroxy, halogen, oxo (=O), C₁₋₆ alkyl, C₁₋₆ alkoxy, and C₁₋₆ alkyl substituted with one or more hydroxy groups;
U is selected from the group consisting of C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", "5- to 10-membered heteroaryl having 1, 2, or 3 heteroatoms being 1, 2, or 3 selected from the group consisting of N, O, and S" substituted with one or more R_{U-5}, and -X-R_{U-6};
X is selected from the group consisting of -C(O)-, -SO₂-, and -O-;
R_{U-1} is selected from the group consisting of halogen, C₃₋₁₀ cycloalkyl, C₆₋₁₀ aryl, "5- to 10-membered heteroaryl having 1, 2, or 3 heteroatoms being 1, 2, or 3 selected from the group consisting of N, O, and S", or "5- to 10-membered heteroaryl having 1, 2, or 3 heteroatoms being 1, 2, or 3 selected from the group consisting of N, O, and S" substituted with one or more R_{U-1-1},
R_{U-1-1} is C₁₋₆ alkyl;
R_{U-2}, R_{U-3}, R_{U-4}, and R_{U-5} are independently selected from the group consisting of halogen, cyano, hydroxy, -SO₂-C₁₋₆ alkyl, -C(O)O-C₁₋₆ alkyl, -C(O)NH-C₁₋₆ alkyl, C₁₋₆ alkyl, C₁₋₆ alkyl substituted with one or more R_{U-3-1}, C₁₋₆ alkoxy, C₁₋₆ alkoxy substituted with one or more R_{U-3-2}, C₃₋₁₀ cycloalkyl, "3- to 10-membered heterocycloalkyl having 1, 2, or 3 heteroatoms being 1, 2, or 3 selected from the group consisting of N, O, and S", and -O-"3- to 10-membered heterocycloalkyl having 1, 2, or 3 heteroatoms being 1, 2, or 3 selected from the group consisting of N, O, and S";
R_{U-3-1} and R_{U-3-2} are independently selected from the group consisting of halogen, C₃₋₁₀ cycloalkyl, cyano;
R_{U-6} is selected from the group consisting of C₆₋₁₀ aryl and "5- to 10-membered heteroaryl having 1, 2, or 3 heteroatoms being 1, 2, or 3 selected from the group consisting of N, O, and S"; and
a compound of Formula (I) is not any one selected from the group consisting of following compounds:

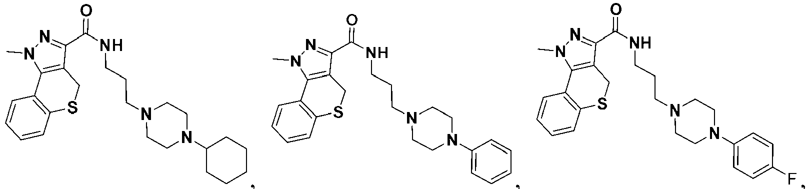

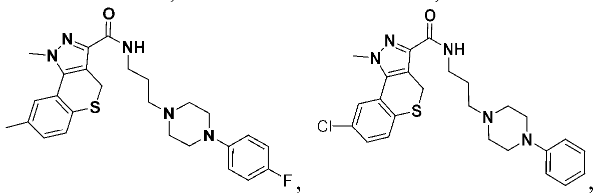

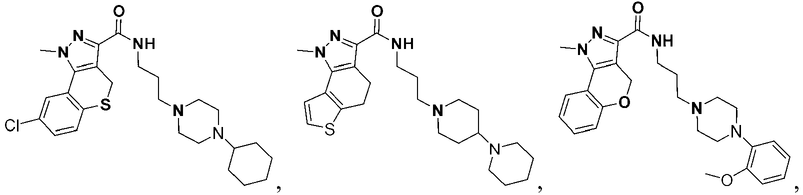

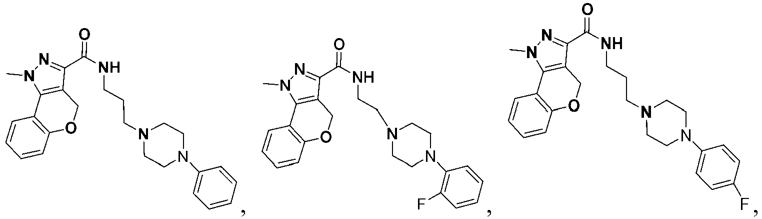

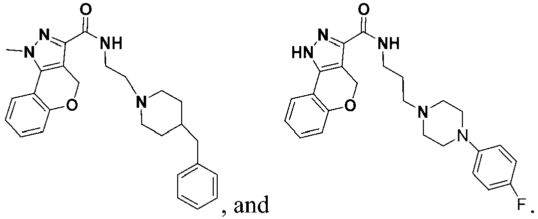

In some embodiments, the compound of Formula (IA) is a compound of Formula (IIA): 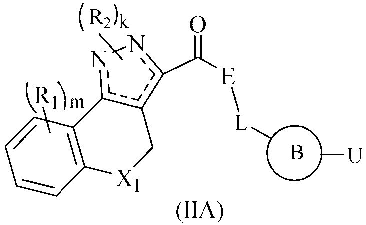
R₁ is selected from the group consisting of halogen, cyano, C₁₋₆ alkyl, C₃₋₁₀ cycloalkyl, C₁₋₆ alkoxy, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁;
R₁₋₁ is C₁₋₆ alkyl;
m and k are independently 0, 1, or 2;
X₁ is selected from the group consisting of -CH₂-, -O-, -S-, and -S(=O)₂-;
R₂ is selected from the group consisting of H, C₁₋₆ alkyl, C₁₋₆ alkyl substituted with one or more R₂₋₁, and "3- to 10-membered heterocycloalkyl having 1, 2, or 3 heteroatoms being 1, 2, or 3 selected from the group consisting of N, O, and S";
R₂₋₁ is selected from the group consisting of hydroxy and C₃₋₁₀ cycloalkyl;
"-̅ -̅ -̅" represents a single bond or a double bond;
E is selected from the group consisting of -N(R₃)- and 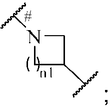 n1 is 1, 2, or 3; # represents an end linked to -C(O)- in Formula (IA);
R₃ is selected from the group consisting of hydrogen and C₁₋₆ alkyl;
L is selected from the group consisting of C₁₋₆ alkylene, C₁₋₆ alkylene substituted with one or more R_{L}, and "-(C₁₋₆ alkylene) -L_{A}-*";
R_{L} is selected from the group consisting of hydroxy and halogen; alternatively, when two R_{L} substituents are on a shared carbon atom, the two R_{L} substituents and the shared carbon atom are joined together to form a 3- to 10-membered saturated carbocyclic ring;
L_{A} is selected from the group consisting of -NH-, -N(C₁₋₆ alkyl)-, and a 3- to 10-membered saturated carbocyclic ring;
ring B is selected from the group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is selected from the group consisting of hydroxy, halogen, C₁₋₆ alkyl, C₁₋₆ alkoxy, and C₁₋₆ alkyl substituted with one or more hydroxy groups;
when U is absent, the ring B is 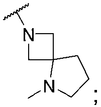
U is absent or selected from the group consisting of -N(R')(R"), C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R_{U-5}; R' and R" are independently selected from the group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl; R_{U-1} is selected from the group consisting of -N(R')(R"), C₃₋₁₀ cycloalkyl, and C₆₋₁₀ aryl; R_{U-2}, R_{U-3}, R_{U-4}, and R_{U-5} are independently selected from the group consisting of halogen, -SO₂-C₁₋₆ alkyl, -C(O)-C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", cyano, hydroxy, C₁₋₆ alkyl, C₁₋₆ alkyl substituted with one or more R_{U-3-1}, C₁₋₆ alkoxy, and C₁₋₆ alkoxy substituted with one or more R_{U-3-2}; and
R_{U-3-1} and R_{U-3-2} are independently selected from the group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S".

In some embodiments, the compound of Formula (I) is a compound of Formula (II): 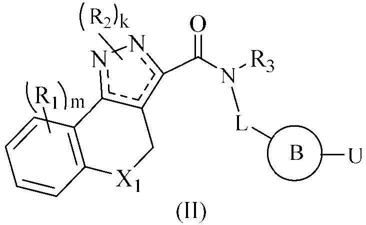
R₁ is halogen;
m and k are independently 0, 1, or 2;
X₁ is selected from the group consisting of -CH₂-, -O-, -S-, and -S(=O)₂-;
R₂ is selected from the group consisting of C₁₋₆ alkyl and C₁₋₆ alkyl substituted with one or more R₂₋₁;
R₂₋₁ is C₃₋₁₀ cycloalkyl;
"-̅ -̅ -̅" represents a single bond or a double bond;
R₃ is selected from the group consisting of hydrogen and C₁₋₆ alkyl;
L is selected from the group consisting of C₁₋₆ alkylene, C₁₋₆ alkylene substituted with one or more R_{L}, and "-(C₁₋₆ alkylene) -L_{A}-*";
R_{L} is selected from the group consisting of hydroxy and halogen; alternatively, when two R_{L} substituents are on a shared carbon atom, the two R_{L} substituents and the shared carbon atom are joined together to form a 3- to 10-membered saturated carbocyclic ring;
L_{A} is selected from the group consisting of -NH- and -N(C₁₋₆ alkyl)-;
ring B is selected from the group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is selected from the group consisting of hydroxy, halogen, C₁₋₆ alkoxy, and C₁₋₆ alkyl substituted with one or more hydroxy groups; and
U is selected from the group consisting of C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R_{U-5};
R_{U-1} is selected from the group consisting of C₃₋₁₀ cycloalkyl and C₆₋₁₀ aryl;
R_{U-2}, R_{U-3}, R_{U-4}, and R_{U-5} are independently selected from the group consisting of halogen, C₃₋₁₀ cycloalkyl, "3- to 10-membered heterocycloalkyl having 1, 2, or 3 heteroatoms being 1, 2, or 3 selected from the group consisting of N, O, and S", cyano, hydroxy, C₁₋₆ alkyl, C₁₋₆ alkyl substituted with one or more R_{U-3-1}, C₁₋₆ alkoxy, and C₁₋₆ alkoxy substituted with one or more R_{U-3-2}; and
R_{U-3-1} and R_{U-3-2} are independently selected from the group consisting of halogen and C₃₋₁₀ cycloalkyl.

In some embodiments, the compound of Formula (IA) is selected from the group consisting of a compound of Formula (IIIA) and a compound of Formula (IIIB): 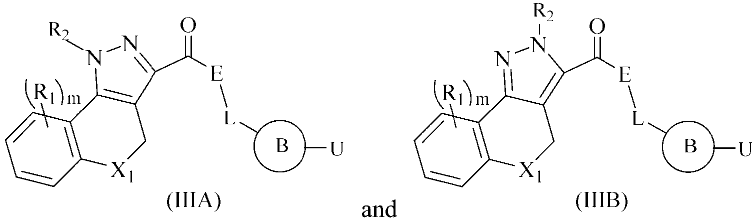
R₁ is selected from the group consisting of halogen, cyano, C₁₋₆ alkyl, C₃₋₁₀ cycloalkyl, C₁₋₆ alkoxy, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁;
R₁₋₁ is C₁₋₆ alkyl;
m is 0, 1, or 2.
X₁ is selected from the group consisting of -CH₂-, -O-, -S-, and -S(=O)₂-;
R₂ is selected from the group consisting of H, C₁₋₆ alkyl, C₁₋₆ alkyl substituted with one or more R₂₋₁, and "3- to 10-membered heterocycloalkyl having 1, 2, or 3 heteroatoms being 1, 2, or 3 selected from the group consisting of N, O, and S";
R₂₋₁ is selected from the group consisting of hydroxy and C₃₋₁₀ cycloalkyl;
E is selected from the group consisting of -N(R₃)- and 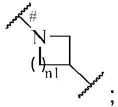 n1 is 1, 2, or 3; # represents an end linked to -C(O)- in Formula (IA);
R₃ is selected from the group consisting of hydrogen and C₁₋₆ alkyl;
L is selected from the group consisting of C₁₋₆ alkylene, C₁₋₆ alkylene substituted with one or more R_{L}, and "-(C₁₋₆ alkylene) -L_{A}-*";
R_{L} is selected from the group consisting of hydroxy and halogen; alternatively, when two R_{L} substituents are on a shared carbon atom, the two R_{L} substituents and the shared carbon atom are joined together to form a 3- to 10-membered saturated carbocyclic ring;
L_{A} is a 3- to 10-membered saturated carbocyclic ring;
ring B is selected from the group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is selected from the group consisting of halogen and C₁₋₆ alkyl;
when U is absent, the ring B is 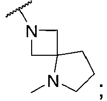
U is absent or selected from the group consisting of -N(R')(R"), C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, and "5- to 10-membered heteroaryl having 1, 2, or 3 heteroatoms being 1, 2, or 3 selected from the group consisting of N, O, and S";
R' and R" are independently selected from the group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl;
R_{U-1} is selected from the group consisting of C₃₋₁₀ cycloalkyl and C₆₋₁₀ aryl;
R_{U-2}, R_{U-3}, and R_{U-4} are independently selected from the group consisting of halogen, hydroxy, -SO₂-C₁₋₆ alkyl, -C(O)-C₁₋₆ alkyl, C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", C₁₋₆ alkoxy, and C₁₋₆ alkyl substituted with one or more R_{U-3-1}; and
R_{U-3-1} is selected from the group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S".

In some embodiments, the compound of Formula (I) is a compound of Formula (III): 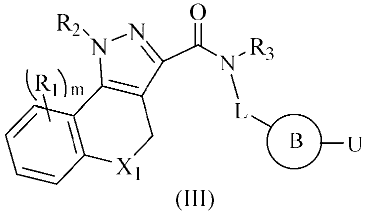
R₁ is halogen;
m is 0 or 1;
X₁ is selected from the group consisting of -CH₂-, -O-, -S-, and -S(=O)₂-;
R₂ is selected from the group consisting of C₁₋₆ alkyl and C₁₋₆ alkyl substituted with one or more R₂₋₁;
R₂₋₁ is C₃₋₁₀ cycloalkyl;
R₃ is selected from the group consisting of hydrogen and C₁₋₆ alkyl;
L is selected from the group consisting of C₁₋₆ alkylene and C₁₋₆ alkylene substituted with one or more R_{L};
R_{L} is selected from the group consisting of hydroxy and halogen;
ring B is selected from the group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is halogen;
U is selected from the group consisting of C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", C₆₋₁₀ aryl, C₆₋₁₀ aryl substituted with one or more R_{U-4}, and "5- to 10-membered heteroaryl having 1, 2, or 3 heteroatoms being 1, 2, or 3 selected from the group consisting of N, O, and S";
R_{U-1} is selected from the group consisting of C₃₋₁₀ cycloalkyl and C₆₋₁₀ aryl;
R_{U-2} and R_{U-4} are independently selected from the group consisting of halogen, C₁₋₆ alkoxy, and C₁₋₆ alkyl substituted with one or more R_{U-3-1}; and
R_{U-3-1} is halogen.

In some embodiments, R₁ is selected from the group consisting of halogen, cyano, C₁₋₆ alkyl, C₃₋₁₀ cycloalkyl, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁; R₁₋₁ is C₁₋₆ alkyl.

In some embodiments, R₁ is halogen.

In some embodiments, m and k are independently 0 or 1.

In some embodiments, R₂ is selected from the group consisting of H, C₁₋₆ alkyl, C₁₋₆ alkyl substituted with one or more R₂₋₁, and "3- to 10-membered heterocycloalkyl having 1, 2, or 3 heteroatoms being 1, 2, or 3 selected from the group consisting of N, O, and S"; and R₂₋₁ is selected from the group consisting of hydroxy and C₃₋₁₀ cycloalkyl.

In some embodiments, R₂ is selected from the group consisting of C₁₋₆ alkyl and C₁₋₆ alkyl substituted with one or more R₂₋₁; and R₂₋₁ is C₃₋₁₀ cycloalkyl.

In some embodiments, E is -N(R₃)-.

In some embodiments, R₃ is selected from the group consisting of hydrogen and C₁₋₆ alkyl.

In some embodiments, R₃ is halogen.

In some embodiments, L is selected from the group consisting of C₁₋₆ alkylene, C₁₋₆ alkylene substituted with one or more R_{L}, and "-(C₁₋₆ alkylene) -L_{A}-*";
R_{L} is selected from the group consisting of hydroxy and halogen; alternatively, when two R_{L} substituents are on a shared carbon atom, the two R_{L} substituents and the shared carbon atom are joined together to form a 3- to 10-membered saturated carbocyclic ring; and
L_{A} is selected from the group consisting of -NH-, -N(C₁₋₆ alkyl)-, and a 3- to 10-membered saturated carbocyclic ring, preferably the 3- to 10-membered saturated carbocyclic ring.

In some embodiments, L is selected from the group consisting of C₁₋₆ alkylene, C₁₋₆ alkylene substituted with one or more R_{L}, and "-(C₁₋₆ alkylene) -L_{A}-*";
R_{L} is selected from the group consisting of hydroxy and halogen; alternatively, when two R_{L} substituents are on a shared carbon atom, the two R_{L} substituents and the shared carbon atom are joined together to form a 3- to 10-membered saturated carbocyclic ring; and
L_{A} is selected from the group consisting of -NH- and -N(C₁₋₆ alkyl)-.

In some embodiments, L is selected from the group consisting of C₁₋₆ alkylene, C₁₋₆ alkylene substituted with one or more R_{L}, and "-(C₁₋₆ alkylene) -L_{A}-*";
R_{L} is selected from the group consisting of hydroxy and halogen; alternatively, when two R_{L} substituents are on a shared carbon atom, the two R_{L} substituents and the shared carbon atom are joined together to form a 3- to 10-membered saturated carbocyclic ring; and L_{A} is -NH-.

In some embodiments, L is selected from the group consisting of C₁₋₆ alkylene and C₁₋₆ alkylene substituted with one or more R_{L}; and R_{L} is selected from the group consisting of hydroxy and halogen.

In some embodiments, L is selected from the group consisting of C₁₋₆ alkylene and C₁₋₆ alkylene substituted with one or more R_{L}; and R_{L} is hydroxy.

In some embodiments, ring B is selected from the group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2}; and R_{B-2} is selected from the group consisting of hydroxy, halogen, C₁₋₆ alkyl, C₁₋₆ alkoxy, and C₁₋₆ alkyl substituted with one or more hydroxy groups, preferably the halogen or the C₁₋₆ alkyl.

In some embodiments, ring B is selected from the group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2}; and R_{B-2} is selected from the group consisting of hydroxy, halogen, C₁₋₆ alkoxy, and C₁₋₆ alkyl substituted with one or more hydroxy groups, preferably the halogen.

In some embodiments, the ring B is "a 3- to 10-membered saturated or unsaturated heterocyclic ring having 1, 2, or 3 heteroatoms being 1, 2, or 3 selected from the group consisting of N, O, and S".

In some embodiments, U is absent or selected from the group consisting of -N(R')(R"), C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R_{U-5};
R' and R" are independently selected from the group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl; R_{U-1} is selected from the group consisting of -N(R')(R"), C₃₋₁₀ cycloalkyl, and C₆₋₁₀ aryl; R_{U-2}, R_{U-3}, R_{U-4}, and R_{U-5} are independently selected from the group consisting of halogen, -SO₂-C₁₋₆ alkyl, -C(O)-C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", cyano, hydroxy, C₁₋₆ alkyl, C₁₋₆ alkyl substituted with one or more R_{U-3-1}, C₁₋₆ alkoxy, and C₁₋₆ alkoxy substituted with one or more R_{U-3-2}; and
R_{U-3-1} and R_{U-3-2} are independently selected from the group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S".

In some embodiments, U is absent or selected from the group consisting of -N(R')(R"), C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3-to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, and "5- to 10-membered heteroaryl having 1, 2, or 3 heteroatoms being 1, 2, or 3 selected from the group consisting of N, O, and S";
R' and R" are independently selected from the group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl; R_{U-1} is selected from the group consisting of C₃₋₁₀ cycloalkyl and C₆₋₁₀ aryl;
R_{U-2}, R_{U-3}, and R_{U-4} are independently selected from the group consisting of halogen, hydroxy, -SO₂-C₁₋₆ alkyl, -C(O)-C₁₋₆ alkyl, C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", C₁₋₆ alkoxy, and C₁₋₆ alkyl substituted with one or more R_{U-3-1}; and
R_{U-3-1} is selected from the group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S".

In some embodiments, U is selected from the group consisting of C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3-to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R_{U-5};
R_{U-1} is selected from the group consisting of C₃₋₁₀ cycloalkyl and C₆₋₁₀ aryl;
R_{U-2}, R_{U-3}, R_{U-4}, and R_{U-5} are independently selected from the group consisting of halogen, C₃₋₁₀ cycloalkyl, "3- to 10-membered heterocycloalkyl having 1, 2, or 3 heteroatoms being 1, 2, or 3 selected from the group consisting of N, O, and S", cyano, hydroxy, C₁₋₆ alkyl, C₁₋₆ alkyl substituted with one or more R_{U-3-1}, C₁₋₆ alkoxy, and C₁₋₆ alkoxy substituted with one or more R_{U-3-2}; and
R_{U-3-1} and R_{U-3-2} are independently selected from the group consisting of halogen and C₃₋₁₀ cycloalkyl.

In some embodiments, U is selected from the group consisting of C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", C₆₋₁₀ aryl, C₆₋₁₀ aryl substituted with one or more R_{U-4}, and "5- to 10-membered heteroaryl having 1, 2, or 3 heteroatoms being 1, 2, or 3 selected from the group consisting of N, O, and S";
R_{U-1} is selected from the group consisting of C₃₋₁₀ cycloalkyl and C₆₋₁₀ aryl;
R_{U-2} and R_{U-4} are independently selected from the group consisting of halogen, C₁₋₆ alkoxy, and C₁₋₆ alkyl substituted with one or more R_{U-3-1}; and
R_{U-3-1} is halogen.

In some embodiments, in the ring C, the "5- to 6-membered heteroaromatic ring having 1, 2, or 3 heteroatoms being 1, 2, or 3 selected from the group consisting of N, O, and S" is "a 5- to 6-membered heteroaromatic ring having 1 or 2 heteroatoms being N", such as a pyridine ring or pyrimidine ring.

In some embodiments, the "C₁₋₆ alkyl" is C₁₋₄ alkyl, and is selected from the group consisting of methyl, ethyl, n-propyl, isopropyl, n-butyl, tert-butyl, isobutyl, and sec-butyl, preferably the methyl or ethyl.

In some embodiments, the "C₁₋₆ alkoxy" is C₁₋₄ alkoxy, and is selected from the group consisting of methoxy, ethoxy, n-propoxy, isopropoxy, n-butoxy, tert-butoxy, isobutoxy, and sec-butoxy, preferably the methoxy.

In some embodiments, the "halogen" is selected from the group consisting of fluorine, chlorine, bromine, and iodine, preferably the fluorine or chlorine.

In some embodiments, the "halogen" is selected from the group consisting of fluorine, chlorine, and bromine, preferably the fluorine.

In some embodiments, when Rₐ, R_{b}, and the shared carbon atom thereof are joined together to form a 3- to 10-membered saturated carbocyclic ring, the "3- to 10-membered saturated carbocyclic ring" is a 3-membered saturated carbocyclic ring, such as 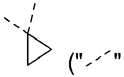 represents a bond linked to the rest of the molecule).

In some embodiments, the "C₃₋₁₀ cycloalkyl" is selected from the group consisting of C₃₋₆ monocyclic cycloalkyl and C₅₋₁₀ polycyclic cycloalkyl (including spiro cycloalkyl, fused cycloalkyl, or bridged cycloalkyl). The C₃₋₆ monocyclic cycloalkyl can be selected from the group consisting of cyclopropyl, cyclobutyl, cyclopentyl, and cyclohexyl, and the C₅₋₁₀ polycyclic cycloalkyl is preferably C₅₋₁₀ spirocyclic cycloalkyl, and more preferably

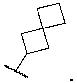

In some embodiments, the "3- to 10-membered heterocycloalkyl having 1, 2, or 3 heteroatoms being 1, 2, or 3 selected from the group consisting of N, O, and S" is selected from the group consisting of 4- to 6-membered monocyclic heterocycloalkyl and 5- to 10-membered polycyclic heterocycloalkyl (including spiro heterocycloalkyl, fused heterocycloalkyl, or bridged heterocycloalkyl). The 4- to 6-membered monocyclic heterocycloalkyl is preferably "4- to 6-membered monocyclic heterocycloalkyl having 1 or 2 heteroatoms being 1 or 2 selected from the group consisting of N and O", more preferably 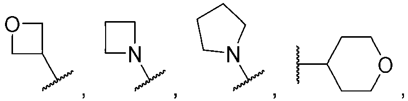 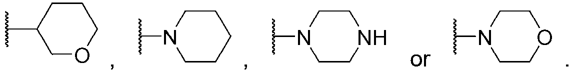 The 5- to 10-membered polycyclic heterocycloalkyl may be 5- to 10-membered spiro heterocycloalkyl, preferably 6-membered spiro 3-membered heterocycloalkyl, more preferably 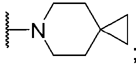 alternatively, the 5- to 10-membered polycyclic heterocycloalkyl may be a 5- to 10-membered bridged heterocycloalkyl, preferably

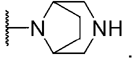

In some embodiments, the "3- to 10-membered heterocycloalkyl having 1, 2, or 3 heteroatoms being 1, 2, or 3 selected from the group consisting of N, O, and S" is selected from the group consisting of 4- to 6-membered monocyclic heterocycloalkyl and 5- to 10-membered polycyclic heterocycloalkyl (including spiro heterocycloalkyl, fused heterocycloalkyl, or bridged heterocycloalkyl), the 4- to 6-membered monocyclic heterocycloalkyl is preferably 6-membered heterocycloalkyl having 1 or 2 heteroatoms being 1 or 2 selected from the group consisting of N and O, more preferably 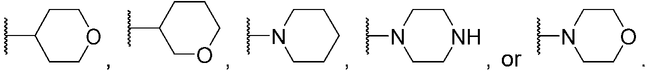 The 5- to 10-membered polycyclic heterocycloalkyl can be 5- to 10-membered spiro heterocycloalkyl, preferably 6-membered spiro 3-membered heterocycloalkyl, more preferably

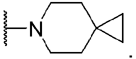

In some embodiments, the "C₆₋₁₀ aryl" is selected from the group consisting of phenyl and naphthyl, preferably the phenyl.

In some embodiments, the "5- to 10-membered heteroaryl having 1, 2, or 3 heteroatoms being 1, 2, or 3 selected from the group consisting of N, O, and S" is 5- to 6-membered monocyclic heteroaryl. The 5- to 6-membered monocyclic heteroaryl is preferably 5- to 6-membered monocyclic heteroaryl having 1, 2, or 3 heteroatoms being N, such as 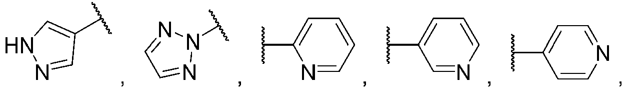 or

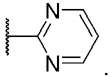

In some embodiments, the "5- to 10-membered heteroaryl having 1, 2, or 3 heteroatoms being 1, 2, or 3 selected from the group consisting of N, O, and S" is 5- to 6-membered monocyclic heteroaryl, the 5- to 6-membered monocyclic heteroaryl is preferably "5- to 6-membered monocyclic heteroaryl having 1 or 2 heteroatoms being N, more preferably

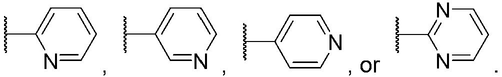

In some embodiments, the "C₁₋₆ alkylene" is C₁₋₄ alkylene, preferably methylene,

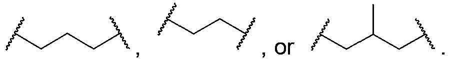

In some embodiments, the "C₁₋₆ alkylene" is C₁₋₄ alkylene, preferably

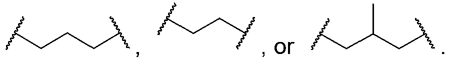

In some embodiments, when two R_{L} substituents are on a shared carbon atom, the "3- to 10-membered saturated carbocyclic ring" formed by the two R_{L} substituents and the shared carbon atom being joined together is a 3-membered saturated carbocyclic ring, such as 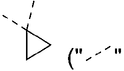 represents a bond linked to the rest of the molecule).

In some embodiments, when L_{A} is a 3- to 10-membered saturated carbocyclic ring, the "3- to 10-membered saturated carbocyclic ring" is a 4- to 6-membered saturated carbocyclic ring, including

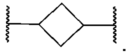

In some embodiments, the "3- to 10-membered saturated or unsaturated heterocyclic ring having 1, 2, or 3 heteroatoms being 1, 2, or 3 selected from the group consisting of N, O, and S" is "a 4- to 8-membered saturated or unsaturated heterocyclic ring having 1 or 2 heteroatoms being 1 or 2 selected from the group consisting of N and O", such as 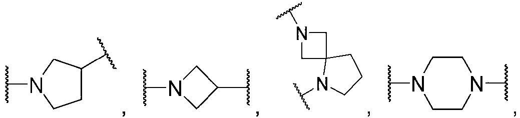 or

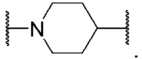

In some embodiments, the "3- to 10-membered saturated or unsaturated heterocyclic ring having 1, 2, or 3 heteroatoms being 1, 2, or 3 selected from the group consisting of N, O, and S" is "a 5- to 6-membered saturated or unsaturated heterocyclic ring having 1 or 2 heteroatoms being N", such as 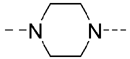 or 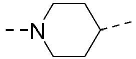 (" 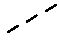 " represents a bond linked to the rest of the molecule).

In some embodiments, the ring C is a benzene ring.

In some embodiments, R₁ is selected from the group consisting of fluorine, chlorine, bromine, cyano, trifluoromethyl, cyclopropyl, trifluoromethoxy, methyl, 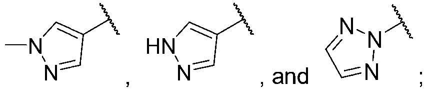 m is 0, 1, or 2.

In some embodiments, R₁ is selected from the group consisting of fluorine and chlorine; m is 0 or 1.

In some embodiments, X₁ is selected from the group consisting of -CH₂-, -O-, -S-, and -S(=O)₂-; X₂ is -CH₂-; n is 0; X₃ and X₄ are C.

In some embodiments, R₂ is selected from the group consisting of hydrogen, methyl, ethyl, 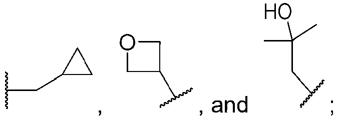 k is 0 or 1.

In some embodiments, R₂ is selected from the group consisting of methyl, ethyl, and 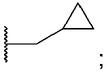 k is 1.

In some embodiments, a structural moiety 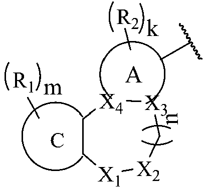 is selected from the group consisting of

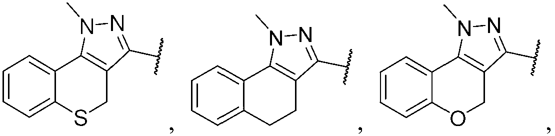

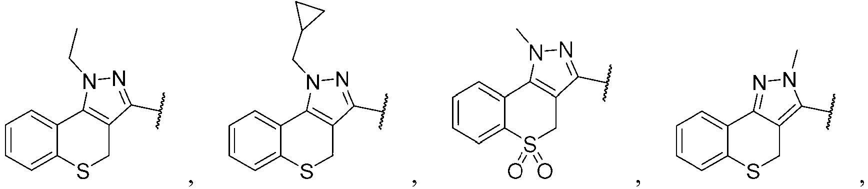

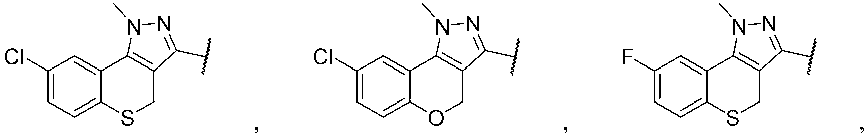

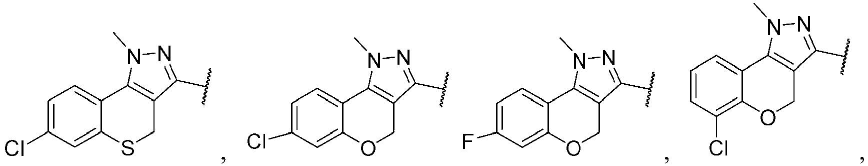

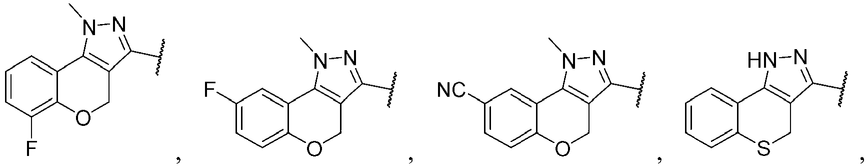

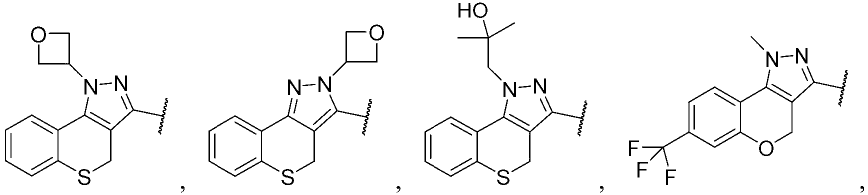

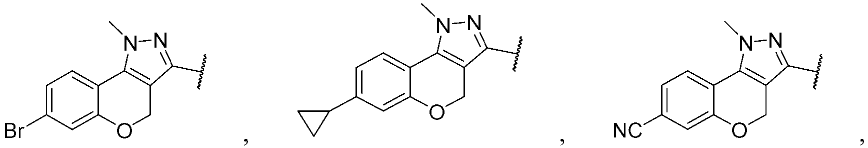

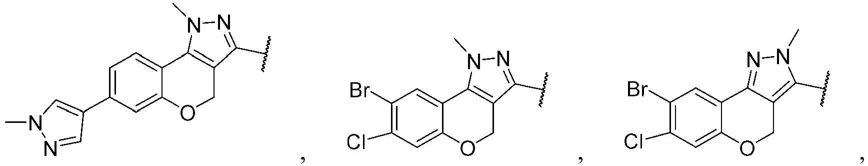

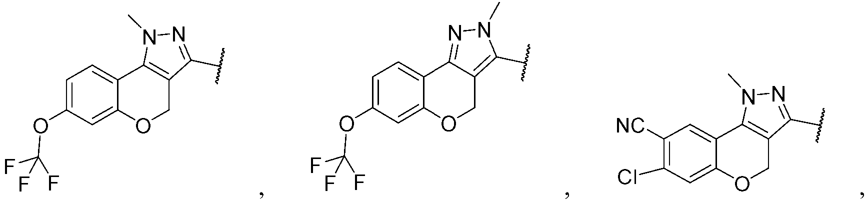

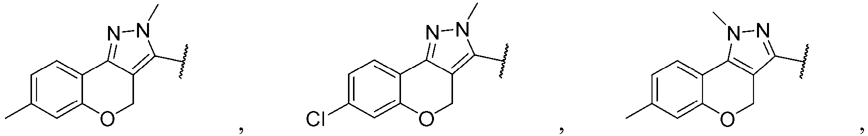

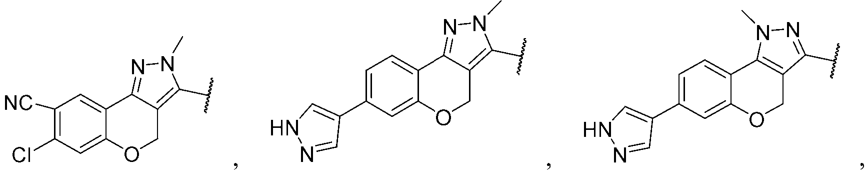

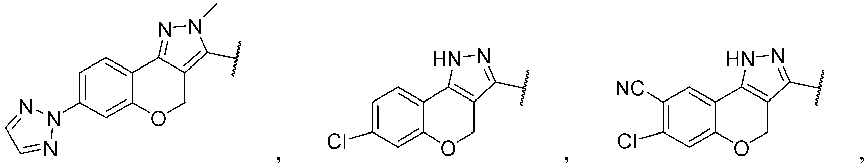

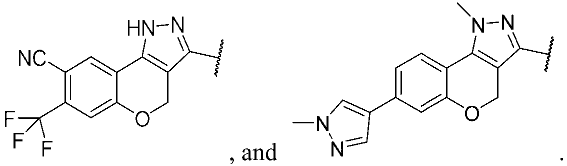

In some embodiments, R₃ is selected from the group consisting of hydrogen and methyl.

In some embodiments, L is selected from the group consisting of methylene,

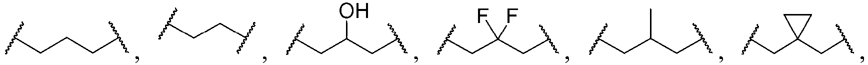

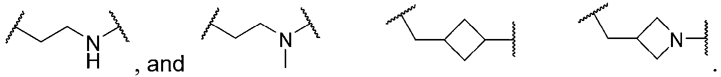

In some embodiments, the ring B is selected from the group consisting of 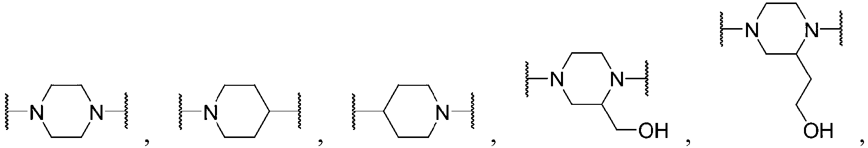 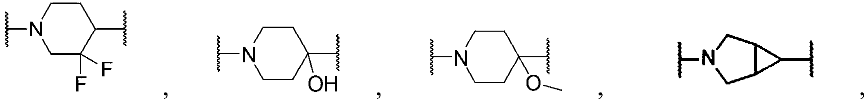 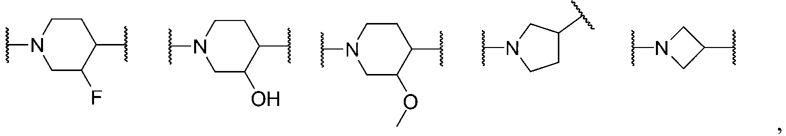 and

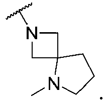

In some embodiments, U is selected from the group consisting of

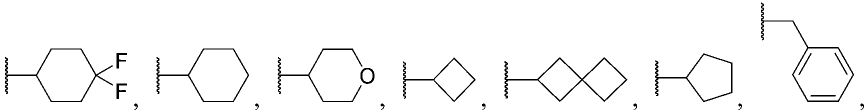

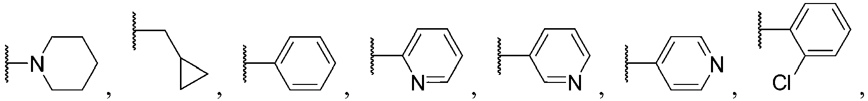

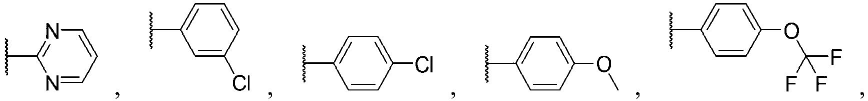

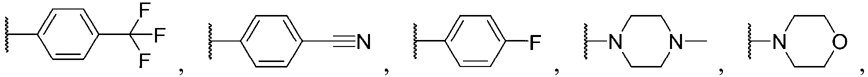

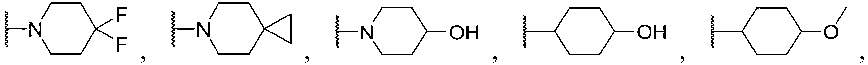

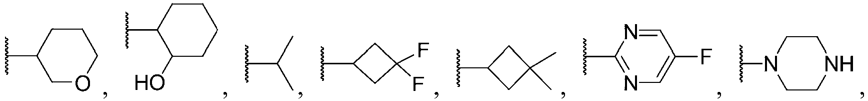

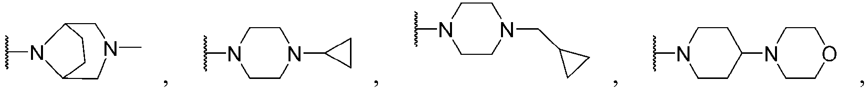

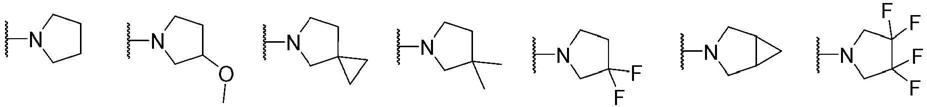

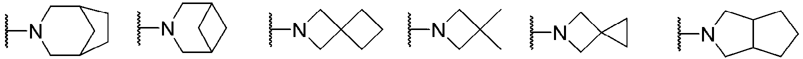

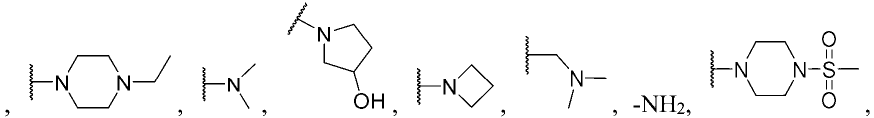

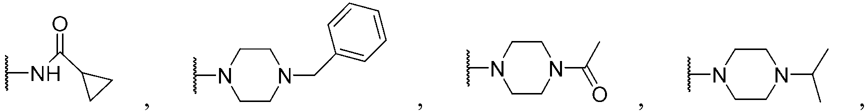

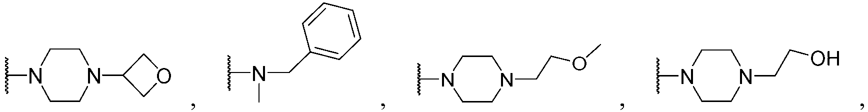

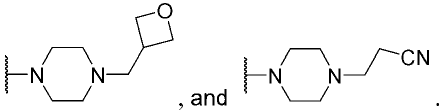

In some embodiments, E is selected from the group consisting of -NH-, -N(CH₃)-, and

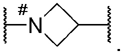

The present disclosure further provides the following compounds or pharmaceutically acceptable salts thereof as shown below:

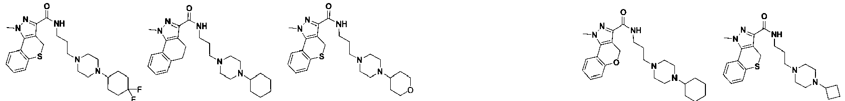

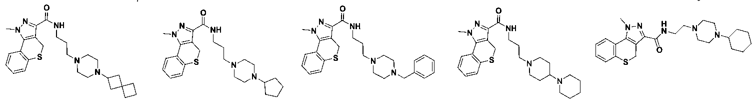

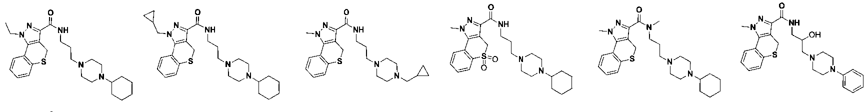

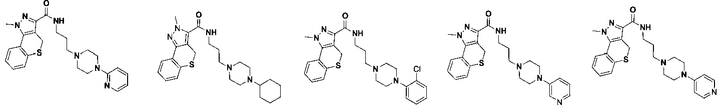

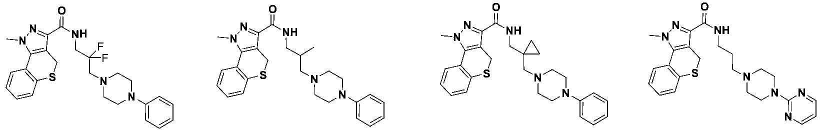

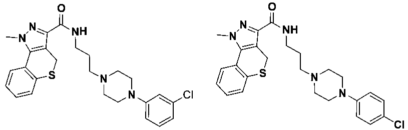

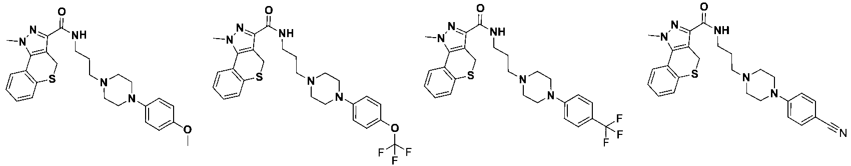

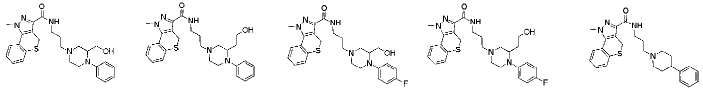

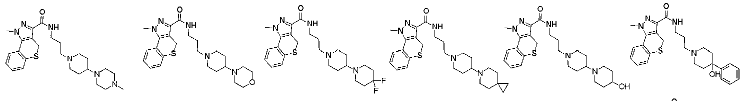

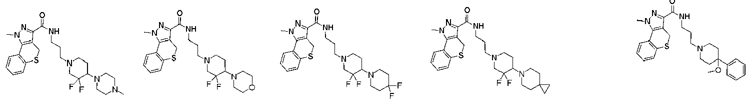

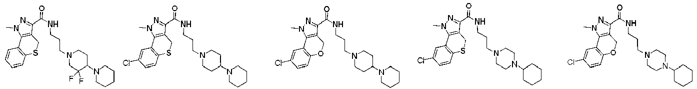

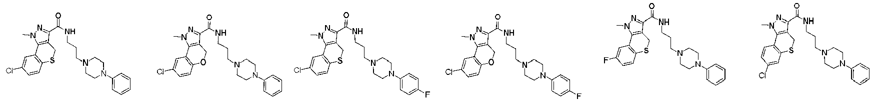

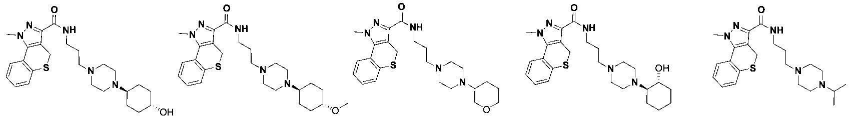

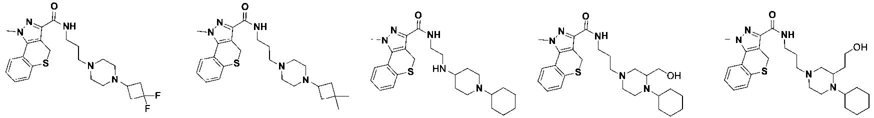

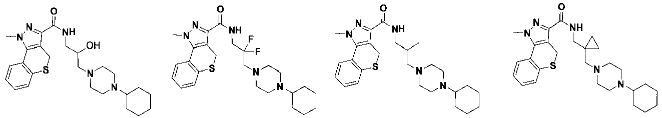

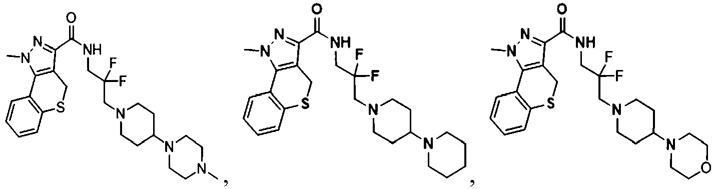

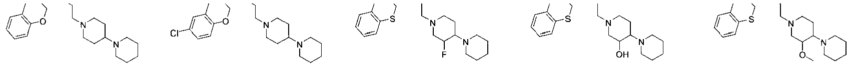

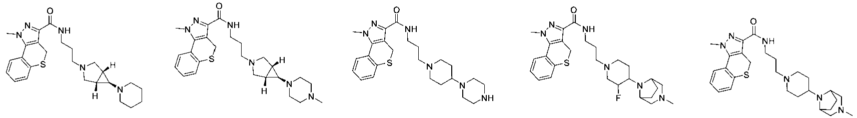

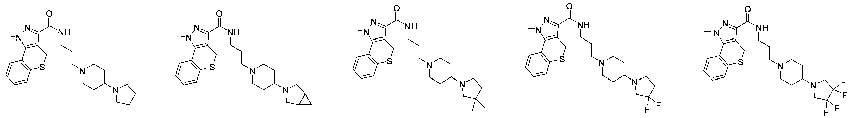

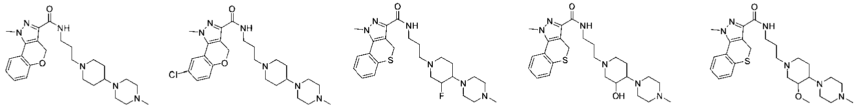

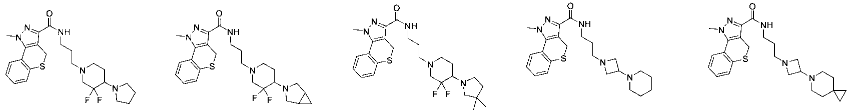

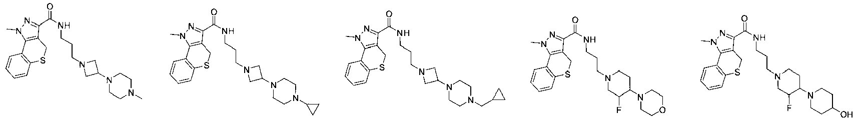

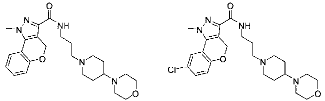

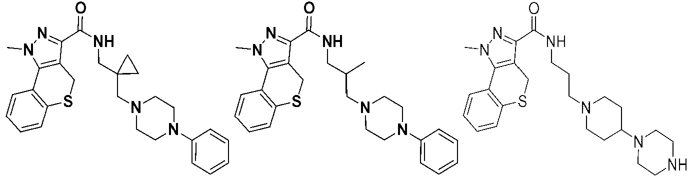

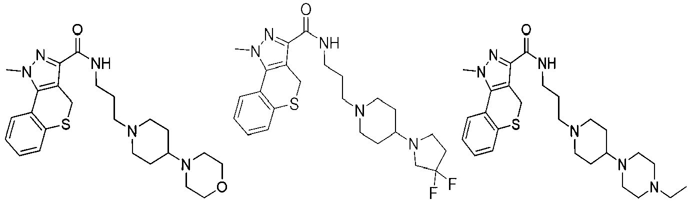

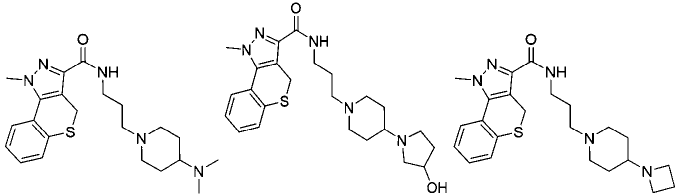

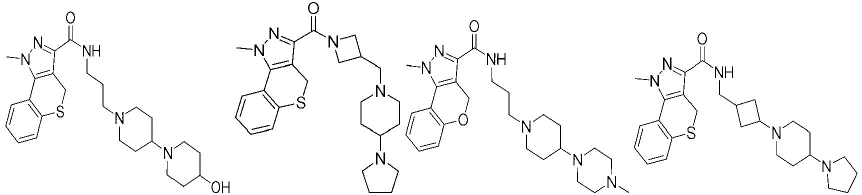

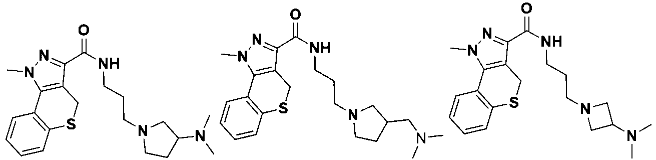

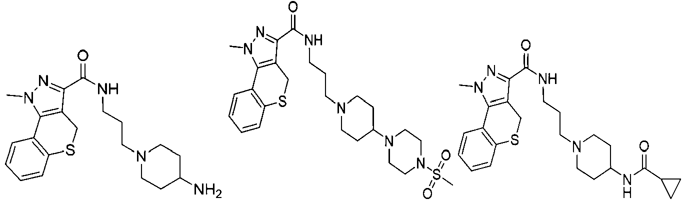

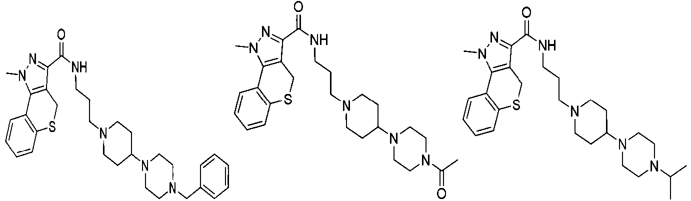

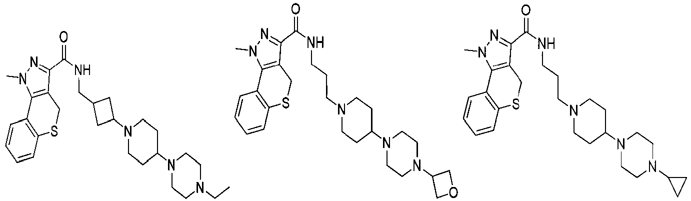

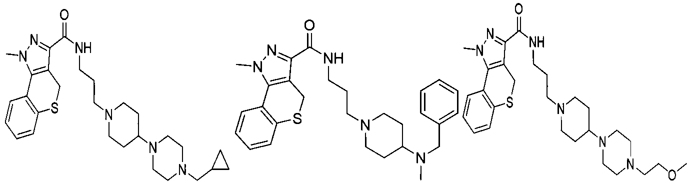

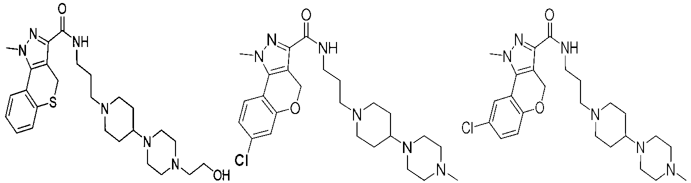

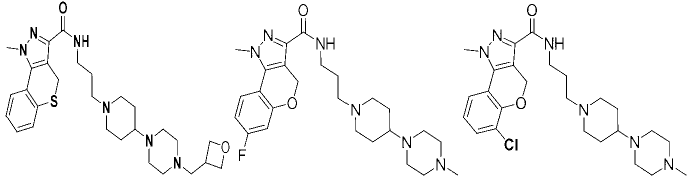

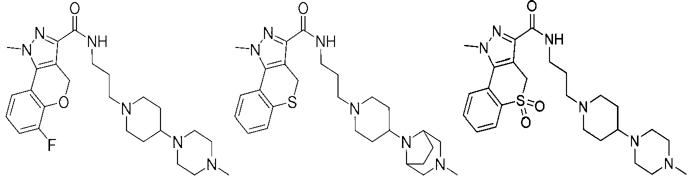

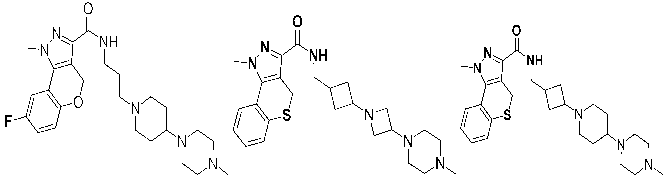

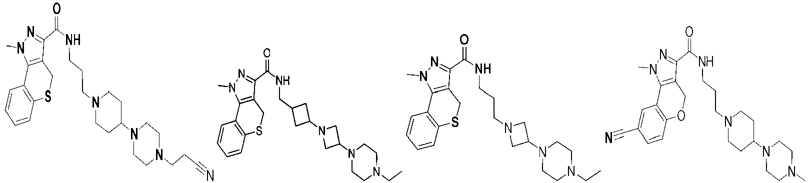

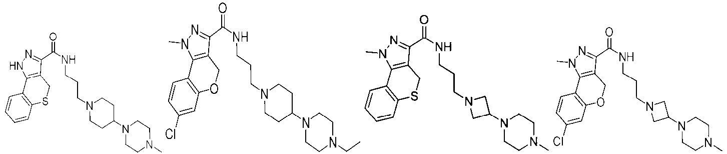

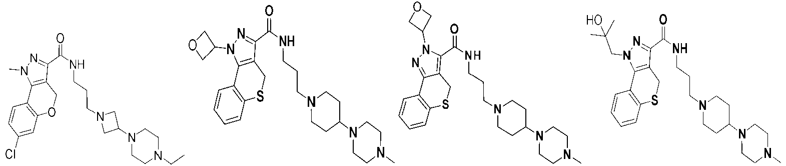

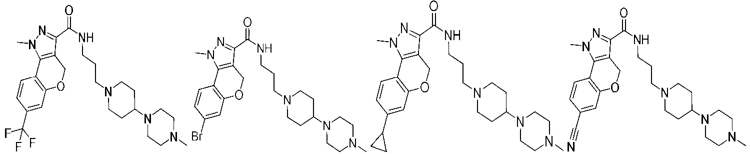

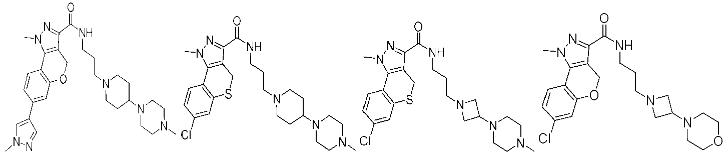

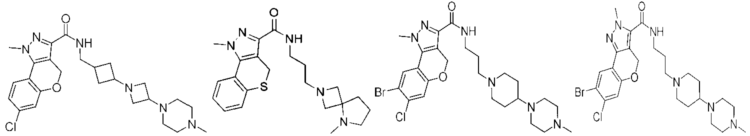

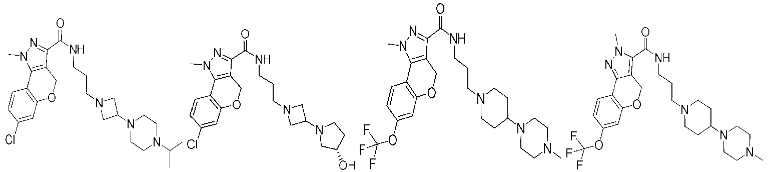

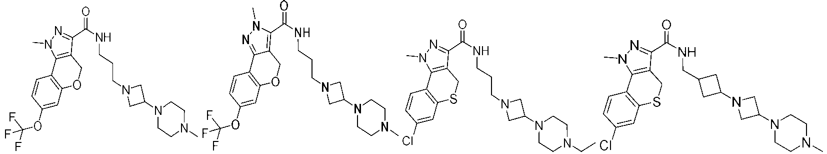

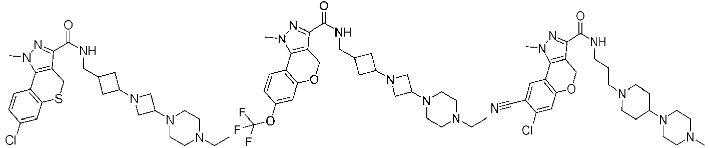

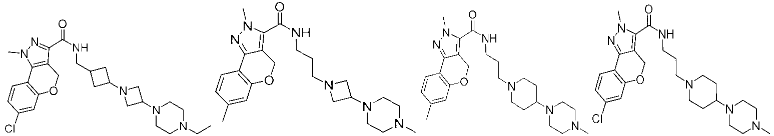

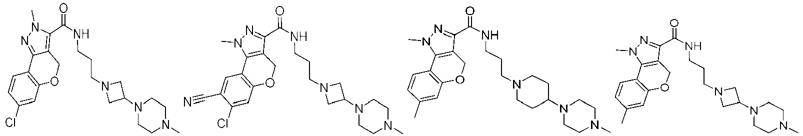

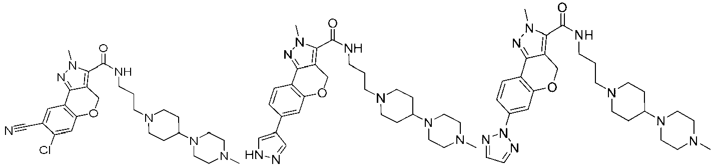

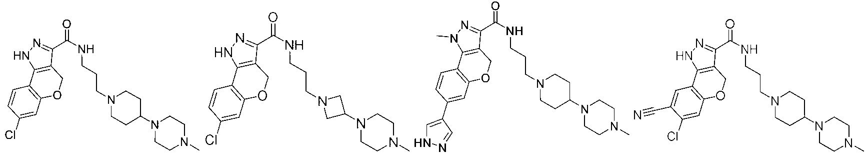

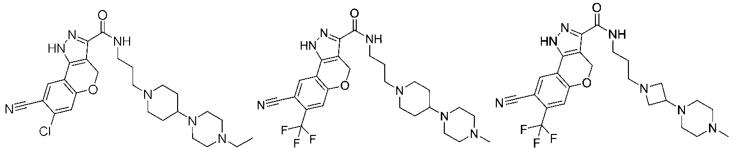

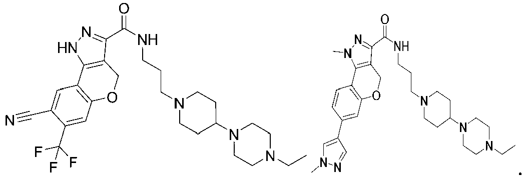

The present disclosure further provides a pharmaceutical composition, including: a substance A and a pharmaceutically acceptable excipient, where the substance A is selected from the group consisting of a compound of Formula (X-A) and a pharmaceutically acceptable salt thereof; 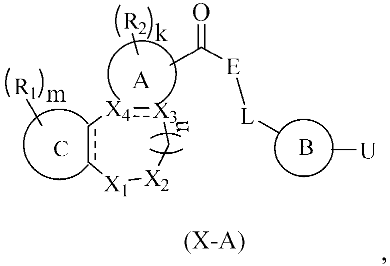 where ring A, ring B, ring C, X₁, X₂, X₃, X₄, R₁, R₂, E, m, n, k, L, B, and U are as defined above.

The present disclosure further provides a pharmaceutical composition, including: (a therapeutically effective amount of) substance A and a pharmaceutically acceptable excipient, where the substance A is a compound of Formula (X) or a pharmaceutically acceptable salt thereof; 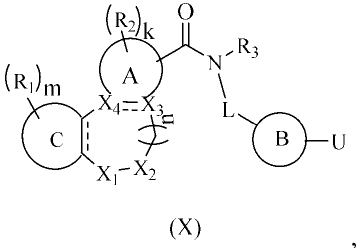 where ring A, ring B, ring C, X₁, X₂, X₃, X₄, R₁, R₂, R₃, m, n, k, L, B, and U are as defined above.

In some embodiments, the pharmaceutical composition is used for treating and/or preventing a KSR2-AMPK-related disease or disorder including cancer, where the cancer includes liver cancer.

The present disclosure further provides use of the substance A or the pharmaceutical composition in preparation of a KSR2-AMPK inhibitor, where the substance A is the compound of Formula (X-A) or the pharmaceutically acceptable salt thereof. Herein, the KSR2-AMPK inhibitor is used in a mammalian organism *in vivo;* this inhibitor is also used *in vitro,* primarily for an experimental purpose. For example, this inhibitor is provided as a standard or a control for comparison, or made into a kit according to conventional methods in the prior art to provide rapid detection of an effect of inhibiting KSR2-AMPK.

The present disclosure further provides use of the substance A or the pharmaceutical composition in preparation of a drug, where the drug is for treating and/or preventing a KSR2-AMPK-related disease or disorder; the substance A is the compound of Formula (X-A) or the pharmaceutically acceptable salt thereof; the substance A is in a therapeutically effective amount; and the KSR2-AMPK-related disease or disorder is preferably a cancer, including liver cancer.

The present disclosure further provides use of the substance A or the pharmaceutical composition in preparation of a drug, where the drug is used for treating and/or preventing a cancer (for example, liver cancer); the substance A is the compound of Formula (X-A) or the pharmaceutically acceptable salt thereof; the substance A is in a therapeutically effective amount.

The present disclosure further provides use of the substance A or the pharmaceutical composition in inhibiting KSR2-AMPK, where the substance A is the compound of Formula (X-A) or the pharmaceutically acceptable salt thereof.

The present disclosure further provides use of the substance A or the pharmaceutical composition in preparation of a KSR2-AMPK inhibitor, where the substance A is the compound of Formula (X) or the pharmaceutically acceptable salt thereof. Herein, the KSR2-AMPK inhibitor is used in a mammalian organism *in vivo;* this inhibitor is also used *in vitro,* primarily for an experimental purpose. For example, this inhibitor is provided as a standard or a control for comparison, or made into a kit according to conventional methods in the prior art to provide rapid detection of an effect of inhibiting KSR2-AMPK.

The present disclosure further provides use of the substance A or the pharmaceutical composition in preparation of a drug, where the drug is for treating and/or preventing a KSR2-AMPK-related disease or disorder; the substance A is the compound of Formula (X) or the pharmaceutically acceptable salt thereof; the substance A is in a therapeutically effective amount; and the KSR2-AMPK-related disease or disorder is preferably a cancer, including liver cancer.

The present disclosure further provides use of the substance A or the pharmaceutical composition in preparation of a drug, where the drug is used for treating and/or preventing a cancer (for example, liver cancer); the substance A is the compound of Formula (X) or the pharmaceutically acceptable salt thereof; the substance A is in a therapeutically effective amount.

The present disclosure further provides use of the substance A or the pharmaceutical composition in inhibiting KSR2-AMPK, where the substance A is the compound of Formula (X) or the pharmaceutically acceptable salt thereof.

In addition to the foregoing, when used in the present disclosure, unless otherwise specifically indicated, the following terms have the meanings shown below.

The term "more" means 2, 3, 4, or 5 when used to describe the number of substituents, heteroatoms, or rings.

Those skilled in the art will understand that, according to conventions used in the art, the 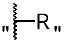 used in the structural formula of a group described in the present disclosure indicates that the corresponding group R (e.g., R₁, R₂, R₃, or R₄) is linked to other fragments or groups in the compound via this site.

The term "pharmaceutically acceptable salt" refers to a salt prepared from a compound of the present disclosure with a relatively non-toxic, pharmaceutically acceptable acid or base. When the compounds of the present disclosure include relatively-acidic functional groups, pharmaceutically acceptable base addition salts can be obtained by contacting the neutral forms of such compounds with a sufficient amount of base in a pure solution or in a suitable inert solvent. The pharmaceutically acceptable base addition salts include, but are not limited to: salts of lithium, sodium, potassium, calcium, aluminum, magnesium, zinc, bismuth, ammonium, and diethanolamine. When the compounds of the present disclosure include relatively-alkaline functional groups, acid addition salts can be obtained by contacting the neutral forms of such compounds with a sufficient amount of acid in a pure solution or in a suitable inert solvent. The pharmaceutically acceptable acids include inorganic acids and organic acids. Details refer to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "pharmaceutical composition" refers to a formulation including a compound of the present disclosure and a medium generally accepted in the art for delivering biologically active compounds to mammals (e.g., humans). This medium includes pharmaceutically acceptable carriers. A purpose of the pharmaceutical composition is to facilitate administration to an organism, promote the absorption of the active ingredient, and thereby exert biological activity.

The term "pharmaceutically acceptable excipient" refers to vehicles and additives used in the production of pharmaceuticals and the compounding of prescriptions, and includes all substances in the pharmaceutical preparation other than the active ingredient. This excipient refers to the Pharmacopoeia of the People's Republic of China (2015 Edition) Part IV, or Handbook of Pharmaceutical Excipients (Raymond C Rowe, 2009 Sixth Edition). Excipients are mainly used to provide a safe, stable, and functional pharmaceutical composition, and can also provide a means to achieve the desired dissolution rate of the active ingredient after administration to a subject, or to promote effective absorption of the active ingredient after the subject receives the composition. The pharmaceutical excipient can be an inert filler or provide a certain function, such as stabilizing the overall pH of the composition or preventing degradation of the active ingredient of the composition. The pharmaceutical excipient can include one or more of the following excipients: binders, suspending agents, emulsifiers, diluents, fillers, granulating agents, adhesives, disintegrants, lubricants, anti-adherents, glidants, wetting agents, gelling agents, absorption retardants, dissolution inhibitors, enhancers, adsorbents, buffers, chelating agents, preservatives, colorants, flavoring agents, and sweeteners.

The term "treating" refers to therapeutic therapy or palliative measures. In relation to a specific disease or condition, treatment means: (1) alleviating one or more biological manifestations of the disease or condition, (2) interfering with (a) one or more points in the biological cascade that causes or induces the condition or (b) one or more biological manifestations of the condition, (3) ameliorating one or more symptoms, effects, or side effects associated with the condition, or one or more symptoms, effects, or side effects associated with the condition or its treatment, or (4) slowing the progression of the condition or one or more biological manifestations of the condition. "Treating" can also mean prolonging survival compared to expected survival without treatment.

The term "preventing" refers to reducing the risk of acquiring or developing a disease or disorder.

The term "therapeutically effective amount" refers to an amount of the compound that, when administered to a patient, is sufficient to effectively treat the disease or disorder described herein. The "therapeutically effective amount" will vary with the compound, the disease or disorder and its severity, and the age of the patient to be treated, but can be adjusted as needed by those skilled in the art.

The term "substituted" or "substituent" means that a hydrogen atom in a group is replaced by a specified group. When a substituted position is not specified, substitution can occur at any position, provided it results in a stable or chemically feasible compound. For example, the structure 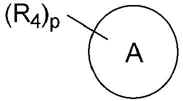 indicates that the hydrogen atom(s) on ring A is/are substituted by p R₄ groups.

When any variable (such as R) occurs more than once in the composition or structure of a compound, its definition in each case is independent. Thus, for example, if a group is substituted by one or more R, then the group can optionally be substituted by at least one R, and R has independent options in each case. Furthermore, combinations of substituents and/or variants thereof are permissible only if such combinations result in stable compounds.

The term "alkyl" refers to a saturated, straight or branched chain, monovalent hydrocarbon group. C₁₋₆ alkyl refers to alkyl having 1 to 6 carbon atoms, preferably C₁₋₄ alkyl having 1 to 4 carbon atoms, specifically methyl, ethyl, n-propyl, isopropyl, n-butyl, isobutyl, sec-butyl, or tert-butyl.

The term "alkylene" is a divalent group linked to the rest of the molecule by two single bonds, with the rest of the definition being the same as the term "alkyl".

The term "alkoxy" refers to -O-C₁₋₆ alkyl, where "C₁₋₆ alkyl" is as defined above. Preferably, it is alkoxy having 1 to 4 carbon atoms, such as methoxy or ethoxy.

The term "cycloalkyl" refers to a saturated, monocyclic or polycyclic (e.g., bicyclic, tricyclic, or more rings in a bridged, fused, or spiro system) carbocyclic ring substituent, which can be linked to the rest of the molecule via any suitable carbon atom by a single bond. For example, 3- to 10-membered cycloalkyl has 3 to 10 carbon atoms. Examples of cycloalkyl include, but are not limited to, cyclopropyl, cyclobutyl, cyclopentyl, cyclohexyl, and

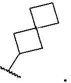

The term "carbocyclic ring" refers to a cyclic, saturated or unsaturated ring group having a specified number of carbon atoms (e.g., C₃₋₁₀), which is monocyclic or polycyclic (e.g., bicyclic, tricyclic, or more rings in a bridged, fused, or spiro system), preferably a 3-membered saturated carbocyclic ring. It satisfies any one of the following conditions: (1) being linked to the rest of the molecule by two or more single bonds, such as 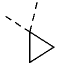 (" 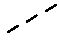 " represents a bond linked to the rest of the molecule) or (2) sharing two atoms and one bond with the rest of the molecule.

The term "heterocyclic ring" refers to a cyclic, saturated or unsaturated ring group having a specified number of ring atoms (e.g., 3- to 10-membered), a specified number of heteroatoms (e.g., 1, 2, or 3), and specified types of heteroatoms (one or more selected from the group consisting of N, O, and S), which is monocyclic or polycyclic (e.g., bicyclic, tricyclic, or more rings in a bridged, fused, or spiro system); preferably a 5- to 6-membered saturated monocyclic heterocyclic ring. It satisfies any one of the following conditions: (1) being linked to the rest of the molecule by two or more bonds, such as 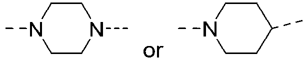 (" 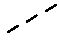 " represents a bond linked to the rest of the molecule) or (2) sharing two atoms and one bond with the rest of the molecule.

The term "heterocycloalkyl" refers to a saturated, monovalent group having a specified number of ring atoms (e.g., 3- to 10-membered), a specified number of heteroatoms (e.g., 1, 2, or 3), and specified types of heteroatoms (one or more selected from the group consisting of N, O, and S), which is monocyclic or polycyclic (e.g., bicyclic, tricyclic, or more rings in a bridged, fused, or spiro system), and is linked to the rest of the molecule via a carbon atom or a heteroatom. Examples of heterocycloalkyl include, but are not limited to, morpholinyl, piperidinyl, piperazinyl, tetrahydropyranyl, and azaspiro[2.5]octyl, such as

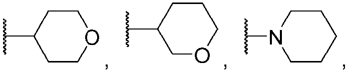

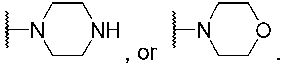

The term "aryl" refers to a cyclic, unsaturated, monovalent hydrocarbon group having a specified number of carbon atoms (e.g., C₆₋₁₀), which is monocyclic or polycyclic (e.g., 2 or 3 rings). When polycyclic, the monocyclic rings share two atoms and one bond, and at least one ring is aromatic. Aryl may be linked to the rest of the molecule via an aromatic ring or a non-aromatic ring. Aryl includes, but is not limited to phenyl or naphthyl.

The term "heteroaryl" refers to a cyclic, unsaturated, monovalent group having a specified number of ring atoms (e.g., 5- to 10-membered), a specified number of heteroatoms (e.g., 1, 2, or 3), and specified types of heteroatoms (one or more selected from the group consisting of N, O, and S), which is monocyclic or polycyclic; when polycyclic, every two monocyclic rings share two atoms and one bond, and at least one ring is aromatic. Heteroaryl may be linked to the rest of the molecule via a carbon atom or a heteroatom; the heteroaryl may be linked to the rest of the molecule via a ring containing heteroatoms or a ring without heteroatoms; the heteroaryl may be linked to the rest of the molecule via an aromatic ring or a non-aromatic ring. Heteroaryl includes, but is not limited to, pyridinyl or pyrimidinyl, such as

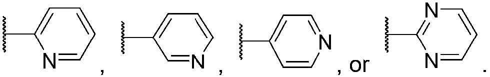

The term "heteroaromatic ring" refers to a cyclic, unsaturated group having a specified number of ring atoms (e.g., 5- to 10-membered), a specified number of heteroatoms (e.g., 1, 2, or 3), and specified types of heteroatoms (one or more selected from the group consisting of N, O, and S), which is monocyclic or polycyclic; when polycyclic, every two monocyclic rings share two atoms and one bond, and at least one ring is aromatic; preferably a 5- to 6-membered monocyclic heteroaromatic ring. It satisfies any one of the following conditions: (1) being linked to the rest of the molecule by two or more bonds; (2) sharing two atoms and one bond with the rest of the molecule.

The term "halogen" refers to fluorine (F), chlorine (CI), bromine (Br), or iodine (I), particularly F or Cl.

On the basis of conforming to common knowledge in the art, the above-mentioned preferred conditions can be combined arbitrarily to obtain preferred embodiments of the present disclosure.

The reagents and raw materials used in the present disclosure are all commercially available.

The advantageous effects of the present disclosure are that the compounds of the present disclosure can effectively inhibit the proliferation of cancer cells.

### Detailed Description of Embodiments

The present disclosure is further described below through examples, but the present disclosure is not limited to the scope of the described examples. The experimental methods in the following examples which are not specified with specific conditions are conducted according to conventional conditions or according to product instructions.

### Example 1

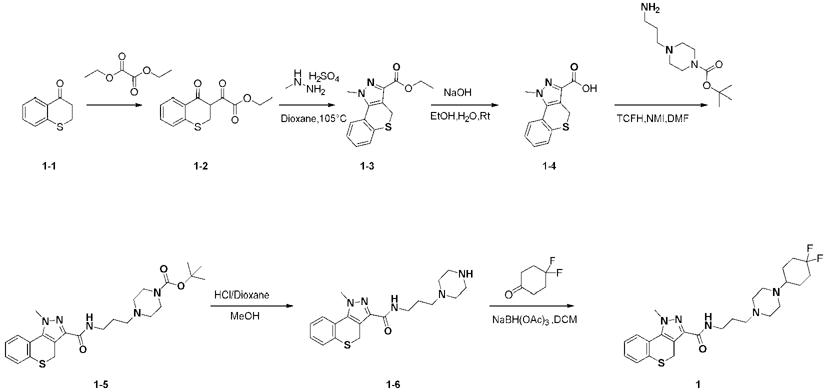

In step 1: To a 500 mL three-necked flask, ethanol (120 mL) and 20 wt% sodium ethoxide in ethanol (83 g, 243.58 mmol) are added sequentially. Under a nitrogen atmosphere, diethyl oxalate (26.7 g, 182.69 mmol) and **compound 1-1** (20 g, 121.79 mmol) are added at 0°C. The mixture is stirred at room temperature for 16 h. The pH of the reaction mixture is adjusted to 6-7 with dilute hydrochloric acid. The ethanol is removed by concentration under reduced pressure. Water and ethyl acetate are added, and the layers are separated. The aqueous phase is extracted with ethyl acetate twice. The combined organic phases are washed twice with brine, dried over anhydrous sodium sulfate, filtered, and the filtrate is concentrated under reduced pressure to obtain a product **compound 1-2** (29 g, yield: 90%), which is used directly in the next step.

ESI(m/z) =263.1 [M-1]
In step 2: **Compound 1-2** (7 g, 26.49 mmol) is dissolved in dioxane (70 mL). Methylhydrazine sulfate (7.64 g, 52.98 mmol) is added with stirring. After the addition, the reaction is conducted at 105°C for 16 h, monitored by liquid chromatography-mass spectrometry (LCMS). The reaction mixture is poured into water (200 mL) and extracted with ethyl acetate (2*500 mL). The organic phase is washed 1 time with brine (200 mL), dried over anhydrous sodium sulfate, filtered, and concentrated under reduced pressure. The residue is purified by column chromatography to afford **compound 1-3** (2.65 g, 36% yield).

ESI(m/z) =275.2[M+H]⁺, RT = 3.657 min.

¹H NMR (400 MHz, DMSO-*d*₆) δ 7.88 - 7.84 (m, 1H), 7.52 (dd, *J* = 7.3, 1.8 Hz, 1H), 7.33 (pd, *J* = 7.4, 1.6 Hz, 2H), 4.30 (q, *J* = 7.1 Hz, 2H), 4.16 (s, 3H), 4.15 (s, 2H), 1.31 (t, *J* = 7.1 Hz, 3H).

Step 3: **Compound 1-3** (2.65 g, 9.66 mmol) is dissolved in ethanol (30 mL) and water (10 mL). Sodium hydroxide (1.16 g, 29 mmol) is added with stirring. The reaction mixture is stirred at 20°C for 16 h. LCMS shows that the reaction is complete. The reaction mixture is concentrated to remove ethanol. The pH is adjusted to 4 with 4N hydrochloric acid. The mixture is triturated with water for 30 min, filtered, and the filter cake is dried to obtain **compound 1-4** (2.25 g, 95% yield).

ESI(m/z) =247.1[M+H]⁺, RT = 3.005 min.

¹H NMR (400 MHz, DMSO-*d*₆) δ 7.85 (dd, *J* = 7.4, 1.7 Hz, 1H), 7.51 (dd, *J* = 7.4, 1.7 Hz, 1H), 7.37 - 7.28 (m, 2H), 4.14 (s, 5H).

Step 4: **Compound 1-4** (150 mg, 0.61 mmol), tert-butyl 4-(3-aminopropyl)piperazine-1-carboxylate (160 mg, 0.66 mmol), and N-methylimidazole (NMI, 150 mg, 1.83 mmol) are dissolved in N,N-dimethylformamide (DMF, 12 mL). Chloro-N,N,N',N'-tetramethylformamidinium hexafluorophosphate (TCFH, 200 mg, 0.71 mmol) is added at 0°C. The reaction mixture is stirred at 20°C for 1 h. LCMS shows that the reaction is complete. The reaction mixture is quenched with water and extracted with ethyl acetate. The organic phase is washed with saturated brine, dried over anhydrous sodium sulfate, and purified by column chromatography to obtain a white solid **compound 1-5** (245 mg, 85%).

ESI(m/z) =472.4[M+H]⁺, RT = 2.803 min.

¹H NMR (400 MHz, Chloroform-*d*) δ 8.20 (s, 1H), 7.60 - 7.54 (m, 1H), 7.52 - 7.47 (m, 1H), 7.26 - 7.19 (m, 2H), 4.29 (s, 2H), 4.08 (s, 3H), 3.58 - 3.50 (m, 6H), 2.54 (t, *J* = 6.2 Hz, 2H), 2.45 (t, *J =* 5.0 Hz, 4H), 1.78 (p, *J* = 6.2 Hz, 2H), 1.47 (s, 9H).

Step 5: **Compound 1-5** (245 mg, 0.52 mmol) is added to a reaction flask, followed by HCl/Dioxane (2 mL, 4 mmol/mL) and methanol (2 mL). The reaction is conducted at 20°C for 1 h. LCMS shows that the reaction is complete. The reaction mixture is poured into saturated aqueous sodium bicarbonate solution and extracted with ethyl acetate. The organic phase is dried over anhydrous sodium sulfate and concentrated to dryness to obtain **compound 1-6** (60 mg, 31%).

ESI(m/z) =372.3[M+H]⁺, RT = 2.281 min.

Step 6: **Compound 1-6** (60 mg, 0.16 mmol) and 4,4-difluorocyclohexanone (40 mg, 0.30 mmol) are dissolved in dichloromethane (DCM, 5 mL). Sodium triacetoxyborohydride (70 mg, 0.33 mmol) is added. The reaction mixture is stirred at 20°C for 16 h. LCMS shows that the reaction is complete. The reaction mixture is quenched with saturated aqueous sodium bicarbonate solution and extracted with ethyl acetate. The organic phase is washed with saturated brine, dried over anhydrous sodium sulfate, filtered, and purified by column chromatography. The resulting product is dissolved in water and lyophilized to obtain **compound 1** (38.9 mg, 49 %).

ESI(m/z) =490.4[M+H]⁺, RT = 6.364 min.

¹H NMR (400 MHz, DMSO-*d*₆) δ 8.34 (t, *J* = 5.7 Hz, 1H), 7.84 (dd, *J* = 7.6, 1.6 Hz, 1H), 7.50 (dd, *J* = 7.4, 1.6 Hz, 1H), 7.37 - 7.27 (m, 2H), 4.18 (s, 2H), 4.13 (s, 3H), 3.32 - 3.17 (m, 6H), 2.47 - 2.23 (m, 6H), 1.99 (t, *J* = 10.9 Hz, 2H), 1.91 (s, 1H), 1.88 - 1.38 (m, 8H).

### Example 2

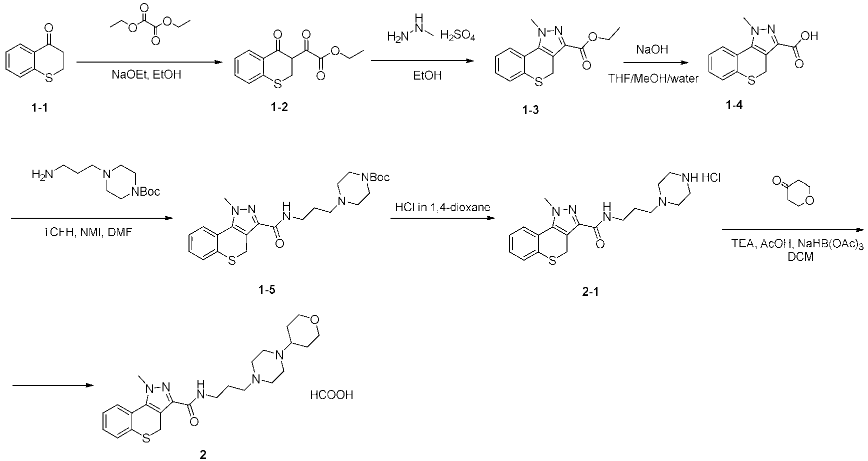

In step 1: To a 500 mL three-necked flask, ethanol (120 mL) and 20 wt% sodium ethoxide in ethanol (83 g, 243.58 mmol) are added sequentially. Under a nitrogen atmosphere, **diethyl oxalate** (26.7 g, 182.69 mmol) and **compound 1-1** (20 g, 121.79 mmol) are added at 0°C. The mixture is stirred at room temperature for 16 h. The pH of the reaction mixture is adjusted to 6-7 with dilute hydrochloric acid. The ethanol is removed by concentration under reduced pressure. Water and ethyl acetate are added, and the layers are separated. The aqueous phase is extracted with ethyl acetate twice. The combined organic phases are washed twice with brine, dried over anhydrous sodium sulfate, filtered, and the filtrate is concentrated under reduced pressure to obtain a product **compound 1-2** (29 g, yield: 90%), which is used directly in the next step.

ESI(m/z) =263.1 [M-1]
In step 2: To a 500 mL single-necked flask, **compound 1-2** (14 g, 52.97 mmol), ethanol (200 mL), and methylhydrazine sulfate (11.45 g, 79.45 mmol) are added sequentially. The mixture is stirred at 80°C for 6 h. The reaction mixture is cooled to room temperature, and the ethanol is removed by concentration under reduced pressure. Water and ethyl acetate are added, and the layers are separated. The aqueous phase is extracted with ethyl acetate twice. The combined organic phases are washed twice with aqueous sodium bicarbonate solution, dried over anhydrous sodium sulfate, filtered, and the filtrate is concentrated under reduced pressure. The residue is purified by column chromatography to obtain a product **compound 1-3** (11 g, yield: 75.7%).

ESI(m/z) =275.1 [M+H]+
¹H NMR (400 MHz, DMSO-*d*₆) δ 7.88 - 7.84 (m, 1H), 7.52 (dd, *J* = 7.3, 1.8 Hz, 1H), 7.33 (pd, *J* = 7.4, 1.6 Hz, 2H), 4.30 (q, *J* = 7.1 Hz, 2H), 4.16 (s, 3H), 4.15 (s, 2H), 1.31 (t, *J* = 7.1 Hz, 3H).

In step 3: To a 250 mL single-necked flask, **compound 1-3** (5 g, 18.23 mmol), tetrahydrofuran (THF, 25 mL), methanol (25 mL), water (50 mL), and sodium hydroxide (0.88 g, 21.88 mmol) are added sequentially. The mixture is stirred at room temperature for 4 h. The reaction mixture is concentrated under reduced pressure to remove the organic solvents. Water is added for dilution, and the pH is adjusted to 2-3 with dilute hydrochloric acid. The mixture is stirred for 30 min and then filtered. The filter cake is dried to obtain a product **compound 1-4** (4.1 g, yield: 91%).

ESI(m/z) =247.1 [M+H]+
In step 4: To a 100 mL single-necked flask, **compound 1-4** (1 g, 4.06 mmol), **tert-butyl 4-(3-aminopropyl)piperazine-1-carboxylate** (1.48 g, 6.09 mmol), DMF (15 mL), NMI (0.83 g, 10.15 mmol), and TCFH (1.37 g, 4.87 mmol) are added sequentially. The mixture is stirred at room temperature for 16 h. Water and ethyl acetate are added to the reaction mixture, and the layers are separated. The aqueous phase is extracted with ethyl acetate twice. The combined organic phases are washed twice with brine, dried over anhydrous sodium sulfate, filtered, and the filtrate is concentrated under reduced pressure. The residue is purified by column chromatography to obtain a product **compound 1-5** (1.4 g, yield: 73.11%).

ESI(m/z) =472.3 [M+H]+
In step 5: To a 100 mL single-necked flask, **compound 1-5** (1.4 g, 2.97 mmol) and a solution of hydrogen chloride in 1,4-dioxane (4 mol/L, 15 mL) are added sequentially. The mixture is stirred at room temperature for 4 h. The reaction mixture is concentrated under reduced pressure to obtain a product **compound 2-1** (1.2 g, yield: 99%). The product is used directly in the next step.

ESI(m/z) = 372.3 [M+H]+
In step 6: To a 25 mL single-necked flask, **compound 2-1** (55 mg, 0.13 mmol), DCM (1 mL), and triethylamine (TEA, 17 mg, 0.17 mmol) are added sequentially. The mixture is stirred at room temperature for 2 min. **Tetrahydro-4*H*-pyran-4-one** (26 mg, 0.26 mmol) and acetic acid (20 mg, 0.33 mmol) are added, and the mixture is stirred at room temperature for 5 min. Then sodium triacetoxyborohydride (55 mg, 0.26 mmol) is added, and the mixture is stirred at 35°C for 6 h. Water and ethyl acetate are added to the reaction mixture, and the layers are separated. The aqueous phase is extracted with ethyl acetate twice. The combined organic phases are washed twice with brine, dried over anhydrous sodium sulfate, filtered, and the filtrate is concentrated under reduced pressure. The residue is purified by reverse-phase preparative chromatography to obtain a product **compound 2** (16.2 mg, yield: 26.37%).

ESI(m/z) =456.4 [M+H]+
¹H NMR (400 MHz, ) δ 8.35 (t, J = 5.7 Hz, 1H), 8.23 (s, 1H), 7.84 (dd, J = 7.5, 1.7 Hz, 1H), 7.50 (dd, J = 7.4, 1.7 Hz, 1H), 7.37 - 7.27 (m, 2H), 4.19 (s, 2H), 4.14 (s, 3H), 3.90 - 3.82 (m, 2H), 3.33 - 3.20 (m, 4H), 2.51 (s, 3H), 2.49 (s, 2H), 2.32 (q, J = 6.8 Hz, 6H), 1.68 (tt, J = 13.5, 9.5 Hz, 4H), 1.36 (qd, J = 12.2, 4.5 Hz, 2H).

### Example 3

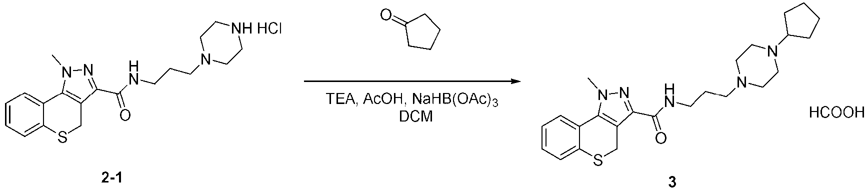

In a 25 mL single-necked flask, **compound 2-1** (100 mg, 0.25 mmol), DCM (2 mL), and TEA (33 mg, 0.33 mmol) are added sequentially. The mixture is stirred at room temperature for 2 min. **Cyclopentanone** (42 mg, 0.50 mmol) and acetic acid (38 mg, 0.63 mmol) are added, and the mixture is stirred at room temperature for 5 min. Then, sodium triacetoxyborohydride (110 mg, 0.50 mmol) is added, and the mixture is stirred at 35°C for 6 h. Water and ethyl acetate are added to the reaction mixture, and the layers are separated. The aqueous phase is extracted with ethyl acetate twice. The combined organic phases are washed twice with brine, dried over anhydrous sodium sulfate, filtered, and the filtrate is concentrated under reduced pressure. The residue is purified by reverse-phase preparative chromatography to obtain a product **compound 3** (38.8 mg, yield: 32.6%).

ESI(m/z) =440.4 [M+H]+
¹H NMR (400 MHz, ) δ 8.37 (t, J = 5.7 Hz, 1H), 8.20 (s, 1H), 7.87 - 7.80 (m, 1H), 7.50 (dd, J = 7.3, 1.7 Hz, 1H), 7.37 - 7.26 (m, 2H), 4.18 (s, 2H), 4.14 (s, 3H), 3.95 (s, 3H), 3.28 (q, J = 6.5 Hz, 2H), 2.64 - 2.52 (m, 3H), 2.47 - 2.27 (m, 5H), 1.78 (dq, J = 11.3, 6.5 Hz, 2H), 1.71 - 1.42 (m, 6H), 1.33 (ddd, J = 16.7, 13.5, 8.4 Hz, 2H).

### Example 4

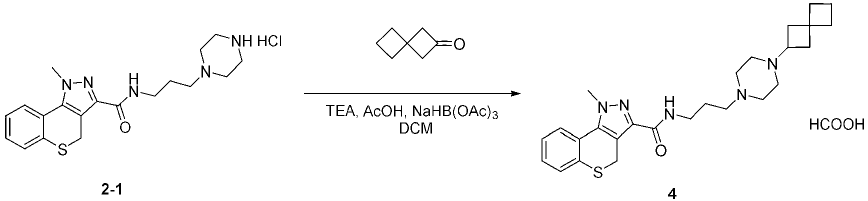

In a 25 mL single-necked flask, **compound 2-1** (100 mg, 0.25 mmol), DCM (2 mL), and TEA (33 mg, 0.33 mmol) are added sequentially. The mixture is stirred at room temperature for 2 min. **Spiro[3.3]heptan-2-one** (55 mg, 0.50 mmol) and acetic acid (38 mg, 0.63 mmol) are added, and the mixture is stirred at room temperature for 5 min. Then, sodium triacetoxyborohydride (110 mg, 0.50 mmol) is added, and the mixture is stirred at 35°C for 6 h. Water and ethyl acetate are added to the reaction mixture, and the layers are separated. The aqueous phase is extracted with ethyl acetate twice. The combined organic phases are washed twice with brine, dried over anhydrous sodium sulfate, filtered, and the filtrate is concentrated under reduced pressure. The residue is purified by reverse-phase preparative chromatography to obtain a product **compound 4** (54.6 mg, yield: 43.53%).

ESI(m/z) =466.4 [M+H]+
¹H NMR (400 MHz, ) δ 8.36 (t, J = 5.7 Hz, 1H), 8.20 (s, 1H), 7.84 (dd, J = 7.6, 1.6 Hz, 1H), 7.50 (dd, J = 7.4, 1.7 Hz, 1H), 7.32 (dtd, J = 16.7, 7.4, 1.6 Hz, 2H), 4.25 (s, 5H), 4.18 (s, 2H), 4.14 (s, 3H), 3.27 (q, J = 6.4 Hz, 2H), 2.76 (p, J = 7.8 Hz, 1H), 2.38 (p, J = 12.9, 11.8 Hz, 9H), 2.02 - 1.88 (m, 2H), 1.86 - 1.71 (m, 2H), 1.72 - 1.52 (m, 4H).

### Example 5

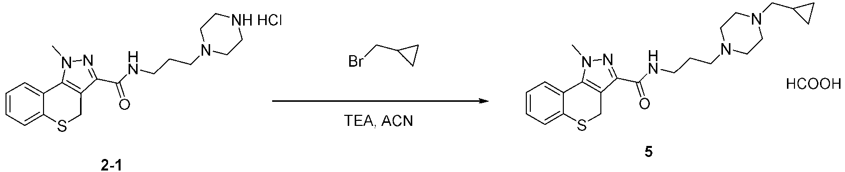

In a 50 mL single-necked flask, **compound 2-1** (100 mg, 0.25 mmol), acetonitrile (2 mL), TEA (76 mg, 0.75 mmol), and **(bromomethyl)cyclopropane** (51 mg, 0.38 mmol) are added sequentially. The mixture is stirred at 60°C for 4 h. The reaction mixture is cooled to room temperature and filtered. The filtrate is purified by reverse-phase preparative chromatography to obtain a product **compound** 5 (24.1 mg, yield: 23.1%).

ESI(m/z) =426.3 [M+H]+
¹H NMR (400 MHz, ) δ 8.38 (t, J = 5.7 Hz, 1H), 8.20 (s, 1H), 7.83 (dd, J = 7.6, 1.7 Hz, 1H), 7.50 (dd, J = 7.3, 1.8 Hz, 1H), 7.37 - 7.26 (m, 2H), 4.18 (s, 2H), 4.13 (s, 3H), 3.28 (q, J = 6.5 Hz, 2H), 2.53 (d, J = 12.5 Hz, 4H), 2.50 - 2.30 (m, 6H), 2.24 (d, J = 6.6 Hz, 2H), 1.66 (p, J = 6.8 Hz, 2H), 0.83 (dddd, J = 12.6, 9.6, 4.6, 3.2 Hz, 1H), 0.50 - 0.43 (m, 2H), 0.12 - 0.05 (m, 2H).

### Example 6

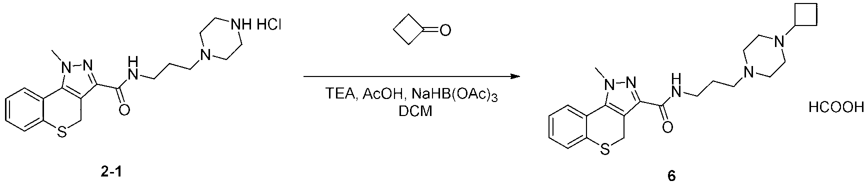

In a 25 mL single-necked flask, **compound 2-1** (100 mg, 0.25 mmol), DCM (2 mL), and TEA (33 mg, 0.33 mmol) are added sequentially. The mixture is stirred at room temperature for 2 min. **Cyclobutanone** (35 mg, 0.50 mmol) and acetic acid (38 mg, 0.63 mmol) are added, and the mixture is stirred at room temperature for 5 min. Then, sodium triacetoxyborohydride (110 mg, 0.50 mmol) is added, and the mixture is stirred at 35°C for 6 h. Water and ethyl acetate are added to the reaction mixture, and the layers are separated. The aqueous phase is extracted with ethyl acetate twice. The combined organic phases are washed twice with brine, dried over anhydrous sodium sulfate, filtered, and the filtrate is concentrated under reduced pressure. The residue is purified by reverse-phase preparative chromatography to obtain a product **compound 6** (45.7 mg, yield: 39.53%).

ESI(m/z) =426.4 [M+H]+
¹H NMR (400 MHz, DMSO-d6) δ 8.35 (t, J = 5.7 Hz, 1H), 8.17 (s, 1H), 7.84 (dd, J = 7.6, 1.6 Hz, 1H), 7.50 (dd, J = 7.4, 1.7 Hz, 1H), 7.37 - 7.27 (m, 2H), 4.18 (s, 2H), 4.14 (s, 3H), 3.27 (q, J = 6.5 Hz, 2H), 2.59 - 2.52 (m, 1H), 2.39 - 2.22 (m, 6H), 2.07 (ddd, J = 9.4, 7.0, 3.0 Hz, 2H), 1.96 (t, J = 7.1 Hz, 2H), 1.80 (dddd, J = 21.9, 8.9, 6.3, 2.2 Hz, 4H), 1.73 - 1.60 (m, 4H).

### Example 7

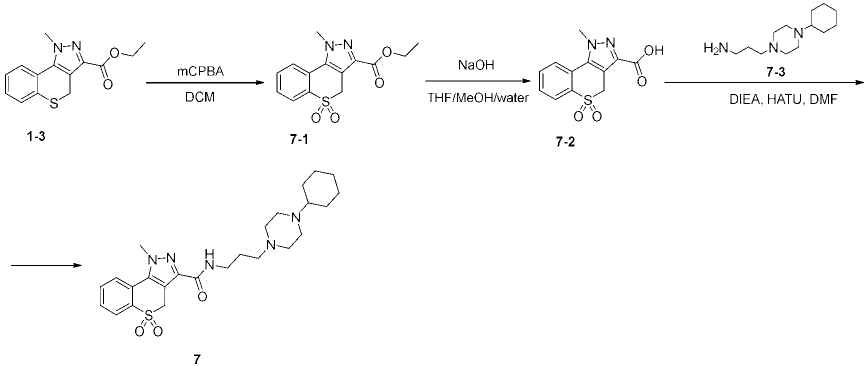

In step 1: In a 50 mL single-necked flask, **compound 1-3** (200 mg, 0.73 mmol), DCM (3 mL), and m-chloroperoxybenzoic acid (*m*-CPBA, 370 mg, 1.82 mmol) are added sequentially. The mixture is stirred at room temperature for 4 h. Aqueous sodium sulfite solution and ethyl acetate are added to the reaction mixture, and the layers are separated. The aqueous phase is extracted with ethyl acetate twice. The combined organic phases are washed twice with aqueous sodium bicarbonate solution, dried over anhydrous sodium sulfate, filtered, and the filtrate is concentrated under reduced pressure to obtain a product **compound 7-1** (185 mg, yield: 83%).

ESI(m/z) =307.1 [M+H]+.

Step 2: In a 50 mL single-necked flask, **compound 7-1** (185 mg, 0.60 mmol), THF (1 mL), methanol (1 mL), water (2 mL), and sodium hydroxide (29 mg, 0.72 mmol) are added sequentially. The mixture is stirred at room temperature for 4 h. The reaction mixture is concentrated under reduced pressure to remove the organic solvents. Water is added for dilution, and the pH is adjusted to 2-3 with dilute hydrochloric acid. The mixture is stirred for 30 min and then filtered. The filter cake is dried to obtain a product **compound 7-2** (150 mg, yield: 89%).

ESI(m/z) =279.0 [M+H]+.

In step 3: In a 50 mL single-necked flask, **compound 7-2** (100 mg, 0.36 mmol), **compound 7-3** (120 mg, 0.54 mmol), DMF (2 mL), *N,N*-diisopropylethylamine (DIPEA, 140 mg, 1.08 mmol), and 2-(7-azabenzotriazol-1-yl)-*N*,*N,N',N'*-tetramethyluronium hexafluorophosphate (HATU, 210 mg, 0.54 mmol) are added sequentially. The mixture is stirred at room temperature for 16 h. The reaction mixture is filtered and purified by reverse-phase preparative chromatography and preparative thin-layer chromatography (TLC) to obtain a product **compound 7** (42.1 mg, yield: 24.12%).

ESI(m/z) =486.3 [M+H]+.

¹H NMR (400 MHz, ) δ 8.51 (t, J = 5.9 Hz, 1H), 8.08 - 7.99 (m, 2H), 7.89 (td, J = 7.7, 1.4 Hz, 1H), 7.73 (td, J = 7.7, 1.1 Hz, 1H), 4.92 (s, 2H), 4.27 (s, 3H), 3.28 (t, J = 6.4 Hz, 6H), 2.84 - 2.54 (m, 3H), 2.36 (d, J = 24.3 Hz, 3H), 1.69 (ddd, J = 51.8, 41.2, 20.9 Hz, 7H), 1.35 - 0.96 (m, 6H).

### Example 8

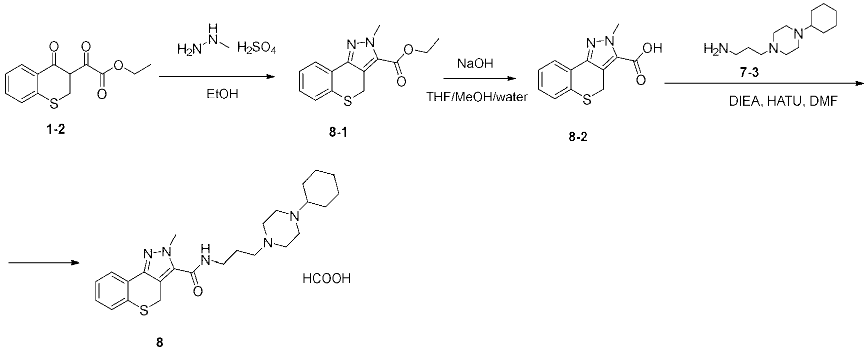

In step 1: In a 500 mL single-necked flask, **compound 1-2** (14 g, 52.97 mmol), ethanol (200 mL), and **methylhydrazine sulfate** (11.45 g, 79.45 mmol) are added sequentially. The mixture is stirred at 80°C for 6 h. The reaction mixture is cooled to room temperature, and the ethanol is removed by concentration under reduced pressure. Water and ethyl acetate are added, and the layers are separated. The aqueous phase is extracted with ethyl acetate twice. The combined organic phases are washed twice with aqueous sodium bicarbonate solution, dried over anhydrous sodium sulfate, filtered, and the filtrate is concentrated under reduced pressure. The residue is purified by column chromatography to obtain a product **compound 8-1** (1.2 g, yield: 8.26%).

ESI(m/z) =275.1 [M+H]+.

In step 2: In a 100 mL single-necked flask, **compound 8-1** (200 mg, 0.73 mmol), THF (1 mL), methanol (1 mL), water (2 mL), and sodium hydroxide (35 mg, 0.88 mmol) are added sequentially. The mixture is stirred at room temperature for 4 h. The reaction mixture is concentrated under reduced pressure to remove the organic solvents. Water is added for dilution, and the pH is adjusted to 2-3 with dilute hydrochloric acid. The mixture is stirred for 30 min and then filtered. The filter cake is dried to obtain a product **compound 8-2** (160 mg, yield: 89%).

ESI(m/z) =247.1 [M+H]+
In step 3: In a 50 mL single-necked flask, **compound 8-2** (100 mg, 0.41 mmol), **compound 7-3** (140 mg, 0.61 mmol), DMF (2 mL), *N,N*-diisopropylethylamine (DIPEA, 160 mg, 1.23 mmol), and HATU (230 mg, 0.61 mmol) are added sequentially. The mixture is stirred at room temperature for 16 h. The reaction mixture is filtered and purified by reverse-phase preparative chromatography to obtain a product **compound 8** (43.1 mg, yield: 21.24%).

ESI(m/z) =454.3 [M+H]+
¹H NMR (400 MHz, DMSO-d6) δ 8.40 (t, J = 5.6 Hz, 1H), 8.23 (s, 1H), 7.81 - 7.73 (m, 1H), 7.37 = 7.29 (m, 1H), 7.26 - 7.17 (m, 2H), 4.07 (s, 2H), 3.95 (s, 3H), 3.28 (q, J = 6.6 Hz, 2H), 2.58 (s, 4H), 2.49 - 2.21 (m, 7H), 1.84 - 1.62 (m, 6H), 1.55 (d, J = 12.3 Hz, 1H), 1.27 - 0.97 (m, 5H).

### Example 9

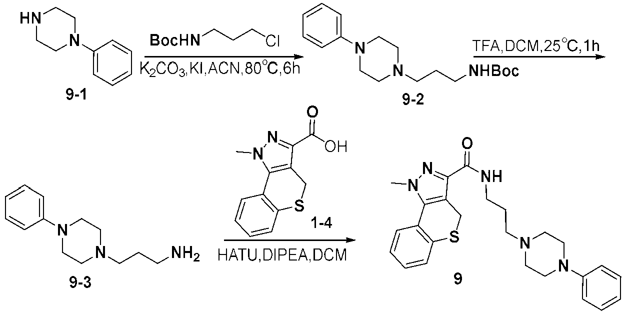

In step 1: **Compound 9-1** (0.8 g, 4.93 mmol), *N*-Boc-3-chloropropylamine (0.95 g, 4.93 mmol), K₂CO₃ (1.7 g, 12.32 mmol), and potassium iodide (0.41 g, 2.46 mmol) are dissolved in acetonitrile (8 mL). The reaction mixture is heated at 80°C for 6 h. LCMS shows that the reaction is complete. The reaction mixture is concentrated directly and purified by dry-column chromatography to obtain **compound 9-2** (0.8 g, yield: 50.79%). m/z [M+H]⁺ = 220

In step 2: **Compound 9-2** (0.8 g, 2.50 mmol) is dissolved in DCM (8 mL). Trifluoroacetic acid (TFA, 2 mL) is added, and the reaction mixture is stirred at 25°C for 1 h. LCMS shows that the reaction is complete. The reaction mixture is concentrated and purified by column chromatography to obtain **compound 9-3** (0.4 g, yield: 72.82%). m/z [M+H]⁺ = 220.3
In step 3: **Compound 1-4** (0.1 g, 0.41 mmol), **compound 9-3** (0.1 g, 0.45 mmol), and HATU (0.23 g, 0.61 mmol) are dissolved in DMF (1 mL). Then DIPEA (0.16 g, 1.23 mmol) is added. The reaction mixture is stirred at 25°C for 3 h. LCMS shows that the reaction is complete. The reaction mixture is purified directly by reverse-phase C₁₈ column chromatography (using 0.5% formic acid in water and acetonitrile) to obtain **compound 9** (23.2 mg, yield: 12.5%). m/z [M+H]⁺ = 448.4.

¹H NMR(400 MHz, DMSO-d6) 1H NMR (400 MHz, DMSO-d6) δ 8.52 (d,J = 5.5 Hz, 1H), 8.15 (s, 0H), 7.79 (dd,*J* = 7.6, 1.5 Hz, 1H), 7.50 (dd,*J* = 7.4, 1.6 Hz, 1H), 7.31 (pd,J = 7.4, 1.5 Hz, 2H), 7.23 - 7.17 (m, 2H), 6.94 (d,J = 8.0 Hz, 2H), 6.77 (t,*J* = 7.2 Hz, 1H), 4.19 (s, 2H), 4.01 (s, 3H), 3.33 (d,J = 6.0 Hz, 2H), 3.21 - 3.18 (m, 4H), 2.58 - 2.53 (m, 4H), 2.45 (t,*J* = 6.6 Hz, 2H), 1.72 (p,*J* = 6.5 Hz, 2H).

### Example 10

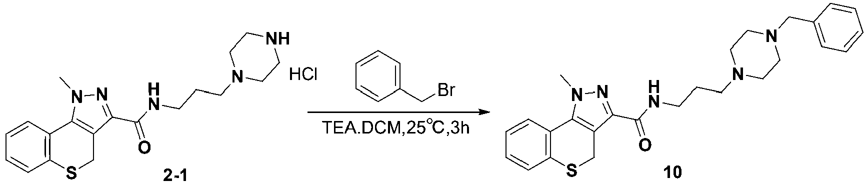

**Compound 2-1** (0.15 g, 0.37 mmol), benzyl bromide (0.063 g, 0.37 mmol), and TEA (0.19 g, 1.85 mmol) are dissolved in DCM (2 mL). The reaction mixture is stirred at 25°C for 3 h. LCMS shows that the reaction is complete. The mixture is then concentrated directly and purified by dry-column chromatography to obtain **compound 10** (58.8 mg, yield: 34.64%). m/z [M+H]⁺ = 462.3
¹H NMR(400 MHz, DMSO-d6) 1H NMR (400 MHz, DMSO-d6) δ 8.39 (t,*J* = 5.6 Hz, 1H), 7.84 (dd,*J* = 7.6, 1.7 Hz, 1H), 7.50 (dd,*J* = 7.5, 1.7 Hz, 1H), 7.35 - 7.28 (m, 6H), 7.24 (ddd,*J* = 8.6, 5.3, 2.2 Hz, 1H), 4.18 (s, 2H), 4.16 (s, 3H), 3.47 (s, 2H), 3.28 (q,*J* = 6.4 Hz, 2H), 2.51 (s, 1H), 2.43 (s, 5H), 2.38 (t,*J* = 6.8 Hz, 4H), 1.66 (p,*J* = 6.6 Hz, 2H).

### Example 11

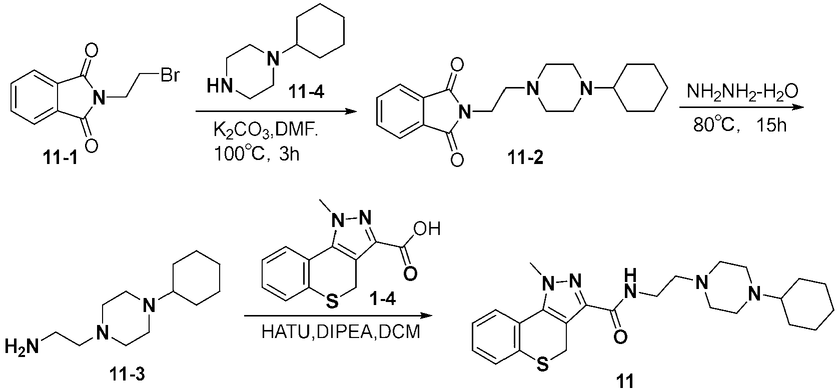

In step 1: **Compound 11-1** (0.8 g, 3.15 mmol), **compound 11-4** (0.53 g, 3.15 mmol), and K₂CO₃ (0.87 g, 6.3 mmol) are dissolved in DMF (8 mL). The reaction mixture is heated at 100°C for 3 h. LCMS shows that the reaction is complete. The mixture is then extracted, concentrated, and purified by dry-column chromatography to obtain **compound 11-2** (0.8 g, yield: 74.41%). m/z [M+H]⁺ = 342.2

In Step 2: **Compound 11-2** (0.8 g, 2.34 mmol) is dissolved in ethanol (8 mL). Hydrazine hydrate (0.31 g, 4.91 mmol) is added, and the reaction mixture is stirred at 80°C for 15 h. LCMS shows that the reaction is complete. The reaction mixture is filtered, and the filtrate is concentrated to obtain **compound 11-3** (0.3 g, yield: 60.58%). m/z [M+H]⁺ = 222.3.

In step 3: **Compound 1-4** (0.1 g, 0.41 mmol), **compound 11-3** (0.14 g, 0.49 mmol), and HATU (0.23 g, 0.61 mmol) are dissolved in DMF (1 mL). Then DIPEA (0.16 g, 1.23 mmol) is added. The reaction mixture is stirred at 25°C for 3 h. LCMS shows that the reaction is complete. The reaction mixture is purified directly by reverse-phase C₁₈ column chromatography (using 0.5% formic acid in water and acetonitrile) to obtain **compound 11** (31.6 mg, yield: 17.7%). m/z [M+H]⁺ = 440.4.

¹H NMR(400 MHz, DMSO-d6) ¹H NMR (400 MHz, DMSO-d6) δ 8.07 (s, 1H), 7.84 (dd,*J* = 7.5, 1.6 Hz, 1H), 7.51 (dd,*J* = 7.4, 1.6 Hz, 1H), 7.32 (dtd,*J* = 16.9, 7.4, 1.6 Hz, 2H), 4.18 (s, 2H), 4.13 (s, 3H), 3.23 (dd,*J* = 10.5, 2.7 Hz, 4H), 2.89 - 2.59 (m, 5H), 2.52 (s, 3H), 1.94 - 1.81 (m, 2H), 1.79 - 1.69 (m, 2H), 1.57 (d,*J* = 11.4 Hz, 1H), 1.22 (q,*J* = 10.3, 8.2 Hz, 5H), 1.14 - 1.01 (m, 1H).

### Example 12

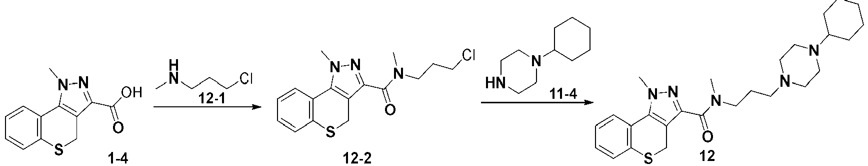

In step 1: **Compound 1-4** (0.2 g, 0.81 mmol), **compound 12-1** (0.1 g, 0.97 mmol), and HATU (0.4 g, 1.05 mmol) are dissolved in DMF (2 mL). Then DIPEA (0.31 g, 2.43 mmol) is added. The reaction mixture is stirred at 25°C for 3 h. LCMS shows that the reaction is complete. The reaction mixture is extracted, concentrated, and purified by dry-column chromatography to obtain **compound 12-2** (0.2 g, yield: 73.33%). m/z [M+H]+ = 336.2.

In step 2: **Compound 12-2** (0.1 g, 0.24 mmol), **compound 11-4** (0.04 g, 0.24 mmol), K₂CO₃ (0.083 g, 0.6 mmol), and potassium iodide (0.02 g, 0.12 mmol) are dissolved in acetonitrile (2 mL). The reaction mixture is heated at 80°C for 6 h. LCMS shows that the reaction is complete. The reaction mixture is concentrated and purified directly by reverse-phase C₁₈ column chromatography (using 0.5% formic acid in water and acetonitrile) to obtain **compound 12** (22 mg, yield: 19.75%). m/z [M+H]⁺= 468.4
¹H NMR(400 MHz, DMSO-d6) ¹H NMR (400 MHz, DMSO-d6) δ 7.62 - 7.57 (m, 1H), 7.49 (dd,*J* = 7.2, 1.9 Hz, 1H), 7.26 - 7.18 (m, 2H), 4.12 (d,*J* = 3.7 Hz, 3H), 4.09 (d,*J* = 4.6 Hz, 2H), 3.81 (t,*J* = 7.4 Hz, 1H), 3.55 (t,*J* = 7.4 Hz, 1H), 3.36 (s, 1H), 3.07 (s, 2H), 2.67 (d,*J* = 35.8 Hz, 7H), 2.44 (dt,J = 43.9, 7.2 Hz, 4H), 2.07 - 1.76 (m, 7H), 1.64 (s, 1H), 1.37 - 1.27 (m, 2H), 1.11 (d,*J* = 12.1 Hz, 2H).

### Example 13

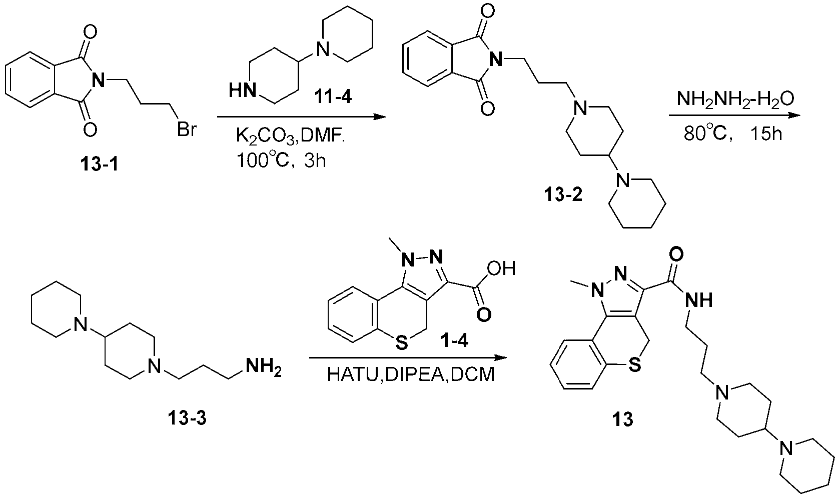

In step 1: **Compound 13-1** (0.5 g, 1.86 mmol), **compound 11-4** (0.31 g, 1.86 mmol), and K₂CO₃ (0.51 g, 3.72 mmol) are dissolved in DMF (5 mL). The reaction mixture is heated at 100°C for 3 h. LCMS shows that the reaction is complete. The mixture is then extracted, concentrated, and purified by dry-column chromatography to obtain **compound 13-2** (0.4 g, yield: 60.34%). m/z [M+H]⁺ = 342.2

In step 2: **Compound 13-2** (0.4 g, 1.13 mmol) is dissolved in ethanol (5 mL). Hydrazine hydrate (0.15 g, 2.37 mmol) is added, and the reaction mixture is stirred at 80°C for 15 h. LCMS shows that the reaction is complete. The reaction mixture is filtered, and the filtrate is concentrated to obtain **compound 13-3** (0.9 g, yield: 74.92%). m/z [M+H]⁺ = 226.3

In step 3: **Compound 1-4** (0.13 g, 0.53 mmol), **compound 13-3** (0.19 g, 1.21 mmol), and HATU (0.26 g, 0.69 mmol) are dissolved in DMF (2 mL). Then DIPEA (0.21 g, 1.59 mmol) is added. The reaction mixture is stirred at 25°C for 3 h. LCMS shows that the reaction is complete. The reaction mixture is purified directly by reverse-phase C₁₈ column chromatography (using 0.5% formic acid in water and acetonitrile) to obtain **compound 13** (14.2 mg, yield: 5.93%). m/z [M+H]⁺ = 440.4.

¹H NMR(400 MHz, DMSO-d6) ¹H NMR (400 MHz, DMSO-d6) δ 8.29 (s, 1H), 7.84 (dd,*J* = 7.6, 1.6 Hz, 1H), 7.51 (dd,*J* = 7.4, 1.6 Hz, 1H), 7.33 (dtd,*J* = 16.9, 7.4, 1.6 Hz, 2H), 4.19 (s, 2H), 4.14 (s, 3H), 3.27 (q,*J* = 6.5 Hz, 5H), 3.19 - 3.08 (m, 2H), 3.04 - 2.91 (m, 3H), 2.23 - 2.00 (m, 2H), 2.00 - 1.91 (m, 2H), 1.83 - 1.56 (m, 9H), 1.48 (s, 2H)

### Example 14

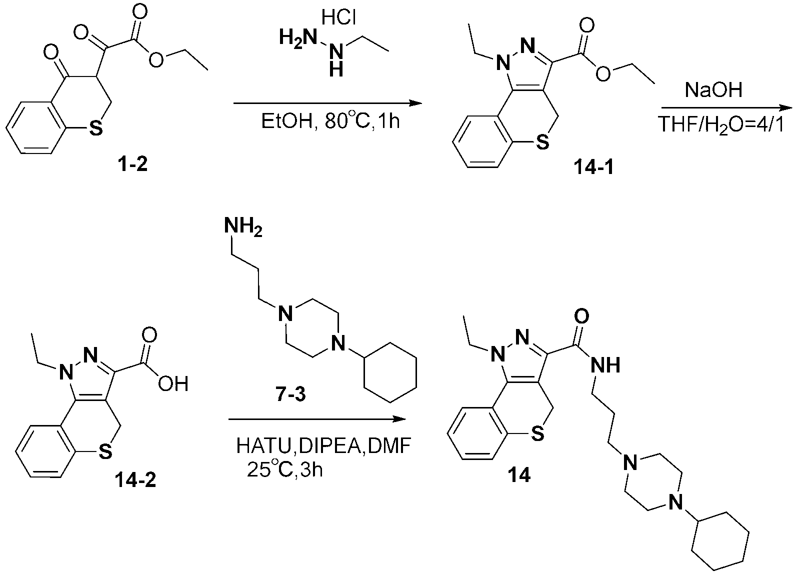

In step 1: **Compound 1-2** (1 g, 3.78 mmol) and ethyl hydrazine hydrochloride (0.23 g, 3.78 mmol) are dissolved in ethanol (10 mL). The reaction mixture is stirred at 80°C for 1 h. LCMS shows that the reaction is complete. The reaction mixture is concentrated, extracted, and purified by dry-column chromatography to obtain **compound 14-1** (0.6 g, yield: 44%). m/z [M-H]⁺ = 289.1

In step 2: **Compound 14-1** (0.2 g, 0.69 mmol) and NaOH (0.055 g, 1.38 mmol) are dissolved in a THF/H₂O (4/1, 2 mL) solution. The reaction mixture is stirred at 60°C for 1 h. LCMS shows that the reaction is complete. The pH of the reaction mixture is adjusted to 5, and the mixture is filtered. The filter cake is a product **compound 14-2** (0.15 g, yield: 83.03%). m/z [M+H]⁺ = 261.1.

In step 3: **Compound 14-2** (0.12 g, 0.46 mmol), **compound 7-3** (0.12 g, 0.55 mmol), and HATU (0.26 g, 0.69 mmol) are dissolved in DMF (1 mL). Then DIPEA (0.18 g, 1.38 mmol) is added. The reaction mixture is stirred at 25°C for 3 h. LCMS shows that the reaction is complete. The reaction mixture is purified directly by reverse-phase C₁₈ column chromatography (using 0.5% formic acid in water and acetonitrile) to obtain **compound 14** (22.2 mg, yield: 10.30%). m/z [M+H]⁺ = 468.4
¹H NMR(400 MHz, DMSO-d6) 1H NMR (400 MHz, DMSO-d6) δ 8.28 (t,*J* = 5.8 Hz, 1H), 7.72 (dd,*J* = 7.8, 1.5 Hz, 1H), 7.51 (dd,*J* = 7.6, 1.5 Hz, 1H), 7.35 (td,*J* = 7.6, 1.6 Hz, 1H), 7.30 (td,*J* = 7.5, 1.5 Hz, 1H), 4.46 (q,*J* = 7.2 Hz, 2H), 4.17 (s, 2H), 3.28 (q,*J* = 6.6 Hz, 4H), 2.71 (s, 4H), 2.59 - 2.51 (m, 2H), 2.42 (s, 3H), 1.90 - 1.77 (m, 2H), 1.71 (dp,*J* = 20.6, 6.9, 4.9 Hz, 4H), 1.56 (d,*J* = 12.4 Hz, 1H), 1.44 (t,*J* = 7.2 Hz, 3H), 1.25 - 1.16 (m, 4H), 1.15 - 1.00 (m, 1H).

### Example 15

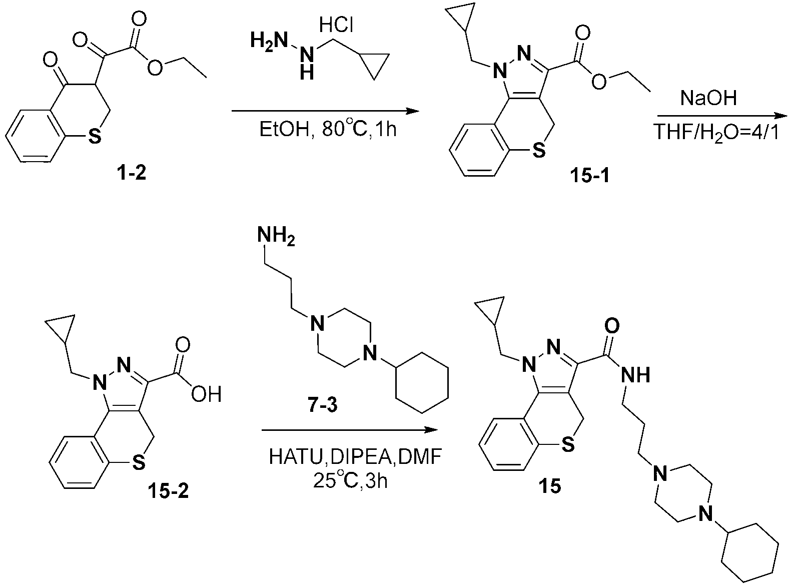

In step 1: **Compound 1-2** (0.8 g, 3.03 mmol) and cyclopropanehydrazide hydrochloride (0.31 g, 3.64 mmol) are dissolved in ethanol (8 mL). The reaction mixture is stirred at 80°C for 1 h. LCMS shows that the reaction is complete. The reaction mixture is concentrated, extracted, and purified by dry-column chromatography to obtain **compound 15-1** (0.6 g, yield: 63.05%). m/z [M-H]⁺ = 315.2

In step 2: **Compound 15-1** (0.23 g, 0.73 mmol) and NaOH (0.058 g, 1.46 mmol) are dissolved in a THF/H₂O (4/1, 3 mL) solution. The reaction mixture is stirred at 60°C for 1 h. LCMS shows that the reaction is complete. The pH of the reaction mixture is adjusted to 5, and the mixture is filtered. The filter cake is a product **compound 15-2** (0.13 g, yield: 62.06%). m/z [M+H]⁺ = 287.1.

In step 3: **Compound 15-2** (0.1 g, 0.35 mmol), **compound 7-3** (0.13 g, 0.42 mmol), and HATU (0.17 g, 0.45 mmol) are dissolved in DMF (1 mL). Then DIPEA (0.14 g, 1.05 mmol) is added. The reaction mixture is stirred at 25°C for 3 h. LCMS shows that the reaction is complete. The reaction mixture is purified directly by reverse-phase C₁₈ column chromatography (using 0.5% formic acid in water and acetonitrile) to obtain **compound 15** (59.3 mg, yield: 34.39%). m/z [M+H]⁺ = 468.4

¹H NMR(400 MHz, DMSO-d6) δ 8.26 (d,*J* = 5.6 Hz, 1H), 7.83 (d,*J* = 7.8 Hz, 1H), 7.51 (dd,*J* = 7.7, 1.5 Hz, 1H), 7.32 (dtd,*J* = 23.5, 7.5, 1.4 Hz, 2H), 4.35 (d,*J* = 6.6 Hz, 2H), 4.17 (s, 2H), 3.28 (q,*J* = 6.5 Hz, 2H), 2.56 (s, 4H), 2.44 (d,*J* = 21.5 Hz, 3H), 2.35 (t,*J* = 6.6 Hz, 3H), 2.29 - 2.18 (m, 1H), 1.77 (d,*J* = 7.3 Hz, 2H), 1.68 (dt,*J* = 13.3, 7.7 Hz, 4H), 1.55 (d,*J* = 11.6 Hz, 1H), 1.33 - 1.24 (m, 1H), 1.16 (d,*J* = 10.2 Hz, 4H), 1.05 (d,J = 11.6 Hz, 1H), 0.54 - 0.47 (m, 2H), 0.35 (q,J = 6.0, 5.3 Hz, 2H).

### Example 16

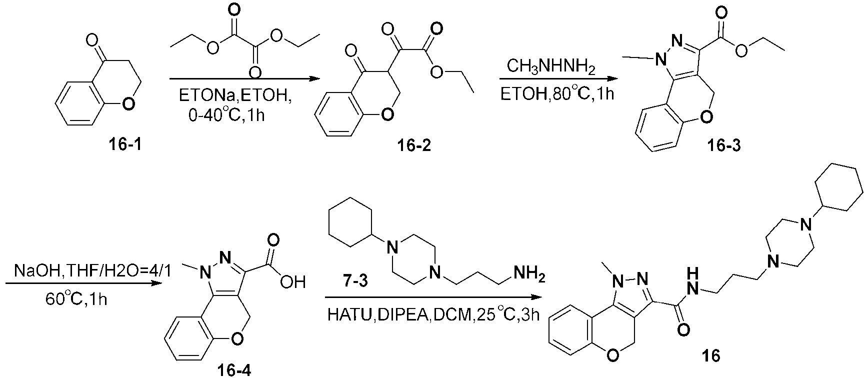

In step 1: **Compound 16-1** (1 g, 6.75 mmol) and sodium ethoxide (0.51 g, 7.43 mmol) are dissolved in ethanol (15 mL). The mixture is stirred at 0°C for 0.5 h. Then diethyl oxalate (0.99 g, 6.75 mmol) is added, and the mixture is stirred at 40°C for 1 h. LCMS shows that the reaction is complete. The reaction mixture is concentrated, extracted, and purified by dry-column chromatography to obtain **compound 16-2** (1 g, yield: 35.81%). m/z [M-H]⁺ = 247.0

In step 2: **Compound 16-2** (1 g, 4.03 mmol) and methylhydrazine sulfate (0.7 g, 4.84 mmol) are dissolved in ethanol (10 mL). The reaction mixture is stirred at 80°C for 1 h. LCMS shows that the reaction is complete. The reaction mixture is concentrated and purified by column chromatography to obtain **compound 16-3** (0.6 g, yield: 57.67%). m/z [M+H]⁺ = 259.3.

In step 3: **Compound 16-3** (0.2 g, 0.77 mmol) and NaOH (0.062 g, 1.54 mmol) are dissolved in a THF/H₂O (4/1, 2 mL) solution. The reaction mixture is stirred at 60°C for 1 h. LCMS shows that the reaction is complete. The pH of the reaction mixture is adjusted to 5, and the mixture is filtered. The filter cake is a product **compound 16-4** (0.1 g, yield: 56.09%). m/z [M+H]⁺ = 231.1.

In step 4: **Compound 16-4** (0.12 g, 0.52 mmol), **compound 7-3** (0.14 g, 0.62 mmol), and HATU (0.3 g, 0.78 mmol) are dissolved in DMF (1 mL). Then DIPEA (0.2 g, 1.56 mmol) is added. The reaction mixture is stirred at 25°C for 3 h. LCMS shows that the reaction is complete. The reaction mixture is purified directly by reverse-phase C₁₈ column chromatography (using 0.5% formic acid in water and acetonitrile) to obtain **compound 16** (63 mg, yield: 27.62%). m/z [M+H]⁺ = 438.4

¹H NMR(400 MHz, DMSO-d6) δ 8.36 - 8.30 (m, 1H), 7.72 (d,*J* = 7.7 Hz, 1H), 7.28 (t,*J* = 7.7 Hz, 1H), 7.07 (t,*J* = 7.4 Hz, 1H), 7.01 (d,*J* = 6.5 Hz, 1H), 5.39 (d,*J* = 1.8 Hz, 2H), 4.15 (d,*J* = 2.4 Hz, 3H), 3.26 (q,*J* = 5.7 Hz, 2H), 2.60 (s, 4H), 2.43 (s, 3H), 2.34 (q,*J* = 14.8, 10.8 Hz, 4H), 1.79 (s, 2H), 1.71 (s, 2H), 1.68 - 1.61 (m, 2H), 1.55 (d,*J* = 11.9 Hz, 1H), 1.18 (t,*J* = 8.9 Hz, 4H), 1.11 - 1.00 (m, 1H).

### Example 17

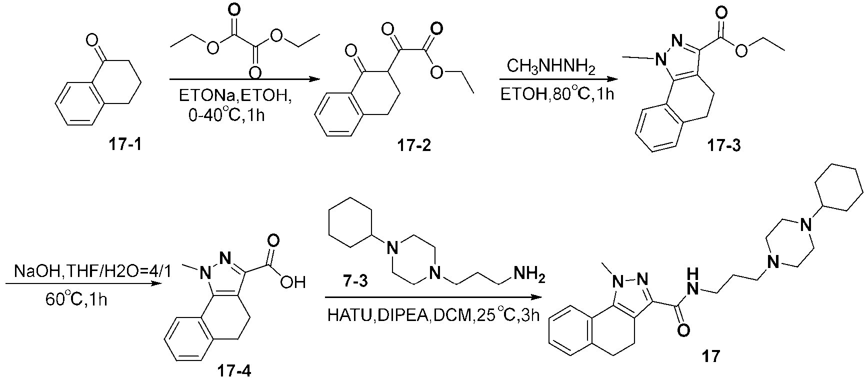

In step 1: **Compound 17-1** (5 g, 34.2 mmol) and sodium ethoxide (2.44 g, 35.91 mmol) are dissolved in ethanol (100 mL). The mixture is stirred at 0°C for 0.5 h. Then diethyl oxalate (5 g, 34.2 mmol) is added, and the mixture is stirred at 40°C for 1 h. LCMS shows that the reaction is complete. The reaction mixture is concentrated, extracted, and purified by dry-column chromatography to obtain **compound 17-2** (8.5 g, yield: 86.35%). m/z [M-H]⁺ = 247.2

In step 2: **Compound 17-2** (2 g, 5.69 mmol) and methylhydrazine sulfate (1.64 g, 11.38 mmol) are dissolved in ethanol (10 mL). The reaction mixture is stirred at 25°C for 16 h. LCMS shows that the reaction is complete. The reaction mixture is concentrated and purified by column chromatography to obtain **compound 17-3** (0.55 g, yield: 37.75%). m/z [M+H]⁺ = 257.2.

In step 3: **Compound 17-3** (0.25 g, 0.98 mmol) and KOH (0.33 g, 5.88 mmol) are dissolved in a MeOH/H₂O (4/1, 2 mL) solution. The reaction mixture is stirred at 60°C for 1 h. LCMS shows that the reaction is complete. The pH of the reaction mixture is adjusted to 5, and the mixture is filtered. The filter cake is a product **compound 17-4** (0.1 g, yield: 56.09%). m/z [M+H]⁺ = 229.2.

In step 4: **Compound 17-4** (0.12 g, 0.52 mmol), **compound 7-3** (0.14 g, 0.62 mmol), and HATU (0.3 g, 0.78 mmol) are dissolved in DMF (1 mL). Then DIPEA (0.2 g, 1.56 mmol) is added. The reaction mixture is stirred at 25°C for 3 h. LCMS shows that the reaction is complete. The reaction mixture is purified directly by reverse-phase C₁₈ column chromatography (using 0.5% formic acid in water and acetonitrile) to obtain **compound 17** (50.8 mg, yield: 28.62%, formate). m/z [M+H]⁺ = 436.4

¹H NMR(400 MHz, DMSO-d6) 8.25 (s, 1H), 8.16 (t, *J* = 5.5 Hz, 1H), 7.72 (d, *J* = 7.6 Hz, 1H), 7.39 - 7.32 (m, 2H), 7.28 (t, *J =* 7.3 Hz, 1H), 4.15 (s, 3H), 3.26 (q, *J =* 6.2 Hz, 2H), 2.89 (d, *J* = 6.9 Hz, 2H), 2.84 (d, J= 6.7 Hz, 2H), 2.65 (s, 4H), 2.48 (s, 2H), 2.38 (t, *J =* 6.7 Hz, 4H), 1.85 - 1.50 (m, 8H), 1.19 (p, *J* = 11.7 Hz, 4H), 1.11 - 0.99 (m, 1H).

### Example 18

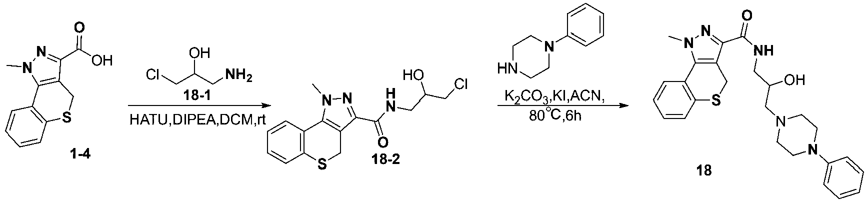

In step 1: **Compound 1-4** (0.14 g, 0.57 mmol), **compound 18-1** (0.075 g, 0.68 mmol), and HATU (0.28 g, 0.74 mmol) are dissolved in DMF (2 mL). Then DIPEA (0.22 g, 1.71 mmol) is added. The reaction mixture is stirred at 25°C for 3 h. LCMS shows that the reaction is complete. The reaction mixture is extracted, concentrated, and purified by dry-column chromatography to obtain **compound 18-2** (0.18 g, yield: 92.3%). m/z [M+H]⁺ = 338.1.

In step 2: **Compound 18-2** (0.18 g, 0.53 mmol), **N-phenylpiperazine** (0.086 g, 0.53 mmol), K₂CO₃ (0.18 g, 1.33 mmol), and potassium iodide (0.044 g, 0.27 mmol) are dissolved in acetonitrile (2 mL). The reaction mixture is heated at 80°C for 6 h. LCMS shows that the reaction is complete. The reaction mixture is concentrated and purified directly by reverse-phase C₁₈ column chromatography (using 0.5% formic acid in water and acetonitrile) to obtain **compound 18** (63 mg, yield: 25.5%). m/z [M+H]⁺ = 464.3

¹H NMR(400 MHz, DMSO-d6) δ 8.26 (t,J = 5.6 Hz, 1H), 7.80 (dd,J = 7.4, 1.8 Hz, 1H), 7.50 (dd,J = 7.1, 1.8 Hz, 1H), 7.31 (pd,J = 7.4, 1.6 Hz, 2H), 7.20 (t,J = 7.7 Hz, 2H), 6.93 (d,*J* = 8.2 Hz, 2H), 6.76 (t,J = 7.2 Hz, 1H), 4.94 (s, 1H), 4.19 (s, 2H), 4.02 (s, 3H), 3.84 (t,J = 6.1 Hz, 1H), 3.34 - 3.29 (m, 2H), 3.18 (t,J = 5.0 Hz, 4H), 2.63 (dt,J = 10.2, 5.0 Hz, 2H), 2.57 (q,J = 5.8, 5.4 Hz, 2H), 2.42 (td,J = 12.5, 10.9, 6.2 Hz, 2H).

### Example 19

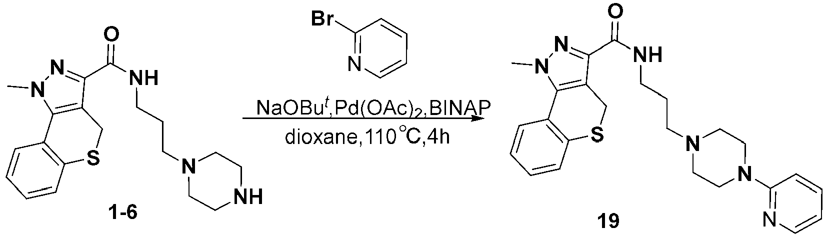

**Compound 1-6** (0.1 g, 0.27 mmol), 2-bromopyridine (0.043 g, 0.27 mmol), palladium acetate (6 mg, 0.027 mmol), (1,1'-binaphthalene)-2,2'-diylbis(diphenylphosphine) (BINAP, 34 mg, 0.054 mmol), and sodium tert-butoxide (78 mg, 0.81 mmol) are dissolved in dioxane (2 mL). The reaction mixture is heated at 110°C for 4 h. LCMS shows that the reaction is complete. The mixture is concentrated directly and purified by reverse-phase C₁₈ column chromatography (using 0.5% formic acid in water and acetonitrile) to obtain **compound 19** (33.5 mg, yield: 27.74%). m/z [M+H]⁺ = 449.3

¹H NMR(400 MHz, DMSO-d6) δ 8.55 (t,J = 5.6 Hz, 1H), 8.11 (dd,J = 5.0, 1.9 Hz, 1H), 7.80 (dd,J = 7.3, 1.9 Hz, 1H), 7.55 - 7.47 (m, 2H), 7.31 (pd,J = 7.4, 1.7 Hz, 2H), 6.82 (d,J = 8.6 Hz, 1H), 6.63 (dd,J = 7.1, 4.9 Hz, 1H), 4.19 (s, 2H), 4.03 (s, 3H), 3.53 (t,J = 5.0 Hz, 5H), 3.32 - 3.30 (m, 3H), 2.48 (s, 2H), 2.43 (t,J = 6.6 Hz, 2H), 1.72 (p,J = 6.7 Hz, 2H).

### Example 20

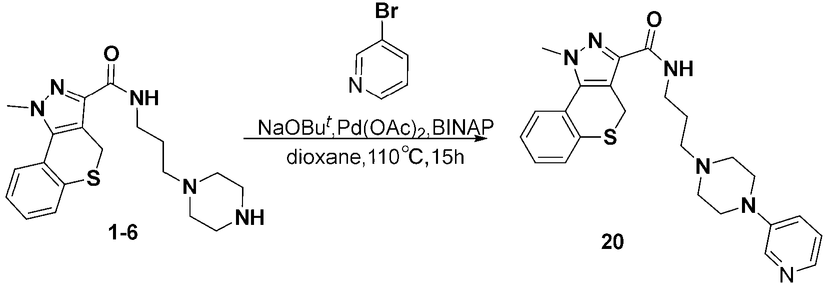

**Compound 1-6** (0.1 g, 0.27 mmol), 3-bromopyridine (0.043 g, 0.27 mmol), palladium acetate (6 mg, 0.027 mmol), BINAP (34 mg, 0.054 mmol), and sodium tert-butoxide (78 mg, 0.81 mmol) are dissolved in dioxane (2 mL). The reaction mixture is heated at 110°C for 15 h. LCMS shows that the reaction is complete. The mixture is concentrated directly and purified by reverse-phase C₁₈ column chromatography (using 0.5% formic acid in water and acetonitrile) to obtain **compound 20** (9.9 mg, yield: 8.2%). m/z [M+H]⁺ = 449.3
¹H NMR(400 MHz, DMSO-d6) δ 8.51 (t,J = 5.6 Hz, 1H), 8.31 (d,J = 2.9 Hz, 1H), 8.01 - 7.97 (m, 1H), 7.82 - 7.77 (m, 1H), 7.50 (dd,J = 7.4, 1.7 Hz, 1H), 7.34 - 7.28 (m, 3H), 7.20 (dd,J = 8.5, 4.5 Hz, 1H), 4.19 (s, 2H), 4.02 (s, 3H), 3.32 - 3.29 (m, 3H), 3.29 - 3.22 (m, 4H), 2.55 (s, 3H), 2.48 - 2.40 (m, 2H), 1.72 (p,J = 6.3 Hz, 2H).

### Example 21

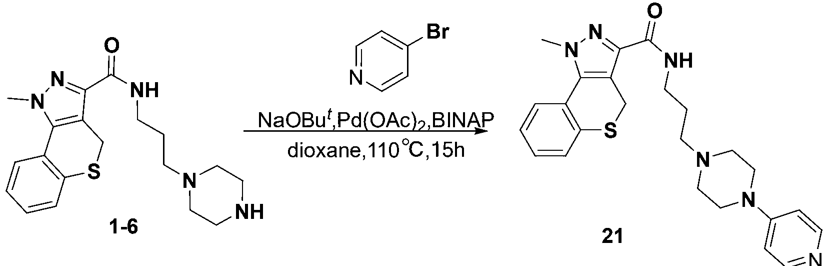

**Compound 1-6** (0.1 g, 0.27 mmol), 4-bromopyridine (0.043 g, 0.27 mmol), palladium acetate (6 mg, 0.027 mmol), BINAP (34 mg, 0.054 mmol), and sodium tert-butoxide (78 mg, 0.81 mmol) are dissolved in dioxane (2 mL). The reaction mixture is heated at 110°C for 15 h. LCMS shows that the reaction is complete. The mixture is concentrated directly and purified by preparative TLC to obtain **compound 21** (29.8 mg, yield: 27.1%). m/z [M+H]⁺ = 449.3

¹H NMR(400 MHz, DMSO-d6) δ 8.50 (t, *J* = 5.6 Hz, 1H), 8.16 (d, *J* = 5.9 Hz, 2H), 7.80 (dd, J = 7.4, 1.8 Hz, 1H), 7.50 (dd, J = 7.3, 1.9 Hz, 1H), 7.35 - 7.27 (m, 2H), 6.88 (d, *J* = 6.0 Hz, 2H), 4.19 (s, 2H), 4.03 (s, 3H), 3.43 (d, *J* = 4.7 Hz, 3H), 3.34 - 3.29 (m, 4H), 2.52 (s, 3H), 2.43 (t, *J =* 6.7 Hz, 2H), 1.70 (q, *J* = 6.7 Hz, 2H).

### Example 22

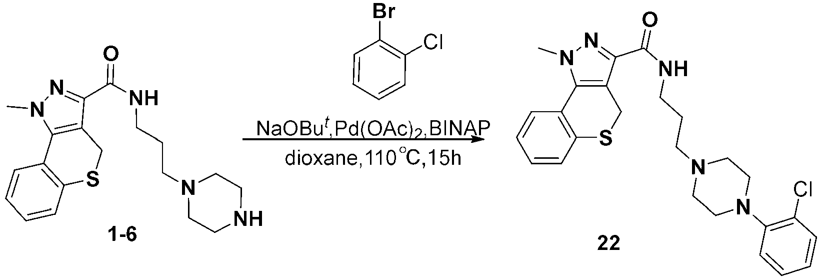

**Compound 1-6** (0.1 g, 0.27 mmol), 2-bromo-1-chlorobenzene (0.043 g, 0.27 mmol), palladium acetate (6 mg, 0.027 mmol), BINAP (34 mg, 0.054 mmol), and sodium tert-butoxide (78 mg, 0.81 mmol) are dissolved in dioxane (2 mL). The reaction mixture is heated at 110°C for 15 h. LCMS shows that the reaction is complete. The mixture is concentrated directly and purified by reverse-phase C₁₈ column chromatography (using 0.5% formic acid in water and acetonitrile) to obtain **compound 22** (27 mg, yield: 20.77%). m/z [M+H]⁺ = 449.3

¹H NMR(400 MHz, DMSO-d6) δ 8.49 (t,J = 5.6 Hz, 1H), 7.83 (dd,J = 7.6, 1.7 Hz, 1H), 7.50 (dd,J = 7.4, 1.7 Hz, 1H), 7.40 (dd,J = 7.9, 1.5 Hz, 1H), 7.31 (dddd,J = 16.0, 8.9, 7.4, 1.6 Hz, 3H), 7.17 (dd,J = 8.1, 1.6 Hz, 1H), 7.03 (td,J = 7.6, 1.5 Hz, 1H), 4.19 (s, 2H), 4.11 (s, 3H), 3.33 (s, 3H), 3.05 (d,J = 4.8 Hz, 4H), 2.59 (s, 3H), 2.47 (d,J = 6.6 Hz, 2H), 1.72 (t,J = 6.6 Hz, 2H).

### Example 23

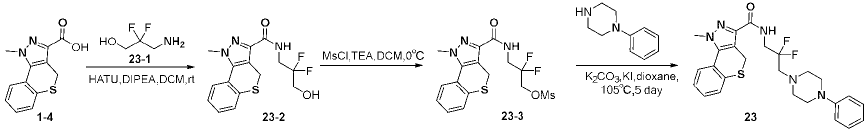

In step 1: **Compound 1-4** (0.15 g, 0.61 mmol), **compound 23-1** (0.086 g, 0.73 mmol), and HATU (0.3 g, 0.79 mmol) are dissolved in DCM (2 mL). Then DIPEA (0.24 g, 1.83 mmol) is added. The reaction mixture is stirred at 25°C for 3 h. LCMS shows that the reaction is complete. The reaction mixture is extracted, concentrated, and purified by dry-column chromatography to obtain **compound 23-2** (0.18 g, yield: 87.09%). m/z [M+H]⁺ = 340.1.

In step 2: **Compound 23-2** (0.27 g, 0.8 mmol), methanesulfonyl chloride (MsCl, 0.11 g, 0.96 mmol), and TEA (0.16 g, 1.6 mmol). LCMS shows that the reaction is complete. The reaction mixture is concentrated and purified by column chromatography to obtain **compound 23-3** (0.25 g, yield: 75.27%). m/z [M+H]⁺ = 418.2.

In step 3: **Compound 23-3** (0.15 g, 0.36 mmol), **N-phenylpiperazine** (0.088 g, 0.54 mmol), K₂CO₃ (0.15 g, 1.08 mmol), and potassium iodide (0.048 g, 0.29 mmol) are dissolved in dioxane (3 mL). The reaction mixture is heated at 105°C for 5 d. LCMS shows that the reaction is complete. The reaction mixture is concentrated and purified directly by reverse-phase C₁₈ column chromatography (using 0.5% formic acid in water and acetonitrile) to obtain **compound 23** (5.2 mg, yield: 2.99%). m/z [M+H]⁺ = 484.3.

¹H NMR(400 MHz, DMSO-d6) δ 8.42 (t, J = 6.2 Hz, 1H), 7.84 - 7.78 (m, 1H), 7.51 (dd, *J* = 7.5, 1.7 Hz, 1H), 7.32 (pd, *J =* 7.4*,* 1.7 Hz, 2H), 7.24 - 7.17 (m, 2H), 6.94 (d, *J = 7.9* Hz, 2H), 6.80 - 6.75 (m, 1H), 4.19 (s, 2H), 4.05 (s, 3H), 3.86 (td, J = 14.4, 6.3 Hz, 2H), 3.22 - 3.16 (m, 4H), 2.92 (t, *J* = 14.0 Hz, 2H), 2.73 (t, *J* = 4.9 Hz, 4H).

### Example 24

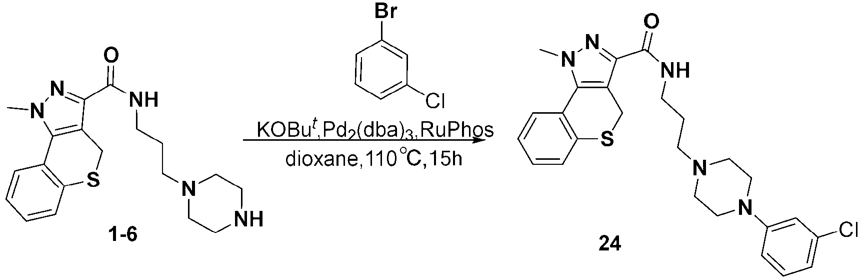

**Compound 1-6** (0.1 g, 0.27 mmol), 1-bromo-3-chlorobenzene (0.062 g, 0.32 mmol), tris(dibenzylideneacetone) dipalladium (0) (Pd₂(dba)₃, 25 mg, 0.027 mmol), RuPhos (25 mg, 0.054 mmol), and potassium tert-butoxide (91 mg, 0.81 mmol) are dissolved in dioxane (2 mL). The reaction mixture is heated at 110°C for 5 h. LCMS shows that the reaction is complete. The mixture is concentrated directly and purified by reverse-phase C₁₈ column chromatography (using 0.5% formic acid in water and acetonitrile) to obtain **compound 24** (32 mg, yield: 24.66%). m/z [M+H]⁺ = 482.3.

¹H NMR(400 MHz, DMSO-d6) δ 8.50 (t,J = 5.6 Hz, 1H), 7.80 (dd,J = 7.4, 1.8 Hz, 1H), 7.50 (dd,J = 7.3, 1.8 Hz, 1H), 7.31 (pd, *J* = 7.4, 1.6 Hz, 2H), 7.20 (t,J = 8.1 Hz, 1H), 6.95 (t,J = 2.2 Hz, 1H), 6.90 (dd,J = 8.4, 2.4 Hz, 1H), 6.78 (dd,J = 7.8, 1.8 Hz, 1H), 4.19 (s, 2H), 4.03 (s, 3H), 3.23 (d,J = 5.4 Hz, 8H), 2.55 (s, 3H), 2.46 (s, 1H), 1.72 (p,J = 6.8 Hz, 2H).

### Example 25

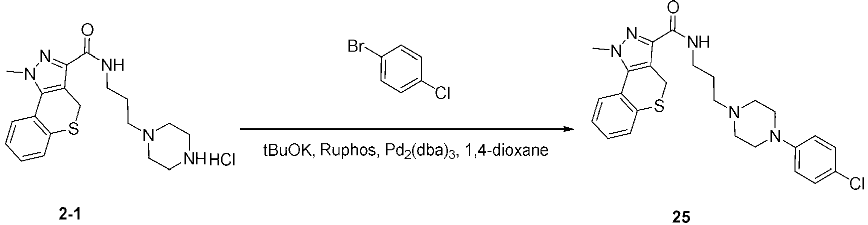

In an 8 mL single-necked flask, **compound 2-1** (100 mg, 0.25 mmol), p-chlorobromobenzene (57 mg, 0.3 mmol), 1,4-dioxane (2 mL), and potassium tert-butoxide (93 mg, 0.82 mmol) are added sequentially. Under a nitrogen atmosphere, 2-dicyclohexylphosphino-2',6'-diisopropoxy-1,1'-biphenyl (12 mg, 0.025 mmol) and tris(dibenzylideneacetone)dipalladium (11 mg, 0.013 mmol) are added. The mixture is stirred at 105°C under a nitrogen atmosphere for 16 h. The reaction mixture is cooled to room temperature and filtered. The filtrate is concentrated under reduced pressure and purified by reverse-phase preparative chromatography to obtain a product **compound 25** (42.8 mg, yield: 36.22%). ESI(m/z) =482.3 [M+H]⁺.

¹H NMR (400 MHz, DMSO-d6) δ 8.53 (t, J = 5.6 Hz, 1H), 7.84 - 7.76 (m, 1H), 7.53 - 7.47 (m, 1H), 7.31 (pd, J = 7.4, 1.7 Hz, 2H), 7.25 - 7.18 (m, 2H), 7.00 - 6.90 (m, 2H), 4.19 (s, 2H), 4.02 (s, 3H), 3.19 (t, *J =* 5.0 Hz, 6H), 2.53 (t, *J =* 5.0 Hz, 4H), 2.44 (t, *J = 6.6* Hz, 2H), 1.71 (p, *J* = 6.6 Hz, 2H).

### Example 26

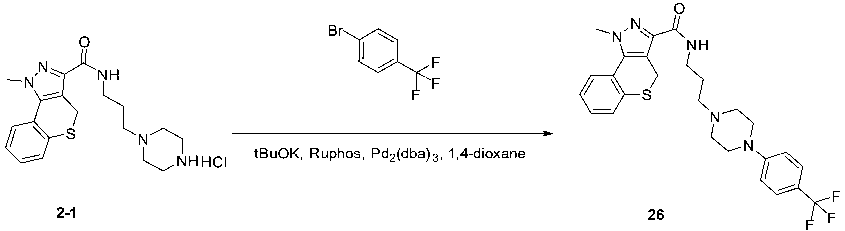

In an 8 mL single-necked flask, **compound 2-1** (100 mg, 0.25 mmol), p-bromobenzotrifluoride (68 mg, 0.3 mmol), 1,4-dioxane (2 mL), and potassium tert-butoxide (93 mg, 0.82 mmol) are added sequentially. Under a nitrogen atmosphere, 2-dicyclohexylphosphino-2',6'-diisopropoxy-1,1'-biphenyl (12 mg, 0.025 mmol) and tris(dibenzylideneacetone)dipalladium (11 mg, 0.013 mmol) are added. The mixture is stirred at 105°C under a nitrogen atmosphere for 16 h. The reaction mixture is cooled to room temperature and filtered. The filtrate is concentrated under reduced pressure and purified by reverse-phase preparative chromatography to obtain a product **compound 26** (36.2 mg, yield: 28.6%). ESI(m/z) =516.3 [M+H]⁺.

¹H NMR (400 MHz, DMSO-d6) δ 8.50 (t, *J* = 5.6 Hz, 1H), 7.80 (dd, *J* = 7.3, 1.9 Hz, 1H), 7.54 - 7.44 (m, 3H), 7.31 (pd, J = 7.4, 1.7 Hz, 2H), 7.07 (d, *J* = 8.6 Hz, 2H), 4.19 (s, 2H), 4.03 (s, 3H), 3.31 (s, 6H), 2.53 (t, *J =* 5.1 Hz, 4H), 2.43 (t, *J* = 6.6 Hz, 2H), 1.72 (p, *J* = 6.7 Hz, 2H).

### Example 27

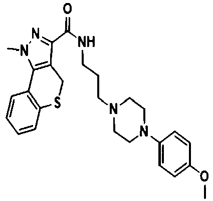

Referring to the synthesis method of Example 26, **compound 27** is synthesized by replacing p-bromobenzotrifluoride with

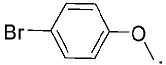

¹H NMR (400 MHz, DMSO-d6) δ 8.54 (t, J = 5.6 Hz, 1H), 8.18 (s, 0.42H), 7.83 (dd, J = 7.3, 1.9 Hz, 1H), 7.53 (dd, J = 7.2, 1.9 Hz, 1H), 7.41 - 7.27 (m, 2H), 6.98 - 6.88 (m, 2H), 6.87 - 6.77 (m, 2H), 4.22 (s, 2H), 4.06 (s, 3H), 3.71 (s, 3H), 3.35 (d, *J =* 6.0 Hz, 2H), 3.11 (t, *J = 4.9* Hz, 4H), 2.59 (d, *J* = 4.8 Hz, 4H), 2.48 (t, *J* = 6.6 Hz, 2H), 1.79 - 1.67 (m, 2H).

### Example 28

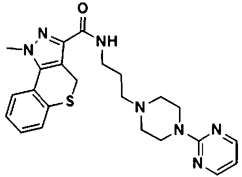

Referring to the synthesis method of Example 26, **compound 28** is synthesized by replacing p-bromobenzotrifluoride with

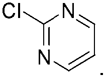

¹H NMR (400 MHz, DMSO-d6) δ 8.56 (t, *J* = 5.7 Hz, 1H), 8.39 (d, *J* = 4.7 Hz, 2H), 7.85 (d, *J* = 7.5 Hz, 1H), 7.53 (dd, *J =* 7.0, 1.8 Hz, 1H), 7.40 - 7.26 (m, 2H), 6.66 (t, *J* = 4.7 Hz, 1H), 4.22 (s, 2H), 4.10 (s, 3H), 3.80 (s, 4H), 3.34 (d, *J* = 8.2 Hz, 2H), 2.48 (s, 4H), 1.75 (s, 2H), 1.26 (s, 2H).

### Example 29

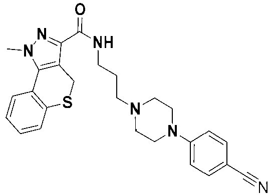

Referring to the synthesis method of Example 26, **compound 29** is synthesized by replacing p-bromobenzotrifluoride with

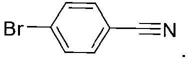

1H NMR (400 MHz, DMSO-d6) δ 8.52 (t, *J* = 5.6 Hz, 1H), 7.81 (dd, *J* = 7.4, 1.9 Hz, 1H), 7.60 - 7.56 (m, 2H), 7.50 (dd, *J =* 7.2, 1.9 Hz, 1H), 7.31 (pd, J = 7.4, 1.7 Hz, 2H), 7.08 - 7.01 (m, 2H), 4.19 (s, 2H), 4.03 (s, 3H), 3.43 - 3.36 (m, 5H), 3.30 (s, 3H), 2.55 (s, 3H), 2.46 (s, 1H), 1.72 (p, *J =* 6.2, 5.8 Hz, 2H).

### Example 30

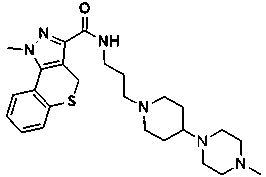

Referring to the synthesis method of Example 13, **compound 30** is synthesized by replacing **compound 11-4** with

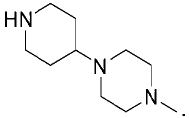

¹H NMR (400 MHz, DMSO-*d*6) δ 8.47 (t, *J* = 6.1 Hz, 1H), 7.88 (dd, *J* = 7.6, 1.6 Hz, 1H), 7.54 (dd, *J* = 7.5, 1.6 Hz, 1H), 7.43 - 7.32 (m, 2H), 4.22 (s, 2H), 4.18 (s, 3H), 3.62 - 3.40 (m, 6H), 3.36 - 3.30 (m, 2H), 3.22 - 2.91 (m, 8H), 2.81 (s, 3H), 2.49 (s, 1H), 2.40 (s, 1H), 1.93 (s, 4H), 1.61 - 1.51 (m, 1H).

### Example 31

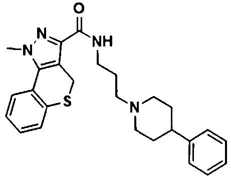

Referring to the synthesis method of Example 13, **compound 31** is synthesized by replacing **compound 11-4** with

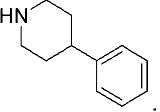

¹H NMR (400 MHz, DMSO-d6) δ 8.45 (t, *J* = 5.9 Hz, 1H), 7.84 (dd, *J* = 7.6, 1.6 Hz, 1H), 7.51 (dd, *J = 7.4,* 1.7 Hz, 1H), 7.37 - 7.29 (m, 4H), 7.26 - 7.20 (m, 3H), 4.20 (s, 2H), 4.11 (s, 3H), 3.34 - 3.28 (m, 6H), 2.76 - 2.59 (m, 2H), 2.04 - 1.68 (m, 7H).

### Example 32

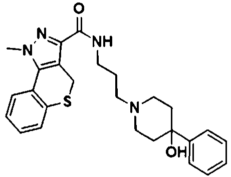

Referring to the synthesis method of Example 13, **compound 32** is synthesized by replacing **compound 11-4** with

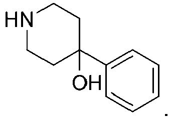

¹H NMR (400 MHz, DMSO-*d*6) δ 8.46 (t, *J* = 5.9 Hz, 1H), 7.84 (dd, *J* = 7.6, 1.6 Hz, 1H), 7.53 - 7.45 (m, 3H), 7.40 - 7.30 (m, 4H), 7.29 - 7.24 (m, 1H), 4.20 (s, 2H), 4.13 (s, 3H), 3.32 (d, *J =* 6.4 Hz, 4H), 3.08 (s, 4H), 2.17 (d, *J =* 14.2 Hz, 2H), 1.92 (s, 2H), 1.83 - 1.75 (m, 2H).

### Example 33

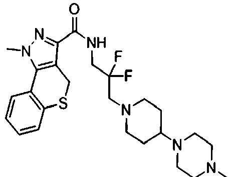

Referring to the synthesis method of Example 23, **compound 33** is synthesized by replacing 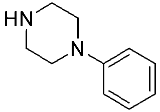 with

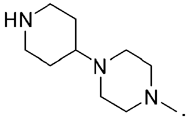

¹H NMR (400 MHz, ) δ 8.42 (t, *J =* 6.1 Hz, 1H), 8.20 (s, 1H), 7.86 (dd, *J* = 7.5, 1.6 Hz, 1H), 7.51 (dd, *J* = 7.4, 1.7 Hz, 1H), 7.33 (dtd, *J* = 16.8, 7.4, 1.6 Hz, 2H), 4.19 (s, 2H), 4.17 (s, 3H), 3.81 (td, *J =* 14.2, 6.1 Hz, 4H), 2.98 (d, *J =* 11.3 Hz, 2H), 2.82 (t, J = 13.9 Hz, 2H), 2.34 (s, 6H), 2.21 - 2.04 (m, 6H), 1.74 (d, *J =* 11.7 Hz, 2H), 1.59 - 1.42 (m, 2H).

### Example 34

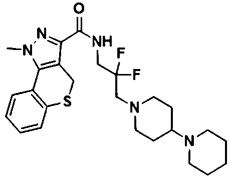

Referring to the synthesis method of Example 23, **compound 34** is synthesized by replacing 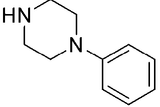 with

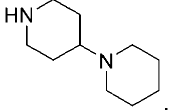

¹H NMR (400 MHz, DMSO-*d*₆) δ 8.36 (t, *J* = 6.3 Hz, 1H), 8.24 (s, 1H), 7.86 (dd, *J* = 7.5, 1.7 Hz, 1H), 7.51 (dd, *J = 7.4,* 1.7 Hz, 1H), 7.39 - 7.27 (m, 2H), 4.17 (s, 3H), 3.80 (d, *J =* 6.3 Hz, 2H), 2.99 (d, *J =* 11.2 Hz, 2H), 2.81 (t, *J =* 14.0 Hz, 2H), 2.56 (t, *J =* 5.2 Hz, 4H), 2.37 - 2.26 (m, 1H), 2.22 - 2.11 (m, 2H), 1.74 (d, *J* = 12.4 Hz, 2H), 1.53 (tt, *J =* 11.2, 4.6 Hz, 6H), 1.39 (t, *J =* 5.9 Hz, 2H).

### Example 35

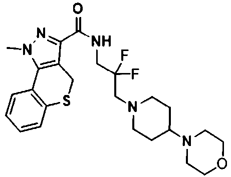

Referring to the synthesis method of Example 23, **compound 35** is synthesized by replacing 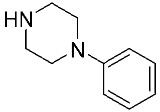 with

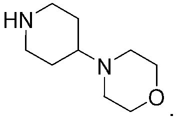

¹H NMR (400 MHz, DMSO-d₆) δ 8.46 (t, *J* = 6.1 Hz, 1H), 8.18 (s, 0H), 7.86 (dd, *J* = 7.5, 1.6 Hz, 1H), 7.51 (dd, *J = 7.4,* 1.7 Hz, 1H), 7.38 - 7.27 (m, 2H), 4.19 (s, 5H), 3.81 (td, *J =* 14.1, 6.0 Hz, 2H), 3.55 (t, *J =* 4.6 Hz, 4H), 2.99 (d, *J =* 11.4 Hz, 2H), 2.83 (t, *J =* 13.9 Hz, 2H), 2.43 (t, *J* = 4.6 Hz, 4H), 2.23 - 2.12 (m, 2H), 2.04 (tt, *J =* 11.1, 3.8 Hz, 1H), 1.77 (d, *J =* 11.8 Hz, 2H), 1.50 (qd, *J =* 12.1, 3.7 Hz, 2H).

### Example 36

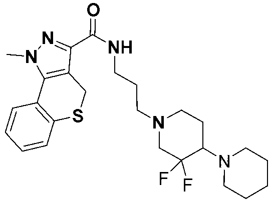

Referring to the synthesis method of Example 13, **compound 36** is synthesized by replacing **compound 11-4** with

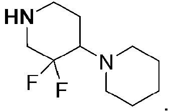

¹H NMR (400 MHz, DMSO-d6) δ 8.27 (s, 1H), 7.84 (dd, J = 7.7, 1.5 Hz, 1H), 7.51 (dd, J = 7.5, 1.6 Hz, 1H), 7.33 (dtd, J = 16.6, 7.4, 1.5 Hz, 2H), 4.18 (s, 2H), 4.14 (s, 3H), 3.33 - 3.10 (m, 10H), 2.66 (s, 1H), 2.37 (s, 2H), 2.13 (s, 1H), 1.86 (s, 1H), 1.75 (s, 6H), 1.47 (s, 2H).

### Example 37

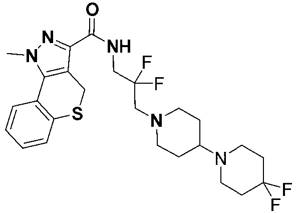

Referring to the synthesis method of Example 23, compound 37 is synthesized by replacing 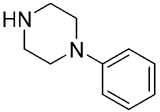 with

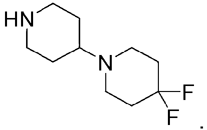

¹H NMR (400 MHz, ) δ 8.36 (t, *J* = 6.2 Hz, 1H), 7.86 (dd, *J* = 7.5, 1.7 Hz, 1H), 7.51 (dd, J = 7.4, 1.7 Hz, 1H), 7.39 - 7.27 (m, 2H), 4.19 (s, 2H), 4.16 (s, 3H), 3.81 (td, *J* = 14.3, 6.2 Hz, 2H), 2.98 (d, *J* = 10.8 Hz, 2H), 2.81 (t, *J* = 14.0 Hz, 2H), 2.57 (t, *J =* 5.6 Hz, 4H), 2.35 - 2.09 (m, 3H), 1.90 (ddt, J = 20.0, 13.7, 5.6 Hz, 4H), 1.69 (d, *J =* 12.1 Hz, 2H), 1.52 (qd, *J* = 11.9, 3.7 Hz, 2H).

### Example 38

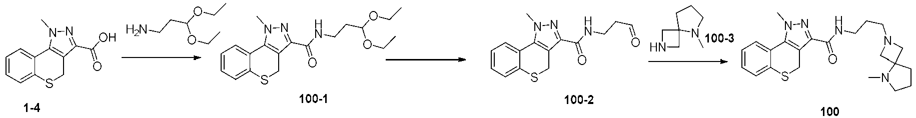

In step 1: **Compound 1-4** (150 mg, 0.61 mmol, 1.0 eq), HATU (325 mg, 0.85 mmol, 1.4 eq), and DIPEA (315 mg, 2.44 mmol, 4.0 eq) are dissolved in DMF (3 mL). 1-Amino-3,3-diethoxypropane (90 mg, 0.61 mmol, 1.0 eq) is added to the reaction mixture. The reaction mixture is stirred at room temperature overnight. After reaction, the reaction mixture is diluted with water (40 mL) and extracted with ethyl acetate (3 × 20 mL). The combined organic phases are washed with saturated brine (40 mL), dried over anhydrous sodium sulfate, filtered, and the filtrate is concentrated under reduced pressure to obtain a crude product **compound 100-1** (300 mg, crude).

In step 2: **Compound 100-1** (100 mg, crude, 0.27 mmol, 1.0 eq) is dissolved in acetone/water (v/v = 10/1, 2 mL /0.2 mL). p-Toluenesulfonic acid (TsOH, 56 mg, 0.29 mmol, 1.1 eq) is added to the reaction mixture. The reaction mixture is stirred at room temperature for 1 h. After reaction, the reaction mixture is diluted with water (30 mL) and extracted with ethyl acetate (3 × 10 mL). The combined organic phases are washed with saturated brine (20 mL), dried over anhydrous sodium sulfate, filtered, and the filtrate is concentrated under reduced pressure to obtain a crude product **compound 100-2** (57 mg, crude). LCMS: *m*/*z* = 302.1 [M+H]⁺

In step 3: **Compound 100-2** (53 mg, crude, 0.18 mmol, 1.5 eq) and **compound 100-3** (40 mg, 0.12 mmol, 1.0 eq) are dissolved in MeOH (1 mL). NaBH₃CN (14 mg, 0.22 mmol, 2.0 eq) is added to the reaction mixture. The reaction mixture is stirred at room temperature overnight. After reaction, the reaction mixture is quenched with water (20 mL) and extracted with ethyl acetate (3 × 10 mL). The combined organic phases are washed with saturated brine (20 mL), dried over anhydrous sodium sulfate, filtered, and the filtrate is concentrated under reduced pressure. The resulting crude product is purified by preparative TLC to obtain a free base product. The free base product is dissolved in methanol, and formic acid is added to form a salt. The resulting salt solution is concentrated under reduced pressure to obtain a colorless oily **compound 100** (17 mg, 37%). LCMS: *m*/*z* = 412.2 [M+H]⁺; ¹H NMR (400 MHz, DMSO-d₆) δ 8.27 (t, *J* = 5.8 Hz, 1H), 8.19 (s, 1H), 7.83 (dd, *J* = 7.6, 1.7 Hz, 1H), 7.50 (dd, *J = 7.4,* 1.7 Hz, 1H), 7.38 - 7.27 (m, 2H), 4.18 (s, 2H), 4.13 (s, 3H), 3.32 - 3.24 (m, 4H), 3.18 - 3.12 (m, 2H), 2.62 - 2.53 (m, 4H), 2.36 (s, 3H), 2.09 - 2.01 (m, 2H), 1.69 - 1.60 (m, 2H), 1.60 - 1.51 (m, 2H).

The following compounds are synthesized by referring to the synthetic method of **compound 100,** using the corresponding amines instead of **compound 100-3.**

| Compound | Structure | Amine intermediate | Spectrum information |
|---|---|---|---|
| **38** | 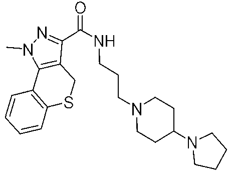 | | ¹H NMR (400 MHz, DMSO-*d*₆) δ 8.44 (t, J = 5.7 Hz, 1H), 8.26 (s, 1.98H), 7.84 (dd, J = 7.7, 1.6 Hz, 1H), 7.50 (dd, J = 7.4, 1.6 Hz, 1H), 7.32 (dtd, J = 16.8, 7.4, 1.5 Hz, 2H), 4.18 (s, 2H), 4.15 (s, 3H), 3.27 (q, J = 6.4 Hz, 2H), 3.03-2.91 (m, 2H), 2.84 (d, J = 6.2 Hz, 4H), 2.49-2.35 (m, 3H), 2.04-1.85 (m, 4H), 1.83-1.54 (m, 8H). LCMS m/z [M+H]⁺ = 440.4 |
| **42** | 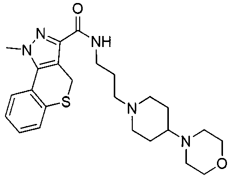 | | ¹H NMR (400 MHz, DMSO-d6) δ 8.61 (t, J = 5.5 Hz, 1H), 8.18 (s, 1.45H), 7.85 (dd, J = 7.6, 1.6 Hz, 1H), 7.50 (dd, J = 7.4, 1.7 Hz, 1H), 7.38 - 7.26 (m, 2H), 4.18 (s, 2H), 4.17 (s, 3H), 3.56 (s, 4H), 3.29 (q, J = 6.2 Hz, 3H), 3.02 (dd, J = 9.5, 5.9 Hz, 2H), 2.48 - 2.41 (m, 5H), 2.15 - 1.96 (m, 3H), 1.81 (d, J = 11.7 Hz, 2H), 1.69 (p, J = 6.6 Hz, 2H), 1.52 (qd, J = 12.1, 3.6 Hz, 2H). LCMS m/z [M+H]⁺ = 456.4 |
| **43** | 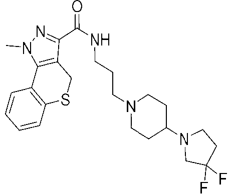 | | LCMS m/z [M+H]⁺ = 476.3. ¹H NMR(400 MHz, DMSO-d6) δ 8.52 (s, 1H), 7.83 (dd,J = 7.6, 1.6 Hz, 1H), 7.50 (dd,J = 7.4, 1.6 Hz, 1H), 7.32 (dtd,J = 18.0, 7.4, 1.5 Hz, 2H), 4.18 (s, 2H), 4.14 (s, 3H), 3.29 (q,J = 6.4 Hz, 7H), 2.92 (t,J = 13.8 Hz, 3H), 2.71 (t,J = 7.0 Hz, 2H), 2.23 (dq,J = 15.3, 7.9, 7.3 Hz, 3H), 1.90 (s, 2H), 1.81 (s, 2H), 1.57 (s, 2H). |
| **44** | 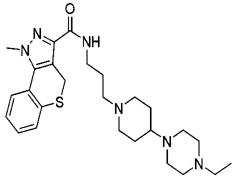 | | ¹H NMR(400 MHz, DMSO-d6) δ 8.48 (t,J = 5.7 Hz, 1H), 7.83 (dd,J = 7.6, 1.6 Hz, 1H), 7.49 (dd,J = 7.5, 1.6 Hz, 1H), 7.36 - 7.27 (m, 2H), 4.18 (s, 2H), 4.14 (s, 3H), 3.28 (q,J = 6.3 Hz, 2H), 3.10 (d,J = 11.2 Hz, 2H), 2.60 (d,J = 20.2 Hz, 12H), 2.25 (d,J = 34.7 Hz, 3H), 1.83 (d,J = 12.6 Hz, 2H), 1.73 (t,J = 6.9 Hz, 2H), 1.58 (q,J = 11.9 Hz, 2H), 1.05 (t,J = 7.2 Hz, 3H). LCMS m/z [M+H]⁺ = 483.3 |
| **45** | 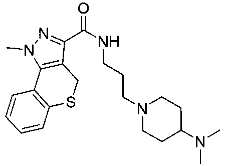 | | ¹H NMR(400 MHz, DMSO-d6) δ 8.38 (t,J = 5.7 Hz, 1H), 7.83 (dd,J = 7.6, 1.6 Hz, 1H), 7.49 (dd,J = 7.4, 1.7 Hz, 1H), 7.36 - 7.26 (m, 2H), 4.17 (s, 2H), 4.13 (s, 3H), 3.26 (q,J = 6.5 Hz, 2H), 2.97 (d,J = 11.2 Hz, 2H), 2.37 (q,J = 6.1, 5.3 Hz, 3H), 2.33 (s, 6H), 1.98 - 1.87 (m, 2H), 1.81 (d,J = 12.3 Hz, 2H), 1.66 (p,J = 6.8 Hz, 2H), 1.51 (qd,J = 11.9, 3.7 Hz, 2H).LCMS m/z [M+H]⁺ = 414.2 |
| **46** | 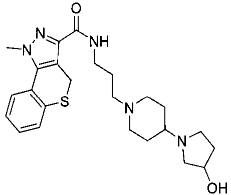 | | ¹H NMR(400 MHz, DMSO-d6) δ 8.56 (t,J = 5.6 Hz, 1H), 7.73 (dd,J = 7.8, 1.5 Hz, 1H), 7.28 (td,J = 7.8, 1.6 Hz, 1H), 7.08 (td,J = 7.6, 1.2 Hz, 1H), 7.01 (dd,J = 8.2, 1.2 Hz, 1H), 5.39 (s, 2H), 4.17 (s, 3H), 3.28 (q, J = 6.3 Hz, 4H), 3.01 (d,J = 11.2 Hz, 2H), 2.58 (s, 2H), 2.46 (s, 6H), 2.22 (s, 3H), 2.18 (s, 1H), 1.96 (s, 2H), 1.79 (d,J = 11.3 Hz, 2H), 1.68 (p,J = 6.6 Hz, 2H), 1.54 (tt,J = 12.1, 6.1 Hz, 2H). LCMS m/z [M+H]⁺ = 456.3 |
| **47** | 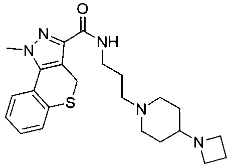 | | ¹H NMR(400 MHz, DMSO-d6) δ 8.45 (t,J = 5.7 Hz, 1H), 7.84 (dd,J = 7.7, 1.6 Hz, 1H), 7.50 (dd,J = 7.4, 1.6 Hz, 1H), 7.38 - 7.26 (m, 2H), 4.18 (s, 2H), 4.16 (s, 3H), 3.39 (t,J = 7.3 Hz, 4H), 3.27 (q,J = 6.4 Hz, 2H), 2.93 - 2.83 (m, 2H), 2.42 (t,J = 6.9 Hz, 3H), 2.02 (q,J = 9.4, 8.2 Hz, 4H), 1.74 - 1.63 (m, 4H), 1.40 - 1.26 (m, 2H). LCMS m/z [M+H]⁺ = 426.2 |
| **48** | 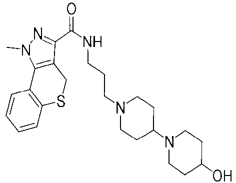 | | ¹H NMR (400 MHz, DMSO-d6) δ 8.41 (t, J = 5.7 Hz, 1H), 8.24 (s, 1.8H), 7.84 (dd, J = 7.6, 1.6 Hz, 1H), 7.50 (dd, J = 7.4, 1.6 Hz, 1H), 7.32 (dtd, J = 16.7, 7.4, 1.5 Hz, 2H), 4.18 (s, 2H), 4.14 (s, 3H), 3.52 (dd, J = 8.7, 4.6 Hz, 1H), 3.27 (q, J = 6.4 Hz, 2H), 3.01 (d, J = 11.4 Hz, 2H), 2.91 (dt, J = 10.6, 4.5 Hz, 2H), 2.50 - 2.37 (m, 6H), 2.04 - 1.91 (m, 2H), 1.79 (dd, J = 20.1, 12.3 Hz, 4H), 1.62 (dtd, J = 36.4, 12.8, 12.3, 7.7 Hz, 4H), 1.45 (qd, J = 9.0, 4.4 Hz, 2H). LCMS m/z [M+H]⁺ = 470.4 |
| **52** | 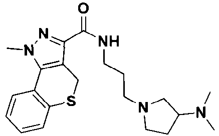 | | ¹H NMR (400 MHz, DMSO-d6) δ 8.33 (t, J = 5.9 Hz, 1H), 7.84 (dd, J = 7.6, 1.6 Hz, 1H), 7.50 (dd, J = 7.5, 1.6 Hz, 1H), 7.32 (dtd, J = 16.9, 7.5, 1.6 Hz, 2H), 4.19 (s, 2H), 4.14 (s, 3H), 3.26 (d, J = 6.5 Hz, 2H), 3.06 (s, 1H), 2.81 (s, 1H), 2.63 (d, J = 33.5 Hz, 4H), 2.29 (s, 6H), 1.97 (dq, J = 14.5, 6.5, 5.9 Hz, 1H), 1.74 (dp, J = 20.9, 7.0 Hz, 3H), 1.24 (d, J = 5.8 Hz, 1H). LCMS m/z [M+H]⁺ = 400.3 |
| **53** | 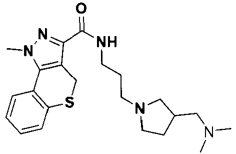 | | ¹H NMR (400 MHz, DMSO-d6) δ 8.38 (t, J = 5.8 Hz, 1H), 7.84 (dd, J = 7.6, 1.7 Hz, 1H), 7.50 (dd, J = 7.4, 1.6 Hz, 1H), 7.32 (dtd, J = 16.8, 7.4, 1.6 Hz, 2H), 4.19 (s, 2H), 4.13 (s, 3H), 3.29 (d, J = 6.5 Hz, 2H), 2.69 (d, J = 26.5 Hz, 4H), 2.47 - 2.21 (m, 4H), 2.18 (s, 6H), 2.04 - 1.88 (m, 1H), 1.74 (p, J = 7.0, 6.5 Hz, 2H), 1.47 (dq, J = 13.2, 7.0 Hz, 1H), 1.24 (d, J = 6.1 Hz, 1H). LCMS m/z [M+H]⁺ = 414.4 |
| **54** | 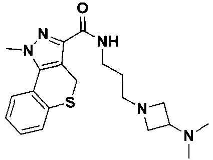 | | ¹H NMR(400 MHz, DMSO-d6) δ 8.33 (t,J = 5.8 Hz, 1H), 7.83 (dd,J = 7.7, 1.6 Hz, 1H), 7.50 (dd,J = 7.4, 1.6 Hz, 1H), 7.37 - 7.27 (m, 2H), 4.18 (s, 2H), 4.14 (s, 3H), 3.50 (dt,J = 7.0, 3.2 Hz, 2H), 3.24 (q,J = 6.6 Hz, 2H), 2.86 (dq,J = 12.7, 6.4 Hz, 3H), 2.54 (d,J = 6.9 Hz, 2H), 2.02 (s, 6H), 1.54 (p,J = 6.9 Hz, 2H).LCMS m/z [M+H]⁺ = 466.3 |
| **56** | 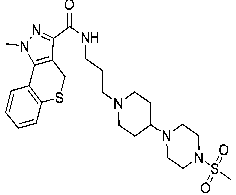 | | ¹H NMR (400 MHz, DMSO-d6) δ 8.47 (t, J = 5.6 Hz, 1H), 7.84 (dd, J = 7.6, 1.6 Hz, 1H), 7.50 (dd, J = 7.5, 1.6 Hz, 1H), 7.32 (dtd, J = 16.9, 7.5, 1.6 Hz, 2H), 4.18 (s, 2H), 4.15 (s, 3H), 3.29 (q, J = 6.3 Hz, 2H), 3.06 (dt, J = 15.9, 7.5 Hz, 6H), 2.86 (s, 3H), 2.57 (t, J = 4.9 Hz, 4H), 2.48 (d, J = 5.8 Hz, 2H), 2.31 - 2.19 (m, 1H), 2.08 (t, J = 11.5 Hz, 2H), 1.85 -1.67 (m, 4H), 1.66 - 1.50 (m, 2H). LCMS m/z [M+H]⁺ = 533.3 |
| **58** | 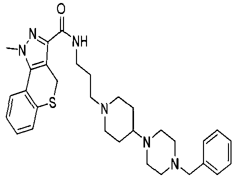 | | ¹H NMR (400 MHz, DMSO-d6) δ 8.56 (t, J = 5.5 Hz, 1H), 8.21 (s, 1.4H), 7.84 (d, J = 7.8 Hz, 1H), 7.50 (d, J = 7.5 Hz, 1H), 7.28 (tt, J = 13.0, 6.4 Hz, 7H), 4.18 (s, 2H), 4.15 (s, 3H), 3.44 (s, 2H), 3.29 (q, J = 6.3 Hz, 2H), 2.97 (d, J = 11.1 Hz, 2H), 2.67 (s, 3H), 2.39 (dd, J = 16.0, 9.5 Hz, 7H), 2.10 (d, J = 12.3 Hz, 1H), 1.91 (t, J = 11.4 Hz, 2H), 1.84 - 1.73 (m, 2H), 1.73 - 1.62 (m, 2H), 1.51 (q, J = 12.7, 12.2 Hz, 2H). LCMS m/z [M+H]⁺ = 545.4 |
| **59** | 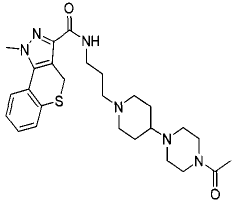 | | ¹H NMR (400 MHz, DMSO-d6) δ 8.55 (t, J = 5.6 Hz, 1H), 8.21 (s, 1.68H), 7.89 - 7.80 (m, 1H), 7.50 (dd, J = 7.3, 1.7 Hz, 1H), 7.32 (tt, J = 8.1, 6.4 Hz, 2H), 4.19 (s, 2H), 4.16 (s, 3H), 3.40 (d, J = 9.2 Hz, 4H), 3.29 (q, J = 6.2 Hz, 2H), 3.00 (d, J = 11.3 Hz, 2H), 2.43 (dq, J = 16.4, 4.9 Hz, 6H), 2.16 (dd, J = 12.8, 8.6 Hz, 1H), 1.97 (s, 5H), 1.83 - 1.74 (m, 2H), 1.68 (p, J = 6.5 Hz, 2H), 1.61 - 1.47 (m, 2H). LCMS m/z [M+H]⁺ = 497.4 |
| **60** | 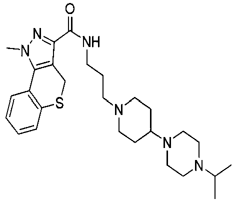 | | ¹H NMR (400 MHz, DMSO-d6) δ 8.51 (t, J = 5.6 Hz, 1H), 8.25 (s, 1.86H), 7.84 (dd, J = 7.6, 1.6 Hz, 1H), 7.50 (dd, J = 7.5, 1.6 Hz, 1H), 7.38 - 7.27 (m, 2H), 4.18 (s, 2H), 4.15 (s, 3H), 3.29 (q, J = 6.3 Hz, 2H), 2.98 (dd, J = 10.3, 4.4 Hz, 3H), 2.66 (dd, J = 13.5, 7.0 Hz, 2H), 2.58 - 2.50 (m, 6H), 2.49 (d, J = 1.8 Hz, 2H), 2.42 (t, J = 6.6 Hz, 2H), 2.22 - 2.09 (m, 1H), 2.01 - 1.87 (m, 2H), 1.78 (d, J = 11.6 Hz, 2H), 1.67 (p, J = 6.6 Hz, 2H), 1.58 - 1.44 (m, 2H), 0.99 (d, J = 6.5 Hz, 4H). LCMS m/z [M+H]⁺ = 497.4 |
| **62** | 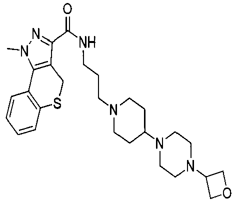 | | ¹H NMR (400 MHz, CD₃OD) δ 7.79 (d, J = 7.3 Hz, 1H), 7.51 - 7.47 (m, 1H), 7.36 - 7.25 (m, 2H), 4.74 - 4.69 (m, 2H), 4.62 (t, J = 6.2 Hz, 2H), 4.17 (s, 5H), 3.72 - 3.59 (m, 3H), 3.52 - 3.44 (m, 3H), 3.18 - 2.93 (m, 10H), 2.32 - 2.16 (m, 3H), 2.14 - 2.00 (m, 5H). LCMS m/z [M+H]⁺ = 511.4 |
| **63** | 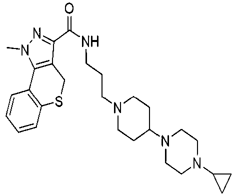 | | ¹H NMR (400 MHz, DMSO-d6) δ 8.51 (dt, J = 18.5, 5.5 Hz, 1H), 8.25 (s, 1.89H), 7.84 (dt, J = 7.7, 1.9 Hz, 1H), 7.50 (dd, J = 7.5, 1.6 Hz, 1H), 7.38 - 7.26 (m, 2H), 4.19 (s, 2H), 4.16 (d, J = 2.6 Hz, 3H), 3.29 (q, J = 6.2 Hz, 2H), 3.03 - 2.91 (m, 3H), 2.73 - 2.53 (m, 2H), 2.40 (dd, J = 14.6, 8.1 Hz, 5H), 2.25 - 2.03 (m, 1H), 1.89 (t, J = 11.7 Hz, 2H), 1.76 (d, J = 12.3 Hz, 2H), 1.66 (p, J = 6.5 Hz, 2H), 1.61 - 1.42 (m, 3H), 0.37 (dt, J = 6.1, 3.0 Hz, 1H), 0.26 (q, J = 3.3, 2.9 Hz, 1H). LCMS m/z [M+H]⁺ = 495.4 |
| **64** | 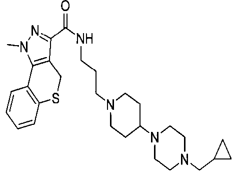 | | ¹H NMR (400 MHz, DMSO-d6) δ 8.38 (s, 1H), 7.78 (d, J = 7.7 Hz, 1H), 7.46 (d, J = 7.5 Hz, 1H), 7.28 (dt, J = 19.7, 7.5 Hz, 2H), 4.19 (s, 2H), 4.16 (s, 3H), 3.35 (q, J = 6.3 Hz, 2H), 2.96 (d, J = 11.2 Hz, 2H), 2.44 (d, J = 18.2 Hz, 8H), 2.24 - 2.03 (m, 3H), 1.91 (d, J = 11.3 Hz, 2H), 1.85 - 1.64 (m, 4H), 1.56 (q, J = 13.5, 12.1 Hz, 2H), 1.26 (d, J = 6.0 Hz, 2H), 0.84 (dt, J = 19.2, 6.8 Hz, 1H), 0.45 (d, J = 7.8 Hz, 2H), 0.07 (d, J = 5.0 Hz, 2H). LCMS m/z [M+H]⁺ = 509.4 |
| **65** | 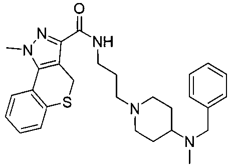 | | LCMS *m*/*z* = 490.2 [M+H]⁺. ¹H NMR (400 MHz, DMSO-d₆) δ 8.45 - 8.32 (m, 1H), 7.85 (dd, J = 7.5, 1.7 Hz, 1H), 7.51 (dd, J = 7.6, 1.6 Hz, 1H), 7.41 - 7.19 (m, 7H), 4.19 (s, 2H), 4.14 (s, 3H), 3.69 - 3.60 (m, 1H), 3.55 - 3.39 (m, 2H), 3.08 - 2.88 (m, 2H), 2.85 - 2.65 (m, 2H), 2.24 - 2.13 (m, 2H), 2.07 (s, 3H), 2.04 - 1.93 (m, 2H), 1.92 - 1.80 (m, 2H), 1.79 - 1.65 (m, 2H). |
| **66** | 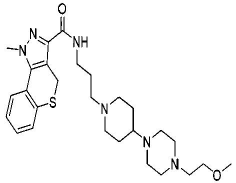 | | ¹H NMR (400 MHz, DMSO-d6) δ 8.48 (s, 1H), 7.84 (dd, J = 7.6, 1.6 Hz, 1H), 7.50 (dd, J = 7.5, 1.7 Hz, 1H), 7.39 - 7.27 (m, 2H), 4.19 (s, 2H), 4.15 (s, 3H), 3.43 (s, 4H), 3.30 - 3.24 (m, 4H), 3.23 (s, 3H), 3.15-2.70 (m, 4H) 2.67 (s, 4H), 2.33 (s, 4H), 1.82 (s, 5H), 1.57 (s, 2H). LCMS m/z [M+H]⁺ = 513.4 |
| **67** | 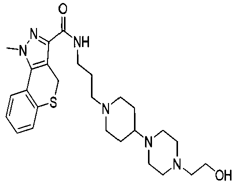 | | ¹H NMR (400 MHz, DMSO-*d*₆) δ 8.47 (s, 1H), 7.84 (dd, *J* = 7.6, 1.6 Hz, 1H), 7.51 (dd, *J* = 7.4, 1.6 Hz, 1H), 7.33 (dtd, *J* = 16.9, 7.4, 1.5 Hz, 2H), 4.19 (s, 2H), 4.15 (s, 3H), 3.54 (s, 2H), 3.28 (t, *J* = 6.2 Hz, 6H), 3.12 (s, 4H), 2.59 (s, 6H), 2.33 (s, 2H), 1.85 (s, 5H), 1.56 (s, 2H). LCMS m/z [M+H]⁺ = 499.4 |
| **70** | 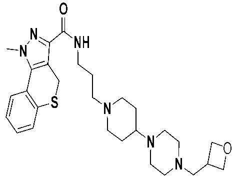 | | ¹H NMR(400 MHz, DMSO-d6) δ 8.52 (t,J = 5.6 Hz, 1H), 7.84 (dd,J = 7.6, 1.6 Hz, 1H), 7.50 (dd,J = 7.5, 1.6 Hz, 1H), 7.39 - 7.26 (m, 2H), 4.62 (dd,J = 7.8, 5.8 Hz, 2H), 4.23 (t,J = 6.1 Hz, 2H), 4.18 (s, 2H), 4.15 (s, 3H), 3.29 (q,J = 6.3 Hz, 2H), 3.20 - 3.08 (m, 1H), 3.01 (d,J = 11.2 Hz, 2H), 2.59 (d,J = 7.3 Hz, 2H), 2.46 (t,J = 6.6 Hz, 6H), 2.32 (s, 4H), 2.15 (t,J = 11.1 Hz, 1H), 2.01 (t,J = 11.5 Hz, 2H), 1.83 - 1.73 (m, 2H), 1.69 (p,J = 6.6 Hz, 2H), 1.57 - 1.45 (m, 2H).LCMS m/z [M+Hl⁺ = 525.3 |
| **74** | 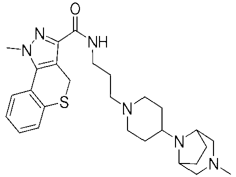 | | LCMS *m*/*z* = 495.2 [M+H]⁺. H NMR: ¹H NMR (400 MHz, DMSO-d₆) δ 8.40 (s, 1H), 7.85 (d, *J* = 9.2 Hz, 1H), 7.51 (d, *J* = 9.2 Hz, 1H), 7.41 - 7.26 (m, 2H), 4.19 (s, 2H), 4.15 (s, 3H), 3.57 - 3.31 (m, 5H), 3.28 - 2.55 (m, 11H), 2.29 - 1.56 (m, 12H). |
| **100** | 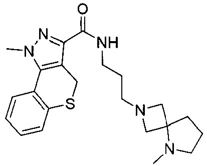 | | LCMS: *m*/*z* = 412.2 [M+H]⁺; ¹H NMR (400 MHz, DMSO-*d*₆) δ 8.27 (t, *J* = 5.8 Hz, 1H), 8.19 (s, 1H), 7.83 (dd, *J* = 7.6, 1.7 Hz, 1H), 7.50 (dd, *J* = 7.4, 1.7 Hz, 1H), 7.38 - 7.27 (m, 2H), 4.18 (s, 2H), 4.13 (s, 3H), 3.32 - 3.24 (m, 4H), 3.18 - 3.12 (m, 2H), 2.62 - 2.53 (m, 4H), 2.36 (s, 3H), 2.09 - 2.01 (m, 2H), 1.69 - 1.60 (m, 2H), 1.60 - 1.51 (m, 2H). |

### Example 39

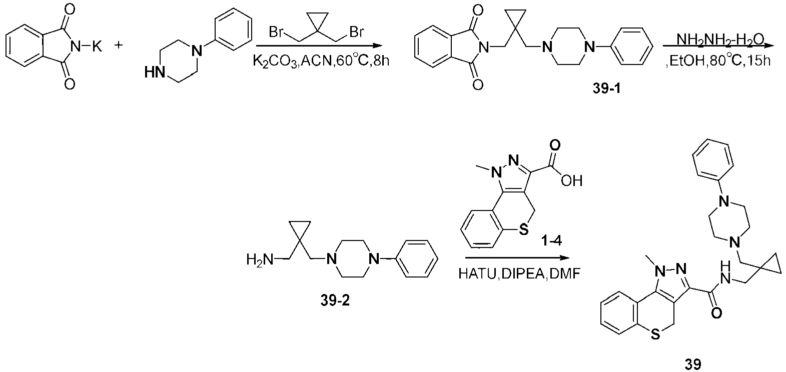

In step 1: Potassium phthalimide (0.6 g, 3.24 mmol), 1,1-bis(bromomethyl)cyclopropane (0.96 g, 4.21 mmol), and potassium carbonate (1.57 g, 11.34 mmol) are dissolved in acetonitrile (10 mL). Then N-phenylpiperazine (0.53 g, 3.24 mmol) is added. The reaction mixture is heated at 60°C for 8 h. LCMS shows that the reaction is complete. The mixture is filtered, and the filtrate is concentrated and purified by dry-column chromatography (petroleum ether/ethyl acetate (PE/EA) = 50%) to obtain **compound 39-1** (0.61 g, yield: 50.15%). m/z [M+H]⁺= 376.4

In step 2: **Compound 39-1** (0.61 g, 1.62 mmol) and hydrazine hydrate (wt 80%, 0.2 g, 3.24 mmol) are dissolved in ethanol (10 mL). The mixture is heated at 80°C overnight. LCMS shows that the reaction is complete. The mixture is filtered, and the filtrate is concentrated. The resulting solid is triturated with petroleum ether, filtered again, and the filtrate is concentrated to obtain **compound 39-2** (0.35 g, yield: 87.8%).

In step 3: **Compound 1-4** (0.106 g, 0.43 mmol), **compound 39-2** (0.21 g, 0.52 mmol), and HATU (0.25 g, 0.62 mmol) are added to DMF (2 mL). Then DIPEA (0.17 g, 1.29 mmol) is added, and the reaction mixture is stirred at room temperature for 3 h. LCMS shows that the reaction is complete. The mixture is purified directly by reverse-phase column chromatography to obtain a product **compound 39** (0.065 g, yield: 31.89%). m/z [M+H]⁺ = 474.4. ¹H NMR(400 MHz, DMSO-d6) δ 8.74 (t,J = 5.5 Hz, 1H), 7.75 (dd,J = 6.7, 2.4 Hz, 1H), 7.49 (dd,J = 6.5, 2.4 Hz, 1H), 7.29 (dt,J = 6.3, 2.6 Hz, 2H), 7.21 (t,J = 7.8 Hz, 2H), 6.96 (d,J = 8.2 Hz, 2H), 6.78 (t,J = 7.2 Hz, 1H), 4.18 (s, 2H), 3.88 (s, 3H), 3.33 (d,J = 5.4 Hz, 3H), 3.31 - 3.27 (m, 4H), 2.64 (t,J = 4.8 Hz, 3H), 2.43 (s, 2H), 0.52 (d,J = 4.7 Hz, 2H), 0.38 (d,J = 4.8 Hz, 2H).

### Example 40

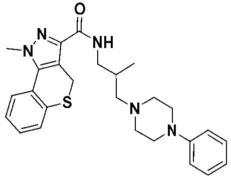

Referring to the synthesis method of Example 39, **compound 40** is synthesized by replacing 1,1-bis(bromomethyl)cyclopropane with 1,1-dibromo-2-methylpropane. ¹H NMR(400 MHz, DMSO-d6) δ 8.86 (d, J = 4.1 Hz, 1H), 7.78 - 7.72 (m, 1H), 7.48 (dd, J = 7.2, 1.9 Hz, 1H), 7.28 (tt, J = 7.4, 5.6 Hz, 2H), 7.23 - 7.16 (m, 2H), 6.94 (d, J = 8.1 Hz, 2H), 6.76 (t, J = 7.2 Hz, 1H), 4.17(s, 2H), 3.91 (s, 3H), 3.41 (dd, J = 13.5, 7.3 Hz, 2H), 3.22 (s, 4H), 3.02 (dd, J = 12.7, 8.6 Hz, 1H), 2.64 (s, 2H), 2.39 (t, J = 10.9 Hz, 2H), 2.28 - 2.20 (m, 1H), 2.04 (s, 1H), 0.86 (d, J = 6.6 Hz, 3H). m/z [M+H]⁺ = 462.4

### Example 41

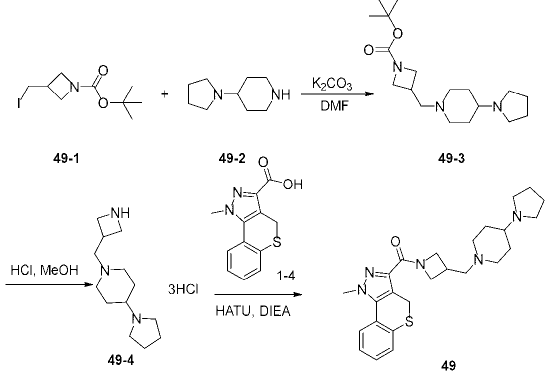

In step 1: To a 50 mL single-necked flask, **compound 49-1** (450 mg, 1.50 mmol), **compound 49-2** (280 mg, 1.80 mmol), and DMF (4 mL) are added sequentially. Potassium carbonate (510 mg, 3.75 mmol) is added. The mixture is stirred at 60°C for 16 h. Water and ethyl acetate are added to the reaction mixture, and the layers are separated. The aqueous phase is extracted with ethyl acetate twice. The combined organic phases are dried over anhydrous sodium sulfate, filtered, and the filtrate is concentrated under reduced pressure. The residue is purified by column chromatography to obtain **compound 49-3** (420 mg, yield: 85.7%). LCMS m/z [M+H]⁺ = 324.3

In step 2: To a 50 mL single-necked flask, **compound 49-3** (320 mg, 0.99 mmol), methanol (1 mL), and a solution of HCl in 1,4-dioxane (4 M, 4 mL) are added sequentially. The mixture is stirred at room temperature for 16 h. The reaction mixture is concentrated to dryness under reduced pressure to obtain **compound** 49-4 (200 mg, yield: 61%), which is used directly in the next step.

In step 3: To a 50 mL single-necked flask, **compound 1-4** (100 mg, 0.41 mmol), **compound 49-4** (160 mg, 0.49 mmol), and DMF (2 mL) are added sequentially. Then DIPEA (320 mg, 2.46 mmol) and HATU (180 mg, 0.47 mmol) are added. The mixture is stirred at room temperature for 4 h. The reaction mixture is filtered, and the filtrate is purified directly by reverse-phase column chromatography to obtain **compound 49** (50.4 mg, yield: 27.5%). ¹H NMR (400 MHz, DMSO-d6) δ 7.83 (dd, J = 7.6, 1.7 Hz, 1H), 7.50 (dd, J = 7.4, 1.7 Hz, 1H), 7.37 - 7.26 (m, 2H), 4.56 (t, J = 9.0 Hz, 1H), 4.16 (s, 2H), 4.15 - 4.04 (m, 5H), 3.63 (dd, J = 9.9, 5.5 Hz, 1H), 2.96 - 2.51 (m, 9H), 2.04 - 1.80 (m, 4H), 1.74 (s, 4H), 1.45 (s, 2H), 1.24 (d, J = 5.9 Hz, 1H). m/z [M+H]⁺ = 452.3

### Example 42

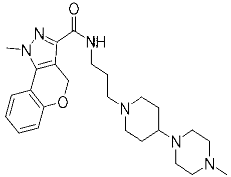

Referring to the synthesis method of Example 30, **compound 50** is synthesized by replacing **compound 1-4** with **compound 16-4.**

### Example 43

Referring to the synthesis method of Example 13, the following compounds are synthesized by replacing **compound 11-4** with amine fragments.

| Compound | Structure | Amine intermediate | Spectrum information |
|---|---|---|---|
| **81** | 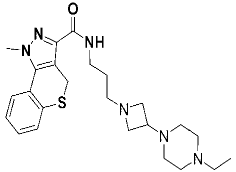 | | ¹H NMR(400 MHz, DMSO-d6) δ 8.33 (t,J = 5.8 Hz, 1H), 8.22 (HCOOH, 1.5H), 7.86 - 7.82 (m, 1H), 7.52 - 7.49 (m, 1H), 7.37 - 7.27 (m, 2H), 4.18 (s, 2H), 4.14 (s, 3H), 3.50 - 3.44 (m, 2H), 3.24 (q,J = 6.5 Hz, 3H), 2.87 (dq,J = 13.2, 6.5 Hz, 3H), 2.53-2.51 (m, 2H), 2.49-2.48 (m, 1H), 2.29 (dd,J = 18.9, 11.7 Hz, 8H), 1.53 (p,J = 6.9 Hz, 2H), 0.97 (t,J = 7.2 Hz, 3H).LCMS m/z [M+H]⁺ = 455.3 |
| **85** | 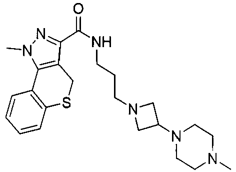 | | LCMS *m*/*z* = 441.2 [M+H]⁺; ¹H NMR (400 MHz, DMSO-*d*₆) δ 8.32 (t, *J =* 5.8 Hz, 1H), 7.84 (dd, *J* = 7.6, 1.6 Hz, 1H), 7.50 (dd, J= 7.5, 1.6 Hz, 1H), 7.38 - 7.26 (m, 2H), 4.18 (s, 2H), 4.14 (s, 3H), 3.56 - 3.53 (m, 2H), 3.24 (q, *J* = 6.5 Hz, 2H), 3.01 - 2.91 (m, 3H), 2.58 (t, *J =* 7.0 Hz, 2H), 2.45 - 2.19 (m, 8H), 2.18 (s, 3H), 1.55 (p, *J =* 6.9 Hz, 2H). |

### Example 44

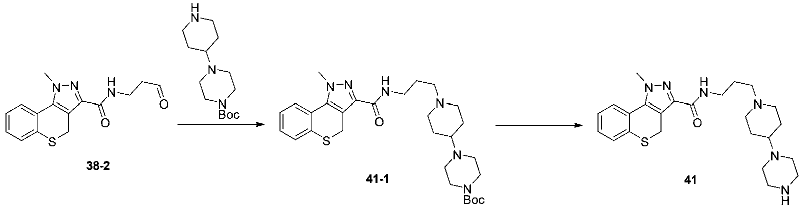

In step 1: To a 100 mL single-necked flask, **compound 38-2** (700 mg, 2.32 mmol), 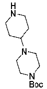 (720 mg, 2.67 mmol), and DCM (15 mL) are added sequentially. Then sodium triacetoxyborohydride (1.08 g, 5.10 mmol) and acetic acid (6 mg, 0.1 mmol) are added. The mixture is stirred at room temperature for 2 h. Water and DCM are added to the reaction mixture. The pH is adjusted to 8-9 with dilute aqueous sodium hydroxide solution, and the layers are separated. The aqueous phase is extracted with DCM twice. The combined organic phases are dried over anhydrous sodium sulfate, filtered, and the filtrate is concentrated to dryness under reduced pressure. The residue is purified by column chromatography to obtain **compound 41-1** (700 mg, yield: 54.3%). LCMS m/z [M+H]⁺ = 555.4

In step 2: To a 25 mL single-necked flask, **compound 41-1** (200 mg, 0.36 mmol), methanol (1 mL), and a solution of HCl in 1,4-dioxane (4 M, 4 mL) are added sequentially. The mixture is stirred at room temperature for 4 h. The reaction mixture is concentrated to dryness under reduced pressure and purified by reverse-phase column chromatography to obtain **compound 41** (125.7 mg, yield: 76.6%) as a hydrochloride salt. ¹H NMR (400 MHz, DMSO-d6) δ 8.48 (t, J = 5.8 Hz, 1H), 8.18 (s, 1H), 7.84 (dd, J = 7.6, 1.6 Hz, 1H), 7.50 (dd, J = 7.5, 1.6 Hz, 1H), 7.32 (dtd, J = 17.6, 7.4, 1.6 Hz, 2H), 4.19 (s, 2H), 4.15 (s, 3H), 3.29 (q, J = 6.3 Hz, 2H), 3.16 (d, J = 11.7 Hz, 2H), 3.02 (t, J = 4.9 Hz, 4H), 2.73 - 2.59 (m, 6H), 2.41 - 2.26 (m, 3H), 1.79 (dt, J = 13.9, 8.9 Hz, 4H), 1.65 (q, J = 11.2 Hz, 2H). LCMS m/z [M+H]⁺ = 455.4

Referring to the synthesis method of Example 41, **compound 55** is synthesized by replacing 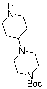 with 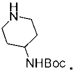 ¹H NMR(400 MHz, DMSO-d6) δ 8.47 (s, 2H), 8.39 (t,J = 6.0 Hz, 1H), 7.84 (dd,J = 7.6, 1.6 Hz, 1H), 7.50 (dd,J = 7.5, 1.6 Hz, 1H), 7.32 (dtd,J = 16.9, 7.4, 1.6 Hz, 2H), 4.19 (d,J = 2.1 Hz, 2H), 4.14 (s, 3H), 3.51 (d,J = 12.3 Hz, 2H), 3.30 (q,J = 6.5 Hz, 3H), 3.00 (q,J = 11.6, 11.1 Hz, 4H), 2.12 (d,J = 13.1 Hz, 2H), 1.97 (dd,J = 14.8, 9.0 Hz, 4H).LCMS m/z [M+H]⁺ = 386.2.

### Example 45

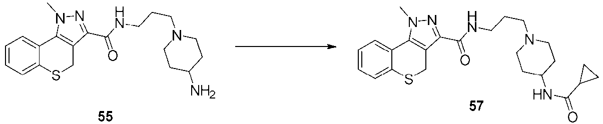

¹H NMR(400 MHz, DMSO-d6) δ 8.38 (t,J = 5.7 Hz, 1H), 8.20 (HCOOH, 0.53H), 7.95 (d,J = 7.4 Hz, 1H), 7.83 (dd,J = 7.7, 1.6 Hz, 1H), 7.50 (dd,J = 7.4, 1.7 Hz, 1H), 7.37 - 7.26 (m, 2H), 4.19 (s, 2H), 4.14 (s, 3H), 3.28 (q,J = 6.5 Hz, 3H), 2.85 (d,J = 11.5 Hz, 2H), 2.38 (t,J = 6.8 Hz, 2H), 2.06 - 1.96 (m, 2H), 1.80 - 1.71 (m, 2H), 1.66 (q,J = 6.8 Hz, 2H), 1.56 - 1.43 (m, 3H), 0.66 - 0.58 (m, 4H).LCMS m/z [M+H]⁺ = 454.2.

### Example 46

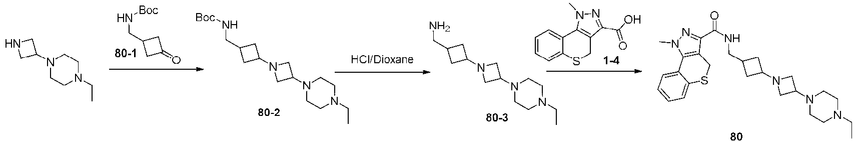

In step 1: 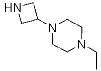 (679.6 mg, 4.0 mmol, 1.0 eq) and **compound 80-1** (800 mg, 4.0 mmol, 1.0 eq) are dissolved in DCE (10 mL). 2 drops of acetic acid are added. The reaction mixture is stirred at room temperature for 0.5 h and then cooled to 0°C. NaBH(OAc)₃ (1.69 g, 8.0 mmol, 2.0 eq) is added to the reaction mixture in portions. The reaction mixture is stirred at 0°C for 5 min, then warmed to room temperature and stirred overnight. After reaction, the reaction mixture is diluted with water (50 mL), and the pH is adjusted to basic with saturated NaHCO₃ solution. The mixture is extracted with ethyl acetate (3 × 40 mL). The combined organic phases are washed with saturated brine, dried over anhydrous sodium sulfate, filtered, and the filtrate is concentrated under reduced pressure. The crude product is purified by silica gel column chromatography (eluent: DCM: methanol = 10:1) to obtain a yellow oily **compound 80-2** (640 mg, 45%). LCMS *m*/*z* = 353.3 [M+H]⁺

In step 2: **Compound 80-2** (640 mg, 1.81 mmol, 1.0 eq) is dissolved in DCM (7 mL). HCl solution (4M in dioxane, 7 mL) is added dropwise to the reaction mixture. The reaction mixture is stirred at room temperature for 3 h. After reaction, the reaction mixture is concentrated under reduced pressure to obtain a white solid **compound 80-3** (630 mg, 100%). LCMS *m*/*z* = 253.3 [M+H]⁺

In step 3: Referring to the synthesis method of Example 13, **compound 80** is synthesized by replacing **compound 13-3** with **compound 61-3.** ¹H NMR(400 MHz, DMSO-d6) δ 8.32 (q,J = 5.6 Hz, 1H), 8.14 (HCOOH, H0.45), 7.87 - 7.81 (m, 1H), 7.51 (dd,J = 7.5, 1.7 Hz, 1H), 7.38 - 7.27 (m, 2H), 4.18 (d,J = 1.7 Hz, 2H), 4.14 (s, 3H), 3.91-3.85 (m, 2H), 3.78-3.72 (m, 1H), 3.65-3.61 (m, 3H), 3.34 - 3.25 (m, 4H), 3.17 (dd,J = 9.9, 5.9 Hz, 2H), 2.83-2.74 (m, 4H), 2.46 - 2.32 (m, 2H), 2.21 (dt,J = 28.0, 7.7 Hz, 2H), 2.02 (t,J = 7.2 Hz, 2H), 1.80 - 1.70 (m, 1H), 1.10 (q,J = 7.5 Hz, 3H).LCMS m/z [M+H]⁺ = 481.3.

### Example 47

Referring to the synthesis method of Example 80, the following compounds are synthesized by replacing 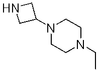 with amine fragments and replacing **compound 1-4** with acid fragments.

| Compound | Structure | Amine intermediate | Acid intermediate | Spectrum information |
|---|---|---|---|---|
| **51** | 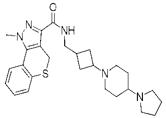 | 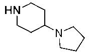 | **1-4** | ¹H NMR(400 MHz, DMSO-d6) δ 8.24 - 8.13 (m, 1H), 7.83 (dd,J = 7.6, 1.6 Hz, 1H), 7.50 (dd,J = 7.5, 1.6 Hz, 1H), 7.38 - 7.27 (m, 2H), 4.18 (s, 2H), 4.13 (d,J = 1.3 Hz, 3H), 3.28 (dt, J = 40.1, 6.7 Hz, 2H), 2.84 - 2.70 (m, 6H), 2.54 (d,J = 7.8 Hz, 1H), 2.36 - 2.28 (m, 1H), 2.20 - 2.03 (m, 2H), 1.92 - 1.81 (m, 3H), 1.78 - 1.66 (m, 6H), 1.60 - 1.35 (m, 4H).LCMS m/z [M+H]⁺ = 466.3 |
| **61** | 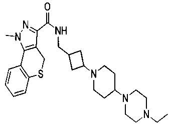 | | **1-4** | ¹H NMR(400 MHz, DMSO-d6) δ 8.22 (HCOOH, 1.45), 8.17 (t,J = 6.1 Hz, 1H), 7.83 (dd,J = 7.7, 1.6 Hz, 1H), 7.50 (dd,J = 7.4, 1.7 Hz, 1H), 7.37 - 7.27 (m, 2H), 4.18 (s, 2H), 4.13 (s, 3H), 3.23 (t,J = 6.2 Hz, 2H), 2.87 (d,J = 11.2 Hz, 2H), 2.61 (d,J = 7.6 Hz, 1H), 2.57 - 2.51 (m, 3H), 2.46 (s, 2H), 2.44 - 2.36 (m, 3H), 2.26 - 2.14 (m, 2H), 2.13 - 2.02 (m, 2H), 2.01 - 1.87 (m, 1H), 1.87 - 1.70 (m, 5H), 1.57 (dt,J = 10.9, 8.6 Hz, 2H), 1.41 (td,J = 12.7, 12.0, 9.2 Hz, 2H), 1.00 (t,J = 7.2 Hz, 3H).LCMS m/z [M+H]⁺ = 509.3 |
| **77** | 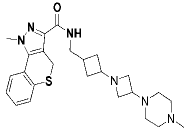 | | **1-4** | ¹H NMR(400 MHz, DMSO-d6) δ 8.33 (t,J = 6.0 Hz, 1H), 8.17 (HCOOH, 1.9H), 7.87 - 7.79 (m, 1H), 7.50 (dd,J = 7.5, 1.6 Hz, 1H), 7.32 (dtd,J = 16.8, 7.4, 1.6 Hz, 2H), 4.18 (d,J = 3.3 Hz, 2H), 4.13 (d,J = 2.3 Hz, 3H), 3.24 (dt,J = 12.4, 6.6 Hz, 6H), 2.90 - 2.77 (m, 3H), 2.22 (ddd,J = 40.8, 21.3, 11.8 Hz, 10H), 1.98 (dq,J = 12.3, 7.1, 5.2 Hz, 2H), 1.85 - 1.71 (m, 2H), 1.59 - 1.46 (m, 1H).LCMS m/z [M+H]⁺ = 467.3 |
| **78** | 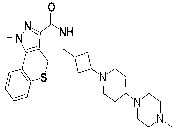 | 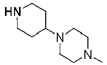 | 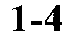 | ¹H NMR(400 MHz, DMSO-d6) δ 8.23 (s, 1H), 8.18 (HCOOH, 1.5H), 7.83 (d,J = 7.6 Hz, 1H), 7.50 (d,J = 7.4 Hz, 1H), 7.32 (p,J = 7.5 Hz, 2H), 4.18 (s, 2H), 4.13 (d,J = 1.9 Hz, 3H), 3.38 - 3.28 (m, 2H), 3.22 (t,J = 6.1 Hz, 2H), 2.81 (m, 2H), 2.54 (s, 1H), 2.44 (s, 3H), 2.30 (s, 4H), 2.14 (s, 4H), 2.04 (s, 2H), 1.84 (s, 1H), 1.68 (s, 4H), 1.51 (s, 1H), 1.32 (s, 2H).LCMS m/z [M+H]⁺ = 495.3 |
| **99** | 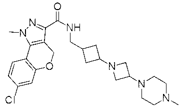 | 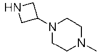 | 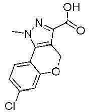 | LCMS m/z = 485.2 [M+H]⁺.¹H NMR (400 MHz, DMSO-*d*₆) δ 8.46 - 8.30 (m, 1H), 8.13 (s, HCOOH, 0.4H), 7.75 (d, *J* = 9.9 Hz, 1H), 7.20 - 7.09 (m, 2H), 4.15 (s, 3H), 3.98 - 3.57 (m, 4H), 3.44 - 3.33 (m, 4H), 3.23 - 3.02 (m, 4H), 3.01 - 2.92 (m, 2H), 2.90 - 2.75 (m, 2H), 2.60 - 2.52 (m, 2H), 2.34 - 1.94 (m, 4H), 1.91 - 1.66 (m, 1H). |
| **110** | 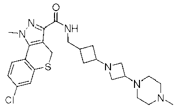 | 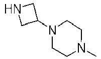 | 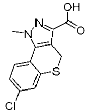 | ¹H NMR (400 MHz, DMSO-*d*₆) δ 8.34 (t, *J* = 6.0 Hz, 1H), 8.22 (s, HCOOH, 2.1H), 7.84 (d, *J* = 8.5 Hz, 1H), 7.61 (s, 1H), 7.39 (d, *J =* 8.4 Hz, 1H), 4.23 - 4.20 (m, 2H), 4.12 (s, 3H), 3.41 - 3.31 (m, 3H), 3.30 - 3.20 (m, 3H), 3.12 - 3.02 (m, 1H), 3.00 - 2.80 (m, 4H), 2.40 - 2.30 (m, 3H), 2.27 - 2.19 (m, 3H), 2.18 - 2.15 (m, 3H), 2.03 - 1.95 (m, 1H), 1.84 - 1.76 (m, 2H), 1.60 - 1.53 (m, 1H). LCMS m/z = 501.3 [M+H]⁺ |
| **111** | 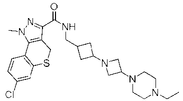 | 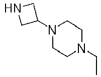 | 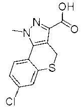 | LCMS m/z = 515.2 [M+H]⁺.¹H NMR (400 MHz, DMSO-*d*₆) δ 8.40 - 8.15 (m, 1H), 8.24 (s, HCOOH, 2H), 7.84 (dd, *J =* 8.5, 2.2 Hz, 1H), 7.62 - 7.59 (m, 1H), 7.41 - 7.36 (m, 1H), 4.23 - 4.21 (m, 2H), 4.13 - 4.11 (m, 3H), 3.35 - 2.98 (m, 9H), 2.95 - 2.58 (m, 4H), 2.39 - 2.16 (m, 7H), 2.05 - 1.90 (m, 1H), 1.82 - 1.52 (m, 2H), 1.10 - 0.82 (m, 3H). |
| **112** | 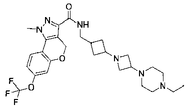 | 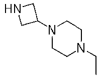 | 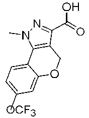 | ¹H NMR (400 MHz, DMSO-*d*₆) δ 8.44 - 8.23 (m, 1H), 8.21 (s, HCOOH, 1.43H), 7.84 (d, *J* = 8.5 Hz, 1H), 7.13 - 6.97 (m, 2H), 5.55 - 5.39 (m, 2H), 4.16 (s, 3H), 3.36 - 3.24 (m, 4H), 3.24 - 3.13 (m, 2H), 2.92 - 2.80 (m, 3H), 2.43 - 2.14 (m, 10H), 2.02 - 1.93 (m, 1H), 1.83 - 1.72 (m, 2H), 1.62 - 1.50 (m, 1H), 1.04 - 0.87 (m, 3H). LCMS m/z = 549.3 [M+H]⁺ |
| **114** | 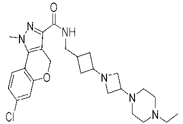 | 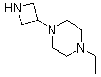 | 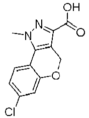 | LCMS: *m*/*z* = 499.2 [M+H]⁺; ¹H NMR (400 MHz, DMSO-*d*₆) δ 8.42 - 8.25 (m, 1H), 8.25 - 8.22 (m, 1H), 7.73 (dd, *J =* 8.2, 1.7 Hz, 1H), 7.18 - 7.10 (m, 2H), 5.49 - 5.42 (m, 2H), 4.15 - 4.13 (m, 3H), 3.45 - 3.33 (m, 2H), 3.29 - 3.07 (m, 3H), 3.01 - 2.83 (m, 3H), 2.47 - 2.16 (m, 9H), 2.05 - 1.95 (m, 1H), 1.89 - 1.74 (m, 2H), 1.65 - 1.52 (m, 1H), 1.02 - 0.93 (m, 3H). |

### Example 48

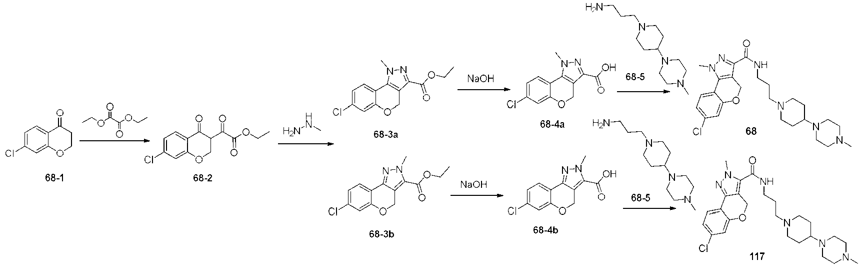

In step 1: Under a nitrogen atmosphere, **compound 68-1** (923 mg, 5.05 mmol, 1.0 eq) is dissolved in THF (9 mL) and cooled to -78°C. NaHMDS (2M in THF, 5.1 mL, 10.11 mmol, 2.0 eq) is added dropwise to the reaction mixture, and the mixture is stirred at -78°C for 0.5 h. Diethyl oxalate (1.48 g, 10.11 mmol, 2.0 eq) is dissolved in THF (4.5 mL) and added dropwise to the reaction mixture. The reaction mixture is stirred at -78°C for 10 min, then warmed to room temperature and stirred for 1.5 h. After reaction, the reaction is quenched with saturated NH₄Cl solution and extracted with ethyl acetate (3 × 40 mL). The combined organic phases are washed with saturated brine (50 mL), dried over anhydrous sodium sulfate, filtered, and the filtrate is concentrated under reduced pressure to obtain a white solid **compound 68-2** (1.9 g, 100%). LCMS *m*/*z =* 283.1 [M+H]⁺.

In step 2: **Compound 68-2** (1.8 g, 6.37 mmol) is dissolved in EtOH (45 mL). Methylhydrazine (293.4 mg, 6.37 mmol) is added to the reaction mixture. The reaction mixture is stirred at room temperature for 4 h. After reaction, the reaction mixture is concentrated. The crude product is purified by silica gel column chromatography (eluent: petroleum ether: ethyl acetate = 10:1) to obtain white solid **compound 68-3a** (350 mg, 19%) and **compound 68-3b** (405 mg, 21.7%). **68-3a:** LCMS *m*/*z* = 293.1 [M+H]⁺; ¹H NMR (400 MHz, DMSO-d₆) δ 7.43 (d, *J =* 8.3 Hz, 1H), 7.04 (d, *J =* 2.1 Hz, 1H), 7.01 (dd, J = 8.3, 2.1 Hz, 1H), 5.49 (s, 2H), 4.42 (q, *J* = 7.1 Hz, 2H), 4.20 (s, 3H), 1.41 (t, *J* = 7.1 Hz, 3H). **68-3b:** LCMS *m*/*z* = 293.1 [M+H]⁺; ¹H NMR (400 MHz, CDCl₃) δ 7.59 (d, *J =* 8.1 Hz, 1H), 6.94 (dd, *J* = 8.1, 2.0 Hz, 1H), 6.91 (d, *J =* 2.0 Hz, 1H), 5.41 (s, 2H), 4.34 (q, *J* = 7.1 Hz, 2H), 4.18 (s, 3H), 1.38 (t, *J* = 7.1 Hz, 3H).

In step 3: **Compound 68-3a** (200 mg, 0.68 mmol, 1.0 eq) is dissolved in THF (1.5 mL). A NaOH solution (NaOH (54.7 mg, 1.37 mmol, 2.0 eq) dissolved in H₂O (0.5 mL)) is slowly added. The reaction mixture is stirred at 60°C for 3 h. After reaction, the pH of the reaction mixture is adjusted to 3-4 with 2N HCl solution, and the mixture is extracted with DCM (3 × 20 mL). The combined organic phases are washed with saturated brine (40 mL), dried over anhydrous sodium sulfate, filtered, and the filtrate is concentrated under reduced pressure to obtain a white solid **compound 68-4a** (200 mg, 100%). LCMS *m*/*z* = 265.0 [M+H]⁺; ¹H NMR (400 MHz, DMSO-*d*₆) δ 7.73 (d, *J* = 8.2 Hz, 1H), 7.16 - 7.09 (m, 2H), 5.42 (s, 2H), 4.16 (s, 3H).

In step 4: **Compound 68-4a** (100 mg, 0.38 mmol, 1.0 eq), HATU (186.8 mg, 0.50 mmol, 1.3 eq), and DIPEA (146.4 mg, 1.14 mmol, 3.0 eq) are dissolved in DMF (2 mL). The reaction mixture is stirred at room temperature for 0.5 h. **Compound 68-5** (170.4 mg, 0.76 mmol, 2.0 eq) is dissolved in DMF (1 mL) and added dropwise to the reaction mixture. The reaction mixture is stirred at room temperature for 2.5 h. After reaction, the reaction mixture is diluted with water (40 mL) and extracted with ethyl acetate (3 × 30 mL). The combined organic phases are washed with saturated brine (40 mL), dried over anhydrous sodium sulfate, filtered, and the filtrate is concentrated under reduced pressure. The resulting crude product is purified by preparative column chromatography to obtain a white solid **compound 68** (26 mg, 28%). LCMS *m*/*z =* 487.3 [M+H]⁺; ¹H NMR (400 MHz, DMSO-*d*₆) δ 8.72 - 8.59 (m, 1H), 7.74 (d, *J* = 8.3 Hz, 1H), 7.16 - 7.06 (m, 2H), 5.44 (s, 2H), 4.17 (s, 3H), 3.27 - 3.23 (m, 2H), 2.96 - 2.89 (m, 2H), 2.49 - 2.41 (m, 5H), 2.39 - 2.26 (m, 5H), 2.14 (s, 3H), 2.10 - 1.94 (m, 1H), 1.90 - 1.80 (m, 2H), 1.80 - 1.70 (m, 2H), 1.69 - 1.57 (m, 2H), 1.57 - 1.43 (m, 2H).

Referring to the synthesis operation of Example 68, **compound 117** is synthesized by replacing **compound 68-3a** with **compound 68-3b** in step 3 ¹H NMR (400 MHz, DMSO-*d*₆) δ 8.32 (t, *J =* 5.6 Hz, 1H), 8.18 (s, HCOOH, 1.27H), 7.60 (d, *J =* 8.7 Hz, 1H), 7.14 - 7.00 (m, 2H), 5.40 (s, 2H), 3.98 (s, 3H), 3.30 - 3.24 (m, 2H), 3.02 (d, *J* = 11.4 Hz, 2H), 2.58 - 2.51 (m, 4H), 2.48 - 2.43 (m, 4H), 2.31 - 2.26 (m, 1H), 2.25 (s, 3H), 2.08 (t, *J* = 11.5 Hz, 2H), 1.80 - 1.68 (m, 4H), 1.49 - 1.39 (m, 2H). LCMS m/z = 487.3 [M+H]⁺.

### Example 49

Referring to the synthesis method of **compound 68** and **compound 117,** the following compounds are synthesized by replacing **compound 68-1** with ketone fragments and replacing **compound 68-5** with amine fragments.

| Compound | Structure | Ketone fragment | Amine fragment | Spectrum information |
|---|---|---|---|---|
| **69** | 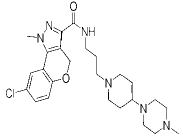 | 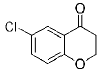 | 68-5 | LCMS: *m*/*z* = 487.2 [M+H]⁺; ¹H NMR (400 MHz, DMSO-*d*₆) δ 8.62 (t, *J =* 5.5 Hz, 1H), 8.15 (s, 1H, HCOOH), 7.71 (d, *J* = 7.8 Hz, 1H), 7.43 (d, *J =* 8.1 Hz, 1H), 7.08 (t, *J =* 8.0 Hz, 1H), 5.52 (s, 2H), 4.18 (s, 3H), 3.28 - 3.27 (m, 2H), 3.01 - 2.96 (m, 2H), 2.49 - 2.47 (m, 4H), 2.46 - 2.31 (m, 6H), 2.20 (s, 3H), 2.14 (s, 1H), 2.02 - 1.95 (m, 2H), 1.83 - 1.76 (m, 2H), 1.71 - 1.62 (m, 2H), 1.55 - 1.46 (m, 2H). |
| **71** | 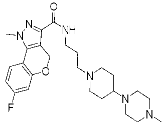 | 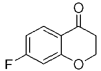 | 68-5 | LCMS m/z = 471.3 [M+H]⁺. ¹H NMR (400 MHz, Chloroform-d) δ 8.26 - 8.07 (m, 1H), 7.45 (dd, *J =* 8.5, 6.1 Hz, 1H), 6.82 - 6.62 (m, 2H), 5.56 (s, 2H), 4.15 (s, 3H), 3.49 (q, *J =* 5.8 Hz, 2H), 3.07 - 2.99 (m, 2H), 2.85 - 2.56 (m, 4H), 2.55 - 2.32 (m, 6H), 2.30 (s, 3H), 2.23 - 2.16 (m, 1H), 2.00 - 1.83 (m, 2H), 1.82 - 1.68 (m, 6H). |
| **72** | 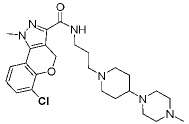 | 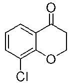 | 68-5 | LCMS: *m*/*z* = 487.2 [M+H]⁺; ¹H NMR (400 MHz, DMSO-*d*₆) δ 8.62 (t, *J =* 5.5 Hz, 1H), 8.15 (s, 1H, HCOOH), 7.71 (d, *J =* 7.8 Hz, 1H), 7.43 (d, *J =* 8.1 Hz, 1H), 7.08 (t, *J =* 8.0 Hz, 1H), 5.52 (s, 2H), 4.18 (s, 3H), 3.28 - 3.27 (m, 2H), 3.01 - 2.96 (m, 2H), 2.49 - 2.47 (m, 4H), 2.46 - 2.31 (m, 6H), 2.20 (s, 3H), 2.14 (s, 1H), 2.02 - 1.95 (m, 2H), 1.83 - 1.76 (m, 2H), 1.71 - 1.62 (m, 2H), 1.55 - 1.46 (m, 2H). |
| **73** | 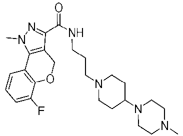 | 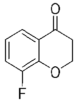 | 68-5 | LCMS *m*/*z* = 471.2 [M+H]⁺ .¹H NMR (400 MHz, CDCl₃) δ 7.29 (d, *J =* 8.0 Hz, 1H), 7.10 - 7.02 (m, 1H), 6.98 - 6.91 (m, 1H), 5.62 (s, 2H), 4.16 (s, 3H), 3.57 - 3.45 (m, 2H), 3.26 - 3.07 (m, 2H), 2.94 - 2.55 (m, 10H), 2.45 (s, 3H), 2.07 - 1.69 (m, 9H). |
| **76** | 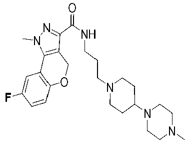 | 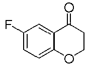 | 68-5 | LCMS m/z = 471.3 [M+H]⁺; ¹H NMR (400 MHz, DMSO-*d*₆) δ 8.65 (t, *J =* 5.5 Hz, 1H), 7.57 (dd, *J =* 9.3, 3.0 Hz, 1H), 7.13 (td, *J =* 8.7, 3.0 Hz, 1H), 7.04 (dd, *J* = 9.0, 4.9 Hz, 1H), 5.38 (s, 2H), 4.19 (s, 3H), 3.29 - 3.26 (m, 2H), 2.93 (d, *J* = 11.1 Hz, 2H), 2.49 - 2.39 (m, 4H), 2.39 - 2.21 (m, 6H), 2.14 (s, 3H), 2.08 - 2.02 (m, 1H), 1.88 - 1.79 (m, 2H), 1.79 - 1.73 (m, 2H), 1.67 - 1.60 (m, 2H), 1.55 - 1.45 (m, 2H) |
| **82** | 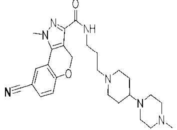 | 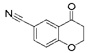 | 68-5 | LCMS: *m*/*z* = 478.3 [M+H]⁺; ¹H NMR (400 MHz, DMSO-*d*₆) δ 8.67 (t, *J* = 5.7 Hz, 1H), 8.19 (s, HCOOH, 2H), 8.14 (s, 1H), 7.74 (d, *J* = 8.5 Hz, 1H), 7.16 (d, *J* = 8.5 Hz, 1H), 5.55 (s, 2H), 4.23 (s, 3H), 3.29 - 3.27 (m, 2H), 2.97 (d, *J* = 11.1 Hz, 2H), 2.43 - 2.30 (m, 6H), 2.19 (s, 3H), 2.16 - 2.04 (m, 1H), 1.97 - 1.86 (m, 2H), 1.84 - 1.70 (m, 2H), 1.70 - 1.61 (m, 2H), 1.58 - 1.43 (m, 2H). |
| **84** | 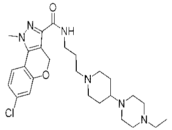 | 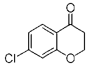 | 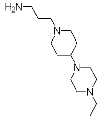 | LCMS *m*/*z* = 501.2 [M+H]⁺; ¹H NMR (400 MHz, DMSO-*d*₆) δ 8.61 (t, *J =* 5.5 Hz, 1H), 8.18 (s, HCOOH, 1.6H), 7.74 (d, *J =* 8.1 Hz, 1H), 7.21 - 7.03 (m, 2H), 5.44 (s, 2H), 4.17 (s, 3H), 3.29 - 3.26 (m, 2H), 2.97 (d, *J =* 11.2 Hz, 2H), 2.49 - 2.31 (m, 10H), 2.18 - 2.08 (m, 1H), 2.05 - 1.86 (m, 4H), 1.78 (d, *J =* 11.8 Hz, 2H), 1.70 - 1.62 (m, 2H), 1.57 - 1.45 (m, 2H), 1.00 (t, *J =* 7.2 Hz, 3H). |
| **86** | 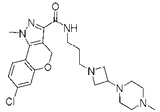 | 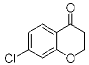 | 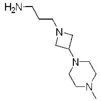 | LCMS m/z = 459.2 [M+H]⁺. ¹H NMR (400 MHz, DMSO-*d*₆) δ 8.39 (t, *J =* 6.0 Hz, 1H), 8.14 (s, 1H), 7.74 (d, *J =* 8.2 Hz, 1H), 7.17 - 7.10 (m, 2H), 5.45 (s, 2H), 4.15 (s, 3H), 3.94 - 3.87 (m, 2H), 3.62 - 3.57 (m, 2H), 3.27 - 3.25 (m, 2H), 3.18 - 3.16 (m, 1H), 2.98 - 2.92 (m, 2H), 2.85 - 2.39 (m, 8H), 2.38 (s, 3H), 1.74 - 1.61 (m, 2H). |
| **87** | 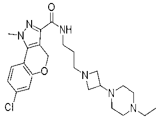 | 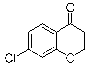 | 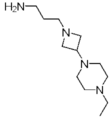 | LCMS m/z = 473.2 [M+H⁺. ¹H NMR (400 MHz, DMSO-d₆) δ 8.37 (t, *J =* 5.8 Hz, 1H), 8.18 (s, HCOOH, 2.46H), 7.74 (d, *J* = 8.1 Hz, 1H), 7.17 - 7.09 (m, 2H), 5.44 (s, 2H), 4.15 (s, 3H), 3.67 (t, *J =* 7.5 Hz, 2H), 3.29 - 3.15 (m, 4H), 3.07 - 2.95 (m, 1H), 2.71 (t, *J =* 7.1 Hz, 2H), 2.50 - 2.22 (m, 10H), 1.65 - 1.51 (m, 2H), 1.01 (t, *J =* 7.1 Hz, 3H). |
| **91** | 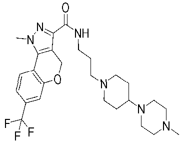 | 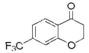 | 68-5 | LCMS: *m*/*z* = 521.2 [M+H]⁺; ¹H NMR (400 MHz, DMSO-*d*₆) δ 8.57 (t, *J* = 5.7 Hz, 1H), 8.15 (s, HCOOH, 2H), 7.94 (d, *J* = 8.1 Hz, 1H), 7.41 (d, *J =* 8.1 Hz, 1H), 7.33 (d, *J =* 1.9 Hz, 1H), 5.51 (s, 2H), 4.21 (s, 3H), 3.29 - 3.26 (m, 2H), 3.12 - 3.08 (m, 2H), 2.68 - 2.54 (m, 11H), 2.33 (s, 3H), 2.26 - 2.17 (m, 2H), 1.86 - 1.80 (m, 2H), 1.77 - 1.70 (m, 2H), 1.62 - 1.55 (m, 2H). |
| **92** | 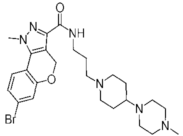 | 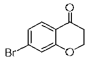 | 68-5 | LCMS: *m*/*z*=531.2 [M+H]⁺; ¹H NMR (400 MHz, DMSO-*d₆*) δ 8.61 (t, *J =* 5.5 Hz, 1H), 8.17 (s, 1H), 7.67 (d, *J =* 8.2 Hz, 1H), 7.28 - 7.24 (m, 2H), 5.44 (s, 2H), 4.16 (s, 3H), 3.29 - 3.26 (m, 2H), 2.97 (d, *J* = 11.4 Hz, 2H), 2.50 - 2.48 (m, 4H), 2.47 - 2.27 (m, 6H), 2.19 (s, 3H), 2.17 - 2.07 (m, 1H), 1.98 - 1.89 (m, 2H), 1.82 - 1.73 (m, 2H), 1.72 - 1.60 (m, 2H), 1.59 - 1.43 (m, 2H) |
| **96** | 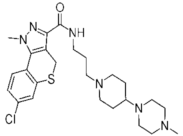 | 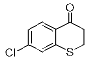 | 68-5 | LCMS: *m*/*z* = 503.2 [M+H]⁺; ¹H NMR (400 MHz, DMSO-*d*₆) δ 8.56 (t, *J =* 5.5 Hz, 1H), 8.15 (s, HCOOH, 1H), 7.85 (d, *J =* 8.6 Hz, 1H), 7.61 (d, *J =* 2.2 Hz, 1H), 7.39 (dd, *J =* 8.5, 2.2 Hz, 1H), 4.22 (s, 2H), 4.14 (s, 3H), 3.30 - 3.26 (m, 4H), 3.02 - 2.99 (m, 2H), 2.48 - 2.36 (m, 7H), 2.21 (s, 3H), 2.18 - 2.13 (m, 1H), 2.04 - 1.95 (m, 3H), 1.82 - 1.76 (m, 2H), 1.71 - 1.65 (m, 2H), 1.56 - 1.48 (m, 2H). |
| **101** | 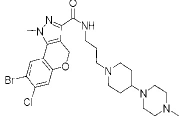 | 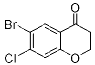 | 68-5 | ¹H NMR (400 MHz, DMSO-*d*₆) δ 8.57 (t, *J =* 5.6 Hz, 1H), 8.15 (s, 1H), 7.97 (s, 1H), 7.33 (s, 1H), 5.46 (s, 2H), 4.19 (s, 3H), 3.29 - 3.27 (m, 2H), 3.02 - 2.98 (m, 2H), 2.57 - 2.51 (m, 4H), 2.46 - 2.40 (m, 6H), 2.23 (s, 3H), 2.18 - 2.15 (m, 1H), 2.03 - 1.98 (m, 2H), 1.81 - 1.77 (m, 2H), 1.70 - 1.66 (m, 2H), 1.55 - 1.49 (m, 2H). LCMS m/z = 565.2 [M+H]⁺ |
| **102** | 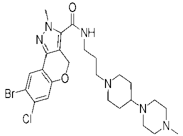 | 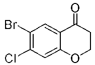 | 68-5 | ¹H NMR (400 MHz, DMSO-*d*₆) δ 8.36 (t, *J =* 5.7 Hz, 1H), 8.20 (s, 1H), 7.84 (s, 1H), 7.28 (s, 1H), 5.43 (s, 2H), 3.99 (s, 3H), 3.31 - 3.22 (m, 2H), 3.03 (d, *J =* 11.3 Hz, 2H), 2.59 - 2.50 (m, 6H), 2.54 - 2.44 (m, 4H), 2.31 - 2.25 (m, 1H), 2.25 (s, 3H), 2.12 (t, *J =* 11.6 Hz, 2H), 1.83 - 1.68 (m, 4H), 1.56 - 1.39 (m, 2H). LCMS: *m*/*z* = 565.1/567.1 [M+H]* |
| **105** | 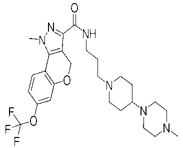 | 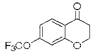 | 68-5 | LCMS m/z = 537.2 [M+H]⁺; ¹H NMR (400 MHz, DMSO-d₆) δ 8.58 (t, *J =* 5.6 Hz, 1H), 8.18 (s, HCOOH, 2.85H), 7.84 (d, *J=* 8.6 Hz, 1H), 7.12 - 7.01 (m, 2H), 5.48 (s, 2H), 4.18 (s, 3H), 3.33 - 3.25 (m, 2H), 3.02 (d, *J =* 11.7 Hz, 2H), 2.63 - 2.51 (m, 5H), 2.49 - 2.40 (m, 5H), 2.24 (s, 3H), 2.21 - 2.13 (m, 1H), 2.10 - 1.97 (m, 2H), 1.85 - 1.75 (m, 2H), 1.73 - 1.63 (m, 2H), 1.61 - 1.46 (m, 2H). |
| **106** | 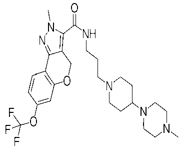 | 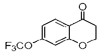 | 68-5 | ¹H NMR (400 MHz, DMSO-*d*₆) δ 8.35 (t, *J* = 5.6 Hz, 1H), 8.17 (s, HCOOH, 1.13H), 7.70 (d, *J =* 8.3 Hz, 1H), 7.02 - 6.97 (m, 2H), 5.44 (s, 2H), 3.99 (s, 3H), 3.29 - 3.24 (m, 2H), 3.02 (d, *J =* 11.4 Hz, 2H), 2.56 - 2.51 (m, 4H), 2.49 - 2.41 (m, 6H), 2.28 - 2.24 (m, 1H), 2.23 (s, 3H), 2.12 - 2.04 (m, 2H), 1.79 - 1.70 (m, 4H), 1.50 - 1.41 (m, 2H). LCMS m/z = 537.2 [M+H]⁺ |
| **97** | 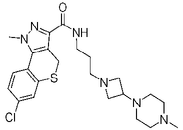 | | 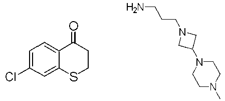 | LCMS: *m*/*z* = 475.2 [M+H]⁺; ¹H NMR (400 MHz, DMSO-*d*₆) δ 8.35 (t, *J =* 5.8 Hz, 1H), 8.22 (s, 3H), 7.84 (d, *J* = 8.5 Hz, 1H), 7.63 - 7.60 (m, 1H), 7.39 (dd, *J* = 8.5, 2.3 Hz, 1H), 4.21 (s, 2H), 4.13 (s, 3H), 3.49 - 3.45 (m, 2H), 3.31 - 3.16 (m, 4H), 2.95 - 2.82 (m, 4H), 2.39 - 2.18 (m, 7H), 2.16 (s, 3H), 1.57 - 1.48 (m, 2H). |
| **98** | 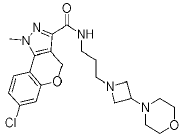 | | 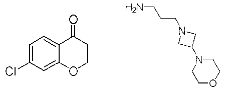 | LCMS m/z = 446.1 [M+H]⁺.¹H NMR (400 MHz, DMSO-*d*₆) δ 8.43 - 8.32 (m, 1H), 8.20 (s, HCOOH, 0.72H), 7.80 - 7.67 (m, 1H), 7.18 - 7.08 (m, 2H), 5.44 (s, 2H), 4.15 (s, 3H), 3.58 - 3.55 (m, 4H), 3.24 - 2.95 (m, 7H), 2.68 - 2.60 (m, 2H), 2.27 - 2.20 (m, 4H), 1.59 - 1.51 (m, 2H). |
| **103** | 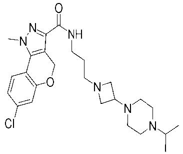 | | 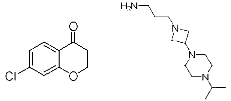 | LCMS: *m*/*z* = 487.3 [M+H]⁺; ¹H NMR (400 MHz, DMSO-d₆) δ 8.38 (t, *J =* 5.9 Hz, 1H), 8.15 (s, HCOOH, 0.77H), 7.74 (d, *J =* 8.2 Hz, 1H), 7.21 - 7.09 (m, 2H), 5.44 (s, 2H), 4.15 (s, 3H), 3.85 (t, *J =* 8.3 Hz, 2H), 3.55 - 3.45 (m,2H), 3.25 (q, *J =* 6.4 Hz, 2H), 3.20 - 3.10 (m, 2H), 3.01 - 2.92 (m, 1H), 2.93 - 2.84 (m, 2H), 2.79 - 2.70 (m, 4H), 2.52 - 2.41 (m, 2H), 1.66 (p, *J* = 6.9 Hz, 2H), 1.39 - 1.31 (m, 1H), 1.09 (d, *J =* 6.5 Hz, 6H). |
| **104** | 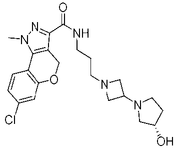 | | 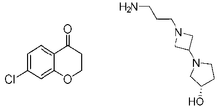 | LCMS: *m*/*z* = 446.2 [M+H]⁺; ¹H NMR (400 MHz, DMSO-*d*₆) δ 8.38 (t, *J =* 5.9 Hz, 1H), 8.15 (s, HCOOH, 0.5H), 7.74 (d, *J =* 8.2 Hz, 1H), 7.17 - 7.11 (m, 2H), 5.45 (s, 2H), 4.24 - 4.20 (m, 1H), 4.15 (s, 3H), 3.84 (t, *J* = 8.3 Hz, 2H), 3.59 - 3.54 (m, 2H), 3.36 - 3.32 (m, 2H), 3.26 - 3.23 (m, 2H), 2.90 (t, *J* = 7.3 Hz, 2H), 2.78 - 2.73 (m, 1H), 2.66 - 2.60 (m, 1H), 2.55 - 2.52 (m, 1H), 2.40 - 2.34 (m, 1H), 2.00 - 1.95 (m, 1H), 1.67 - 1.56 (m, 3H). |
| **107** | 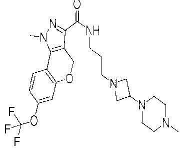 | 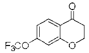 | 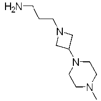 | LCMS m/z = 509.3 [M+H]⁺; ¹H NMR (400 MHz, DMSO-*d*₆) δ 8.37 (t, *J =* 5.8 Hz, 1H), 8.20 (s, 1H), 7.84 (d, *J* = 8.5 Hz, 1H), 7.11 - 7.05 (m, 1H), 7.05 - 7.02 (m, 1H), 5.48 (s, 2H), 4.17 (s, 3H), 3.51 - 3.47 (m, 2H), 3.25 - 3.20 (m, 2H), 2.94 - 2.86 (m, 3H), 2.57 - 2.51 (m, 2H), 2.44 - 2.19 (m, 8H), 2.17 (s, 3H), 1.58 - 1.48 (m, 2H). |
| **108** | 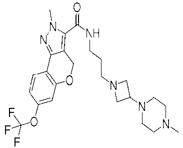 | 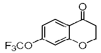 | 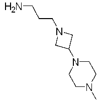 | LCMS m/z = 509.2 [M+H]⁺ .¹H NMR (400 MHz, DMSO-*d*₆) δ 8.49 - 8.39 (m, 1H), 7.76 - 7.62 (m, 1H), 7.06 - 6.91 (m, 2H), 5.47 (s, 2H), 4.00 (s, 3H), 3.86 - 3.72 (m, 2H), 3.51 - 3.39 (m, 2H), 3.32 - 3.23 (m, 2H), 3.16 - 3.07 (m, 2H), 2.95 - 2.82 (m, 3H), 2.61 - 2.53 (m, 2H), 2.43 - 2.29 (m, 7H), 1.74 - 1.62 (m, 2H). |
| **109** | 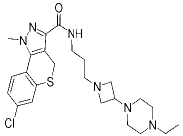 | 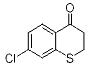 | 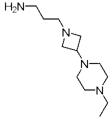 | LCMS m/z = 489.2 [M+H]⁺. ¹H NMR (400 MHz, DMSO-*d*₆) δ 8.37 (t, *J =* 5.9 Hz, 1H), 8.18 (s, 1H), 7.84 (d, *J* = 8.5 Hz, 1H), 7.61 (s, 1H), 7.39 (d, *J = 8.4* Hz, 1H), 4.21 (s, 2H), 4.13 (s, 3H), 3.60 - 3.59 (m, 4H), 3.27 - 3.22 (m, 2H), 3.12 - 3.06 (m, 2H), 3.01 - 2.95 (m, 1H), 2.65 (t, *J =* 7.0 Hz, 2H), 2.46 - 2.25 (m, 8H), 1.62 - 1.54 (m, 2H), 0.99 (t, *J* = 7.1 Hz, 3H). |
| **118** | 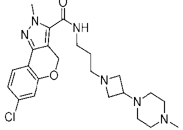 | 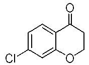 | 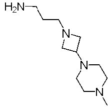 | ¹H NMR (400 MHz, DMSO-d₆) δ 8.33 (t, *J* = 5.6 Hz, 1H), 8.24 (s, HCOOH, 1H), 7.65 - 7.57 (m, 1H), 7.10 - 7.02 (m, 2H), 5.41 (s, 2H), 3.98 (s, 3H), 3.62 - 3.50 (m, 2H), 3.27 - 3.22 (m, 2H), 3.07 - 3.61 (m, 6H), 2.50 - 1.96 (m, 10H), 1.58 - 1.54 (m, 2H). LCMS m/z = 459.2 [M+H]⁺ |

### Example 50

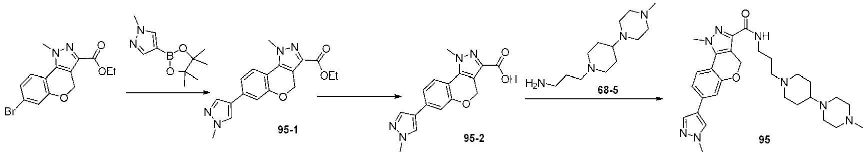

In step 1: 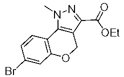 (200 mg, 0.59 mmol, 1.0 eq) and 1-Methylpyrazole-4-boronic acid pinacol ester (370 mg, 1.78 mmol, 3.0 eq) are mixed in Dioxane (4 mL) and water (1 mL). Pd(dppf)₂Cl₂ (45 mg, 0.059 mmol, 0.1 eq) and potassium carbonate (244.6 mg, 1.78 mmol, 3.0 eq) are added. The reaction mixture is purged with nitrogen three times and stirred at 90°C under a nitrogen atmosphere for 2 h. After reaction, the reaction mixture is diluted with water (50 mL) and extracted with ethyl acetate (3 × 30 mL). The combined organic phases are washed with saturated brine (50 mL), dried over anhydrous sodium sulfate, filtered, and the filtrate is concentrated under reduced pressure. The resulting crude product is purified by silica gel column chromatography to obtain **compound 95-1** (190 mg, 94.6%) as a yellow solid. LCMS m/z = 339.1 [M+H]⁺

In step 2: Referring to the synthesis method of Example 68, **compound 95** is synthesized by replacing **compound 68-3a** with **compound 95-1.LCMS** m/z = 533.3 [M+H]⁺. ¹H NMR (400 MHz, DMSO-*d*₆) δ 8.45 (t, *J* = 5.8 Hz, 1H), 8.20 (s, 1H), 8.15 (s, HCOOH, 3.5H), 7.92 (s, 1H), 7.69 (d, *J* = 8.1 Hz, 1H), 7.28 (dd, J = 8.0, 1.8 Hz, 1H), 7.24 (d, *J* = 1.8 Hz, 1H), 5.41 (s, 2H), 4.16 (s, 3H), 3.86 (s, 3H), 3.30 - 3.26 (m, 2H), 3.18 - 3.15 (m, 2H), 2.73 - 2.60 (m, 10H), 2.40 (s, 3H), 2.38 - 2.31 (m, 3H), 1.89 - 1.76 (m, 4H), 1.66 - 1.54 (m, 2H).

### Example 51

Referring to the synthesis method of Example 95, the following compounds are synthesized by replacing 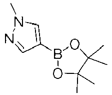 with boronic acids or borates, replacing 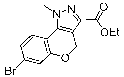 with bromo fragments, and replacing **compound 68-5** with amine fragments.

| Compound | Structure | Boronic acid or borate | Bromo fragment | Amine fragment | Spectrum information |
|---|---|---|---|---|---|
| 93 | 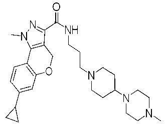 | 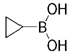 | 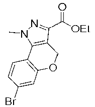 | 68-5 | LCMS m/z = 493.3 [M+H]⁺.¹H NMR (400 MHz, DMSO-*d*₆) δ 8.60-8.46 (m, 1H), 8.20 (s, HCOOH, 3.23H), 7.59-7.56 (m, 1H), 6.81-6.70 (m, 2H), 5.35 (s, 3H), 4.13 (s, 2H), 3.29 - 3.25 (m, 4H), 3.01 - 2.96 (m, 4H), 2.47 - 2.39 (m, 4H), 2.22 - 2.21 (m, 4H), 1.99 - 1.97 (m, 2H), 1.93 - 1.87 (m, 1H), 1.81 - 1.76 (m, 4H), 1.68 - 1.66 (m, 2H), 1.57 - 1.47 (m, 2H), 0.97 - 0.95 (m, 2H), 0.70 - 0.68 (m, 2H). |
| 116 | 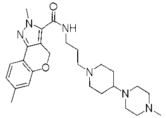 | 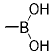 | 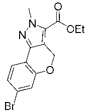 | 68-5 | ¹H NMR (400 MHz, DMSO-*d*₆) δ 8.30 (t, *J =* 5.6 Hz, 1H), 8.17 (s, 1H), 7.48 (d, *J* = 7.7 Hz, 1H), 6.82 (dd, *J = 7.8,* 1.6 Hz, 1H), 6.78 (d, *J =* 1.5 Hz, 1H), 5.32 (s, 2H), 3.96 (s, 3H), 3.29 - 3.23 (m, 2H), 3.09 (d, *J =* 11.5 Hz, 2H), 2.64 - 2.52 (m, 10H), 2.37 - 2.19 (m, 6H), 1.83 - 1.72 (m, 4H), 1.55 - 1.46 (m, 2H). LCMS m/z = 467.3 [M+H]⁺ |
| 115 | 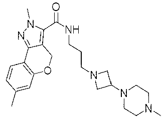 | 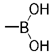 | 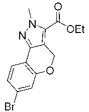 | 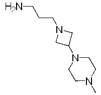 | ¹H NMR (400 MHz, DMSO-d₆) δ 8.33 (t, *J =* 5.5 Hz, 1H), 8.23 (s, HCOOH, 1H), 7.48 (d, *J* = 7.7 Hz, 1H), 6.82 (d, *J* = 7.8 Hz, 1H), 6.78 (s, 1H), 5.33 (s, 2H), 3.97 (s, 3H), 3.63 (t, *J =* 7.4 Hz, 2H), 3.27 - 3.13 (m, 4H), 3.06 - 2.90 (m, 2H), 2.70 (t, *J* = 7.1 Hz, 2H), 2.46 - 2.28 (m, 7H), 2.26 (s, 3H), 2.22 (s, 3H), 1.68 - 1.52 (m, 2H). LCMS m/z = 439.2 [M+H]⁺ |
| 120 | 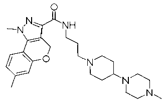 | 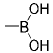 | 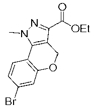 | 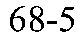 | LCMS: *m*/*z* = 467.3 [M+H]⁺; ¹H NMR (400 MHz, DMSO-*d*₆) δ 8.52 (t, *J =* 5.7 Hz, 1H), 8.19 (s, HCOOH, 2.75H), 7.61 (d, *J =* 7.9 Hz, 1H), 6.91 - 6.80 (m, 2H), 5.36 (s, 2H), 4.14 (s, 3H), 3.32 - 3.25 (m, 3H), 3.04 - 2.96 (m, 3H), 2.59 - 2.52 (m, 2H), 2.48 - 2.41 (m, 5H), 2.29 (s, 3H), 2.23 (s, 3H), 2.20 - 2.14 (m, 1H), 2.06 - 1.95 (m, 3H), 1.82 - 1.76 (m, 2H), 1.73 - 1.65 (m, 2H), 1.61 - 1.50 (m, 2H). |
| 121 | 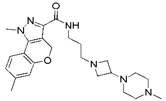 | 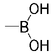 | 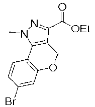 | 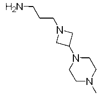 | LCMS: *m*/*z* = 439.3 [M+H]⁺; ¹H NMR (400 MHz, DMSO-*d*₆) δ 8.31 (t, *J =* 5.8 Hz, 1H), 8.20 (s, HCOOH, 3H), 7.60 (d, *J* = 7.9 Hz, 1H), 6.94 - 6.81 (m, 2H), 5.36 (s, 2H), 4.13 (s, 3H), 3.57 - 3.52 (m, 2H), 3.28 - 3.18 (m, 2H), 3.00 - 2.90 (m, 3H), 2.57 (t, *J =* 7.0 Hz, 2H), 2.43 - 2.31 (m, 4H), 2.29 - 2.19 (m, 5H), 2.18 (s, 3H), 2.04 - 1.94 (m, 1H), 1.62 - 1.48 (m, 2H). |
| 123 | 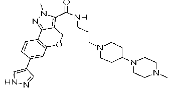 | 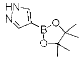 | 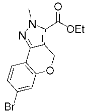 | 68-5 | ¹H NMR (400 MHz, DMSO-*d*₆) δ 9.72 (s, 1H), 8.39 (t, *J =* 5.8 Hz, 1H), 8.10 - 8.01 (m, 2H), 7.57 (d, *J =* 7.9 Hz, 1H), 7.28 (d, *J* = 7.9 Hz, 1H), 7.22 (s, 1H), 5.38 (s, 2H), 3.99 (s, 3H), 3.33 - 3.29 (m, 4H), 3.12 - 2.86 (m, 10H), 2.77 (s, 3H), 2.70 - 2.51 (m, 3H), 2.02 - 1.62 (m, 6H). LCMS m/z = 519.4 [M+H]⁺ |
| 127 | 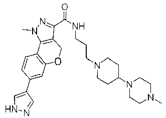 | 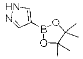 | 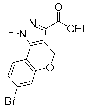 | 68-5 | LCMS m/z = 519.4 [M+H]⁺; ¹H NMR (400 MHz, DMSO-*d*₆) δ 8.52 - 8.42 (m, 1H), 8.25 (s, HCOOH, 2.94H), 8.12 (s, 2H), 7.67 (d, *J* = 8.0 Hz, 1H), 7.36 - 7.27 (m, 2H), 5.40 (s, 2H), 4.16 (s, 3H), 3.27 (q, *J* = 6.2 Hz, 2H), 3.11 (d, *J =* 11.2 Hz, 2H), 2.70 - 2.51 (m, 10H), 2.35 (s, 3H), 2.31 - 2.20 (m, 3H), 1.85 - 1.70 (m, 4H), 1.63 - 1.51 (m, 2H). |
| 133 | 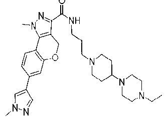 | 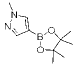 | 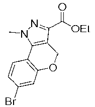 | 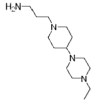 | LCMS m/z = 547.4 [M+H]⁺; ¹H NMR (400 MHz, DMSO-*d*₆) δ 8.55 - 8.45 (m, 1H), 8.20 (s, 1H), 8.15 (s, HCOOH, 2.02H), 7.91 (s, 1H), 7.69 (d, *J* = 8.0 Hz, 1H), 7.27 (d, *J =* 8.1 Hz, 1H), 7.24 (s, 1H), 5.40 (s, 2H), 4.17 (s, 3H), 3.86 (s, 3H), 3.29 - 3.27 (m, 2H), 3.08 - 3.04 (m, 2H), 2.65 - 2.52 (m, 10H), 2.49 - 2.45 (m, 2H), 2.29 - 2.22 (m, 1H), 2.18 - 2.06 (m, 2H), 1.86 - 1.79 (m, 2H), 1.75 - 1.67 (m, 2H), 1.61 - 1.50 (m, 2H), 1.04 (t, *J* = 7.2 Hz, 3H). |

### Example 52

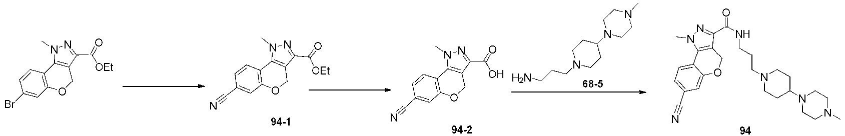

In step 1: Under a nitrogen atmosphere, 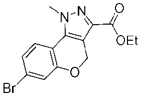 (300 mg, 0.89 mmol, 1.0 eq) is dissolved in DMF (10 mL). Zn(CN)₂ (209 mg, 1.78 mmol, 2.0 eq) and tetrakis(triphenylphosphine) palladium(0) (Pd(PPh₃)₄, 205.6 mg, 0.178 mmol, 0.2 eq) are added to the reaction mixture. The reaction mixture is purged with nitrogen three times and stirred at 130°C under a nitrogen atmosphere for 2 h. After reaction, the reaction mixture is filtered. The filtrate is diluted with water (100 mL) and extracted with ethyl acetate (3 × 30 mL). The combined organic phases are washed with saturated brine, dried over anhydrous sodium sulfate, filtered, and the filtrate is concentrated under reduced pressure. The crude product is purified by silica gel column chromatography (eluent: ethyl acetate: petroleum ether = 1:1) to obtain a yellow solid **compound 94-1** (140 mg, 55%). LCMS: m/z = 284.1 [M+H]⁺

In step 2: Referring to the synthesis method of Example 68, **compound 94** is synthesized by replacing **compound 68-3a** with **compound 94-1.** LCMS: *m*/*z =* 478.3 [M+H]⁺. ¹H NMR (400 MHz, DMSO-*d*₆) δ 8.69 (t, *J =* 5.4 Hz, 1H), 8.20 (s, 2H), 7.90 (d, *J* = 8.1 Hz, 1H), 7.57 - 7.46 (m, 2H), 5.50 (s, 2H), 4.21 (s, 3H), 3.28 (q, *J* = 6.2 Hz, 2H), 2.96 (d, *J =* 11.2 Hz, 2H), 2.50 - 2.46 (m, 4H), 2.43 - 2.30 (m, 6H), 2.17 (s, 3H), 2.14 - 2.07 (m, 1H), 1.94 - 1.85 (m, 2H), 1.80 - 1.74 (m, 2H), 1.69 - 1.61 (m, 2H), 1.55 - 1.45 (m, 2H).

### Example 53

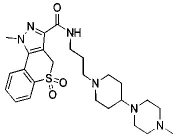

Referring to the synthesis method of Example 30, **compound 75** is synthesized by replacing **compound 1-4** with **compound** 7-2. LCMS *m*/*z* = 501.3 [M+H]⁺; ¹H NMR (400 MHz, DMSO-*d*₆) δ 8.75 - 8.63 (s, 1H), 8.04 (t, *J* = 7.8 Hz, 2H), 7.90 (t, *J* = 7.8 Hz, 1H), 7.73 (t, *J =* 7.7 Hz, 1H), 4.92 (s, 2H), 4.29 (s, 3H), 3.24 - 3.18 (m, 2H), 3.15 - 3.07 (m, 2H), 3.06 - 2.82 (m, 2H), 2.77 - 2.51 (m, 10H), 2.32 (s, 3H), 2.04 - 1.94 (m, 1H), 1.90 - 1.80 (m, 2H), 1.79 - 1.67 (m, 2H), 1.62 - 1.46 (m, 2H).

### Example 54

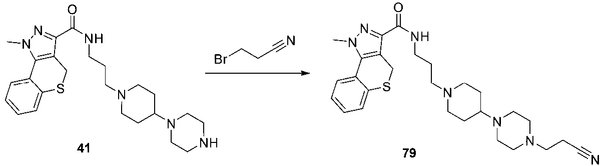

**Compound 41** (0.13 g, 0.25 mmol), 3-bromopropionitrile (0.12 g, 0.88 mmol), and potassium carbonate (0.17 g, 1.25 mmol) are added to acetonitrile (2 mL). The reaction mixture is stirred at room temperature overnight. LCMS shows that the reaction is complete. The mixture is extracted, concentrated, and purified by preparative TLC to obtain **compound 79** (0.03 g, yield: 23.98%). ¹H NMR(400 MHz, DMSO-d6) δ 8.55 (t,J = 5.6 Hz, 1H), 7.84 (dd,J = 7.6, 1.6 Hz, 1H), 7.50 (dd,J = 7.5, 1.6 Hz, 1H), 7.32 (dtd,J = 17.0, 7.5, 1.5 Hz, 2H), 4.18 (s, 2H), 4.15 (s, 3H), 3.26 (m, 6H), 2.97 (m, 2H), 2.64 (t,J = 6.7 Hz, 2H), 2.52 (m, 3H), 2.39 (m, 4H), 2.14 (dd,J = 13.3, 5.6 Hz, 1H), 2.05 - 1.87 (m, 2H), 1.79 (d,J = 11.3 Hz, 2H), 1.72 - 1.63 (m, 2H), 1.59 - 1.44 (m, 2H), 1.27 (d,J = 16.0 Hz, 1H). LCMS m/z [M+H]⁺ = 508.3.

### Example 55

Referring to the synthesis method of Example 94, the following compounds are synthesized by replacing 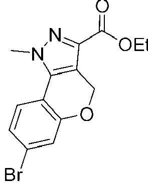 with bromo fragments and replacing **compound 68-5** with amine fragments. Fragments 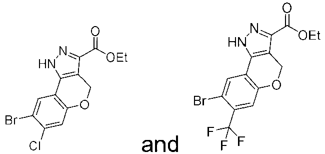 are prepared by referring to the synthetic method of intermediate 83-2.

| Compound | Structure | Bromo fragment | Amine fragment | Spectrum information |
|---|---|---|---|---|
| 113 | 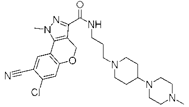 | 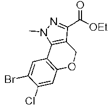 | 68-5 | LCMS m/z = 512.3 [M+H]⁺.¹H NMR (400 MHz, DMSO-*d*₆) δ 8.68 (t, *J =* 5.5 Hz, 1H), 8.23 (s, 1H), 8.17 (s, 1H), 7.41 (s, 1H), 5.60 (s, 2H), 4.22 (s, 3H), 3.29 - 3.26 (m, 2H), 3.01 - 2.97 (m, 2H), 2.59 - 2.49 (m, 4H), 2.47 - 2.38 (m, 6H), 2.21 (s, 3H), 2.18 - 2.10 (m, 1H), 1.99 - 1.93 (m, 2H), 1.82 - 1.77 (m, 2H), 1.69 - 1.64 (m, 2H), 1.57 - 1.48 (m, 2H). |
| 119 | 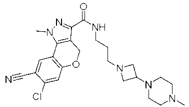 | 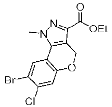 | 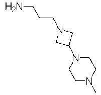 | ¹H NMR (400 MHz, DMSO-*d*₆) δ 8.42 (t, *J* = 5.8 Hz, 1H), 8.22 (s, 1H), 8.17 (s, HCOOH, 2.18H), 7.42 (s, 1H), 5.60 (s, 2H), 4.20 (s, 3H), 3.27 - 3.19 (m, 2H), 3.02 - 2.90 (m, 3H), 2.59 (t, *J =* 7.0 Hz, 2H), 2.42 - 2.14 (m, 11H), 2.02 - 1.95 (m, 2H), 1.57 - 1.51 (m, 2H). LCMS m/z = 484.2 [M+H]⁺ |
| **122** | 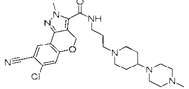 | 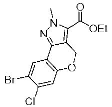 | **68-5** | ¹H NMR (400 MHz, DMSO-*d*₆) δ 8.44 (t, *J =* 5.7 Hz, 1H), 8.18 (s, HCOOH, 1.57H), 8.04 (s, 1H), 7.37 (s, 1H), 5.59 (s, 2H), 4.00 (s, 3H), 3.30 - 3.25 (m, 2H), 3.12 (d, *J =* 11.6 Hz, 2H), 2.63 - 2.58 (m, 9H), 2.38 - 2.28 (m, 7H), 1.84 - 1.77 (m, 4H), 1.59 - 1.52 (m, 2H). LCMS: *m*/*z* = 512.2 [M+H]⁺ |
| **128** | 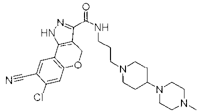 | 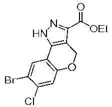 | **68-5** | LCMS m/z = 498.2 [M+H]⁺; ¹H NMR (400 MHz, DMSO-*d*₆) δ 8.48 (t, *J =* 5.8 Hz, 1H), 8.15 (s, HCOOH, 1.26H), 8.10 (s, 1H), 7.36 (s, 1H), 5.69 (s, 2H), 3.29 - 3.24 (m, 2H), 3.11 (d, *J* = 11.6 Hz, 2H), 2.68 - 2.55 (m, 10H), 2.38 - 2.22 (m, 6H), 1.84 - 1.72 (m, 4H), 1.58 - 1.49 (m, 2H). |
| **129** | 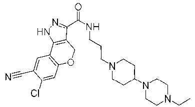 | 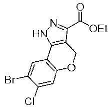 | 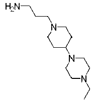 | LCMS m/z = 512.2 [M+H]⁺; ¹H NMR (400 MHz, DMSO-*d*₆) δ 8.57 - 8.51 (m, 1H), 8.13 (s, overlapped with HCOOH, 1H), 7.36 (s, 1H), 5.69 (s, 2H), 3.29 - 3.24 (m, 2H), 2.97 - 2.55 (m, 15H), 1.89 - 1.78 (m, 4H), 1.70 - 1.60 (m, 2H), 1.55 - 1.38 (m, 2H), 1.12 (d, *J* = 7.1 Hz, 3H). |
| **130** | 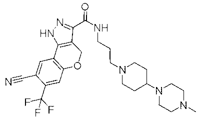 | 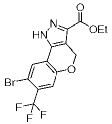 | **68-5** | LCMS: *m*/*z* = 532.25 [M+H]⁺; ¹H NMR (400 MHz, DMSO-*d*₆) δ 8.48 (t, *J =* 5.8 Hz, 1H), 8.25 (s, 1H), 8.19 (s, HCOOH, 3.19H), 7.50 (s, 1H), 5.75 (s, 2H), 3.28 - 3.25 (m, 2H), 2.97 (d, *J* = 11.3 Hz, 2H), 2.45 - 2.33 (m, 7H), 2.26 - 2.14 (m, 5H), 2.05 - 1.92 (m, 4H), 1.76 - 1.66 (m, 4H), 1.48 - 1.40 (m, 2H) |
| **131** | 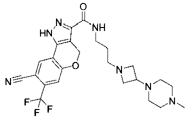 | 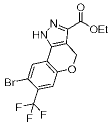 | 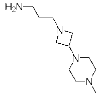 | LCMS m/z = 504.2 [M+H]⁺; ¹H NMR (400 MHz, DMSO-*d*₆) δ 8.51 - 8.45 (m, 1H), 8.24 (s, 1H), 8.20 (s, HCOOH, 1.57H), 7.50 (s, 1H), 5.75 (s, 2H), 3.54 - 3.45 (m, 2H), 3.32 - 3.19 (m, 2H), 2.98 - 2.88 (m, 4H), 2.60 - 2.53 (m, 2H), 2.36 - 2.21 (m, 7H), 2.17 (s, 3H), 1.62 - 1.51 (m, 2H). |
| **132** | 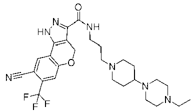 | 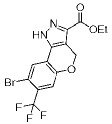 | 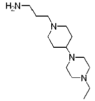 | LCMS m/z = 546.3 [M+H]⁺; ¹H NMR (400 MHz, DMSO-*d*₆) δ 8.52 (t, *J =* 5.6 Hz, 1H), 8.29 (s, 1H), 8.19 (s, HCOOH, 1.29H), 7.49 (s, 1H), 5.75 (s, 2H), 3.30 - 3.24 (m, 2H), 3.03 (d, *J* = 11.2 Hz, 2H), 2.65 - 2.53 (m, 7H), 2.49 - 2.44 (m, 5H), 2.34 - 2.25 (m, 1H), 2.10 (t, *J* = 11.6 Hz, 2H), 1.82 - 1.75 (m, 2H), 1.75 - 1.67 (m, 2H), 1.56 - 1.43 (m, 2H), 1.03 (t, *J =* 7.2 Hz, 3H). |

### Example 56

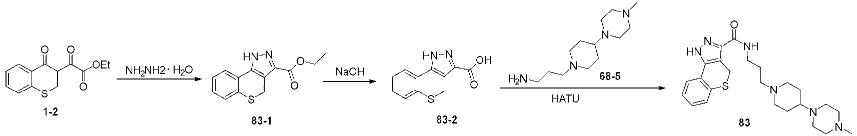

In step 1: **Compound 1-2** (6.0 g, 22.7 mmol, 1.0 eq) is dissolved in EtOH (60 mL). Hydrazine hydrate (1.42 g, 22.7 mmol, 1.0 eq) is added to the solution. The reaction mixture is stirred at room temperature overnight. After reaction, the reaction mixture is concentrated. The crude product is purified by silica gel column chromatography (eluent: petroleum ether: ethyl acetate = 8:1) to obtain **compound 83-1** (4.45 g, 75.39%) as a yellow solid. ¹H NMR (400 MHz, DMSO-*d*₆) δ 14.06 (s, 1H), 7.87 - 7.71 (m, 1H), 7.43 - 7.32 (m, 1H), 7.28 - 7.21 (m, 2H), 4.38 - 4.27 (m, 2H), 4.27 - 4.21 (m, 2H), 1.36 - 1.29 (m, 3H). LCMS m/z = 261.1 [M+H]⁺.

In step 2: Referring to the synthesis method of Example 14, **compound 83** is synthesized by replacing **compound 14-1** with **compound 83-1** and replacing **compound 7-3** with **compound 68-5**.¹H NMR (400 MHz, DMSO-*d*₆) δ 8.41 - 8.37 (m, 1H), 7.73 - 7.69 (m, 1H), 7.37 - 7.35 (m, 1H), 7.25 - 7.23 (m, 2H), 4.24 (s, 2H), 3.25 - 3.21 (m, 2H), 2.78 - 2.66 (m, 10H), 2.64 - 2.62 (m, 2H), 2.46 - 2.43 (m, 1H), 2.40 (s, 3H), 1.97 - 1.93 (m, 2H), 1.85 - 1.79 (m, 4H), 1.62 - 1.52 (m, 2H). LCMS m/z =455.2 [M+H]⁺.

### Example 57

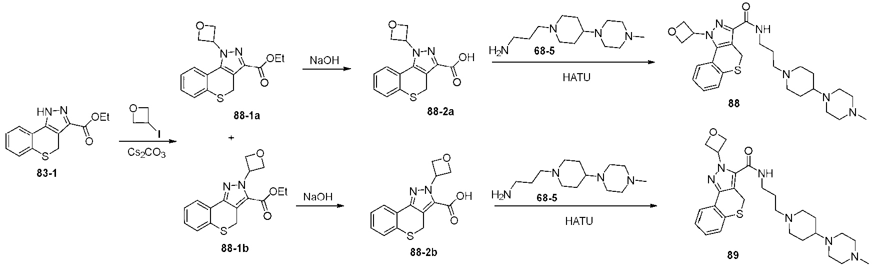

In step 1: **Compound 83-1** (2.0 g, 7.69 mmol) is dissolved in DMF (30 mL). Cesium carbonate (3.76 g, 11.53 mmol) and 3-iodooxetane (1.7 g, 9.23 mmol) are added to the reaction mixture. The reaction mixture is stirred at 110°C for 1 h. After reaction, the reaction mixture is diluted with water (200 mL) and extracted with ethyl acetate (3 × 80 mL). The combined organic phases are washed with saturated brine, dried over anhydrous sodium sulfate, filtered, and the filtrate is concentrated under reduced pressure. The resulting crude product is purified by silica gel column chromatography (petroleum ether: ethyl acetate = 8:1) to obtain a yellow solid **compound 88-1a** (170 mg, 2.88 %) and a yellow solid **compound 88-1b** (1.3 g, 53.43%). ¹H NMR (400 MHz, DMSO-*d*₆) δ 7.55 - 7.52 (m, 1H), 7.50 - 7.47 (m, 1H), 7.36 - 7.32 (m, 2H), 6.01 - 5.92 (m, 1H), 5.03 - 4.97 (m, 4H), 4.35 (q, *J* = 7.1 Hz, 2H), 4.14 (s, 2H), 1.33 (d, *J* = 7.1 Hz, 3H). LCMS m/z = 317.1 [M+H]⁺.

In step 2: **Compound 88-1a** (63 mg, 0.199 mmol, 1.0 eq) is dissolved in a mixture of MeOH (1 mL) and THF (1 mL). A NaOH solution (NaOH (25.32 mg, 0.633 mmol, 2.0 eq) dissolved in H₂O (1 mL)) is slowly added. The reaction mixture is stirred at 60°C for 2 h. After reaction, the pH of the reaction mixture is adjusted to 3-4 with 2N HCl solution, and the mixture is extracted with DCM (3 × 20 mL). The combined organic phases are washed with saturated brine, dried over anhydrous sodium sulfate, filtered, and the filtrate is concentrated under reduced pressure to obtain a yellow solid **compound 88-2a** (57 mg, 100%). LCMS m/z = 289.0 [M+H]⁺.

In step 3: **Compound 88-2a** (57 mg, 0.198 mmol), HATU (112.93 mg, 0.297 mmol), and DIPEA (76.77 mg, 0.594 mmol) are dissolved in DMF (2 mL). The reaction mixture is stirred at room temperature for 0.5 h. **Compound 68-5** (118.82 mg, 0.494 mmol) is added to the reaction mixture. The reaction mixture is stirred at room temperature overnight. After reaction, the reaction mixture is diluted with water (40 mL) and extracted with ethyl acetate (3 × 20 mL). The combined organic phases are dried over anhydrous sodium sulfate, filtered, and the filtrate is concentrated under reduced pressure. The resulting crude product is purified by preparative column chromatography to obtain **compound 88** (20.6 mg, 20.4%). ¹H NMR (400 MHz, DMSO-*d*₆) δ 8.46 (t, *J* = 6.1 Hz, 1H), 8.17 (s, 1H), 7.53 - 7.44 (m, 2H), 7.35 - 7.30 (m, 2H), 5.99 - 5.88 (m, 1H), 5.12 - 5.01 (m, 4H), 4.17 (s, 2H), 3.33 - 3.31 (m, 2H), 3.23 - 3.19 (m, 2H), 2.75 - 2.63 (m, 10H), 2.47 - 2.32 (m, 7H), 2.11 - 1.94 (m, 1H), 1.92 - 1.84 (m, 4H), 1.72 - 1.64 (m, 2H). LCMS m/z = 511.2 [M+H]⁺.

Referring to the synthesis method of Example 88, **compound 89** is synthesized by replacing **compound 88-1a** with **compound 88-1b.** ¹H NMR (400 MHz, DMSO-*d*₆) δ 8.51 - 8.41 (m, 1H), 8.22 (s, HCOOH, 1H), 7.98 - 7.83 (m, 1H), 7.43 - 7.19 (m, 3H), 5.88 - 5.73 (m, 1H), 5.05 - 4.83 (m, 4H), 4.09 (s, 2H), 3.32 - 3.10 (m, 2H), 3.04 - 2.90 (m, 2H), 2.72 - 2.58 (m, 1H), 2.41 - 2.28 (m, 10H), 2.17 (s, 3H), 2.04 - 1.89 (m, 2H), 1.80 - 1.66 (m, 4H), 1.48 - 1.33 (m, 2H). LCMS m/z = 511.2 [M+H]⁺.

### Example 58

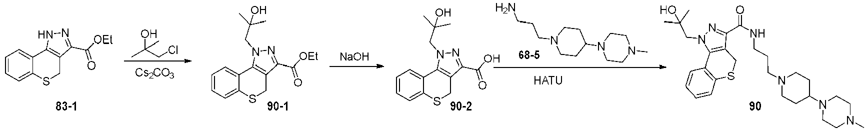

Referring to the synthesis method of Example 88, **compound 90** is synthesized by replacing 3-iodooxetane with 1-chloro-2-methyl-2-propanol. ¹H NMR (400 MHz, DMSO-*d*₆) δ 8.33 (t, *J =* 6.2 Hz, 1H), 8.23 (dd, *J* = 7.6, 1.6 Hz, 1H), 7.51 (dd, *J* = 7.4, 1.6 Hz, 1H), 7.35 - 7.26 (m, 2H), 4.92 (s, 1H), 4.30 (s, 2H), 4.16 (s, 2H), 3.33 - 3.28 (m, 2H), 3.17 - 2.54 (m, 16H), 1.90 - 1.72 (m , 6H), 1.19 (s, 6H). LCMS: *m*/*z =* 527.3 [M+H]⁺.

### Example 59

Referring to the synthesis method of Example 83, the following compounds are synthesized by replacing **compound 1-2** with 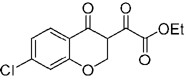 and replacing **compound 68-5** with appropriate amine fragments.

| Compound | Structure | Amine fragment | Spectrum information |
|---|---|---|---|
| **125** | 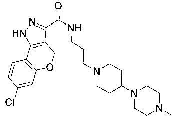 | **68-5** | LCMS: *m*/*z* = 473.3 [M+H] ⁺; ¹H NMR (400 MHz, DMSO-*d*₆) δ 8.46 (t, *J =* 5.8 Hz, 1H), 8.19 (s, HCOOH, 1.34H), 7.64 (d, *J =* 8.2 Hz, 1H), 7.16 - 6.99 (m, 2H), 5.52 (s, 2H), 3.27 (q, *J =* 6.5 Hz, 2H), 3.13 (d, *J =* 11.6 Hz, 2H), 2.67 - 2.57 (m, 10H), 2.41 - 2.24 (m, 6H), 1.91 - 1.70 (m, 4H), 1.65 - 1.52 (m, 2H). |
| **126** | 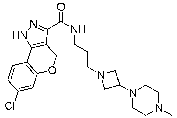 | 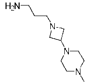 | LCMS m/z = 445.3 [M+H]⁺; ¹H NMR (400 MHz, DMSO-*d*₆) δ 8.46 (t, *J =* 5.9 Hz, 1H), 8.20 (s, HCOOH, 2.08H), 7.66 (d, *J =* 8.2 Hz, 1H), 7.12 (dd, *J =* 8.2, 2.1 Hz, 1H), 7.07 (d, *J* = 2.0 Hz, 1H), 5.55 (s, 2H), 3.76 - 3.73 (m, 2H), 3.34 - 3.22 (m, 5H), 3.09 - 3.05 (m, 1H), 2.80 (t, *J =* 7.3 Hz, 2H), 2.51 - 2.30 (m, 7H), 2.28 (s, 3H), 1.68 - 1.60 (m, 2H). |

### Example 60

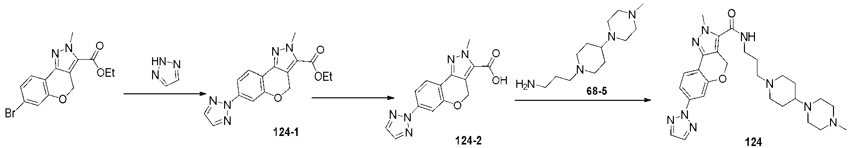

In step 1: 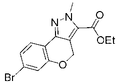 (300 mg, 0.89 mmol, 1.0 eq), 1,2,3-triazole (74 mg, 1.07 mmol, 1.2 eq), and K₃PO₄ (380 mg, 1.78 mmol, 2.0 eq) are mixed in toluene (5 mL). 4MetBuXPhos (85 mg, 0.18 mmol, 0.2 eq) and Pd₂(dba)₃ (80 mg, 0.09 mmol, 0.1 eq) are added to the mixture. The reaction mixture is purged with nitrogen three times and stirred at 120°C under a nitrogen atmosphere for 5 h. After reaction, the reaction mixture is quenched with water (50 mL) and extracted with ethyl acetate (3 × 30 mL). The combined organic phases are washed with saturated brine, dried over anhydrous sodium sulfate, filtered, and the filtrate is concentrated under reduced pressure. The crude product is purified by silica gel column chromatography (eluent: petroleum ether: ethyl acetate = 5:1) to obtain a white solid **compound 124-1** (100 mg, 34.6 %). LCMS *m*/*z =* 326.1 [M+H]⁺; ¹H NMR (400 MHz, DMSO-*d*₆) δ 8.13 (s, 2H), 7.77 (d, *J =* 8.3 Hz, 1H), 7.68 (dd, *J* = 8.4, 2.1 Hz, 1H), 7.55 - 7.50 (m, 1H), 5.51 (s, 2H), 4.31 (q, *J =* 7.1 Hz, 2H), 4.14 (s, 3H), 1.33 (t, *J* = 7.0 Hz, 3H).

In step 2: Referring to the synthesis method of Example 95, **compound 124** is synthesized by replacing **compound 95-1** with **compound** 124-1.LCMS *m*/*z =* 520.4 [M+H]⁺; ¹H NMR (400 MHz, DMSO-*d*₆) δ 8.34 (t, *J* = 5.6 Hz, 1H), 8.13 (s, 2H), 7.78 (d, *J* = 8.3 Hz, 1H), 7.69 (dd, *J =* 8.3, 2.1 Hz, 1H), 7.56 (d, *J* = 2.0 Hz, 1H), 5.46 (s, 2H), 4.00 (s, 3H), 3.30 - 3.36 (m, 2H), 3.00 (d, *J* = 11.2 Hz, 2H), 2.55 - 2.51 (m, 4H), 2.48 - 2.32 (m, 6H), 2.28 - 2.22 (m, 1H), 2.20 (s, 3H), 2.06 - 1.97 (m, 2H), 1.78 - 1.68 (m, 4H), 1.48 - 1.39 (m, 2H).

### Effect Example

### Cell IC₅₀ Assay

(1) Cell Preparation: Hepatoma cells (PLC/PRF/5, sourced from the Chinese Academy of Sciences; MHCC97H, sourced from this research institute) in good growth condition are prepared. The cells are trypsinized, diluted, and seeded into 96-well cell culture plates. 1,000-3,000 cells are added per well. The plates are then placed in a cell culture incubator at 37°C with 5% CO₂. Drugs are added after the cells have adhered for 24 h.
(2) Drug Addition: The drugs are subjected to gradient dilution, typically setting 9 concentrations. Control groups containing no drug medium and medium containing only the drug solvent are also set up. The diluted drugs are added to the cell culture plates seeded the previous day. The plates are incubated for 72 h.
(3) CCK8 Assay for Cell Viability: CCK8 is mixed with culture medium at a 1:10 ratio. 100 µL of this mixture is added to each well of the cell culture plate. After incubating for 2-4 h, the OD values are measured using a microplate reader.
(4) Data Analysis: Based on the corresponding OD values, the data are compiled, IC₅₀ curves are plotted, and the IC₅₀ values are obtained.
(5) IC₅₀ Verification: The theoretical IC₅₀ value is identified from the IC₅₀ curve. Drug concentrations at 1/2 times, 1 time, and 2 times this theoretical value are used for verification.

| Compound | Structure | IC₅₀ value of cell viability (µM) | |
|---|---|---|---|
| | | PLC/PRF/5 KSR2 OE (PLC cells overexpressing KSR2) | MHCC97H KSR2 OE (97H cells overexpressing KSR2) |
| **1** | 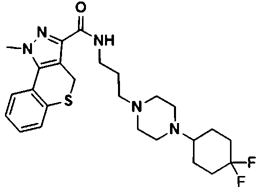 | **45** | **41** |
| **17** | 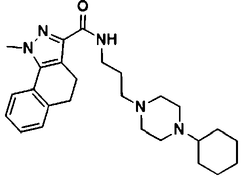 | **21** | **57** |
| **2** | 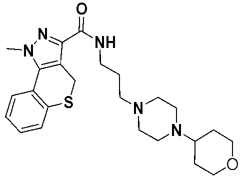 | **84** | **>100** |
| **16** | 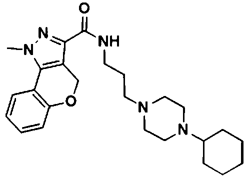 | **26** | **46** |
| **6** | 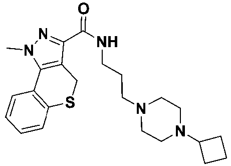 | **42** | **70** |
| **4** | 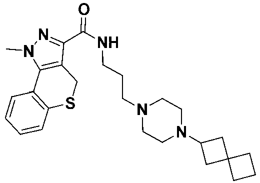 | **22** | **15** |
| **3** | 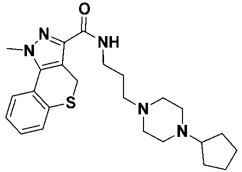 | **32** | **56** |
| **10** | 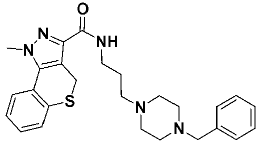 | **16** | **38** |
| **13** | 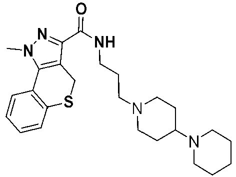 | **2** | **2** |
| **11** | 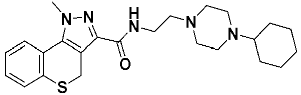 | **35** | **62** |
| **14** | 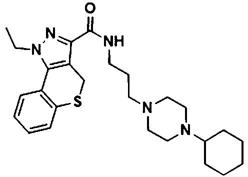 | **39** | **14** |
| **15** | 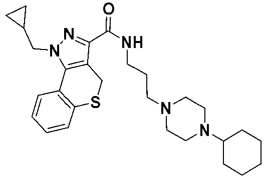 | **10** | **39** |
| **5** | 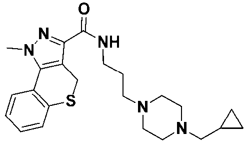 | **25** | **44** |
| **7** | 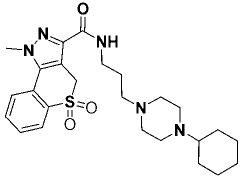 | **85** | **88** |
| **12** | 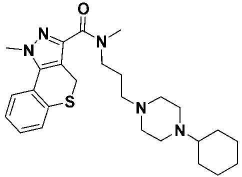 | **56** | **86** |
| **18** | 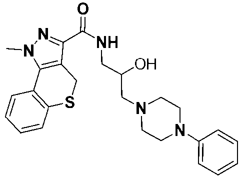 | **42** | **40** |
| **19** | 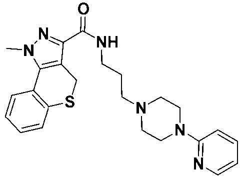 | **40** | **40** |
| **8** | 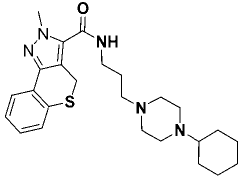 | **61** | **62** |
| **22** | 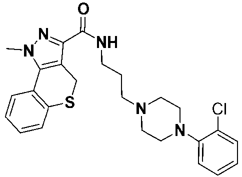 | **15** | **30** |
| **21** | 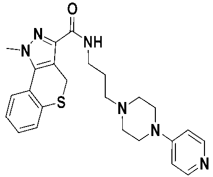 | **7** | **6** |
| **20** | 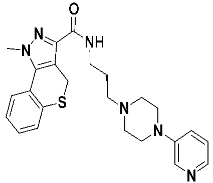 | **29** | **32** |
| **23** | 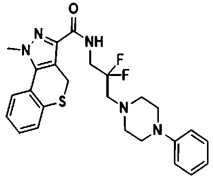 | **8** | **8** |
| **25** | 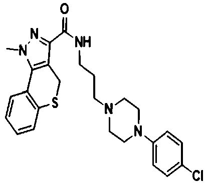 | **20** | **37** |
| **26** | 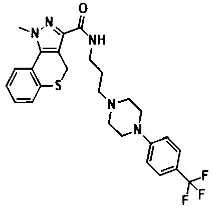 | **14** | **>100** |
| **27** | 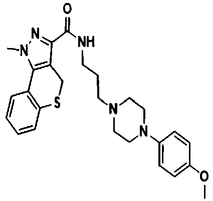 | **31** | **13** |
| **24** | 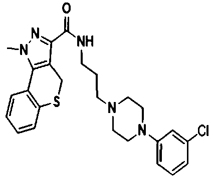 | **18** | **39** |
| **28** | 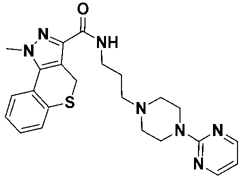 | **11** | **31** |
| **29** | 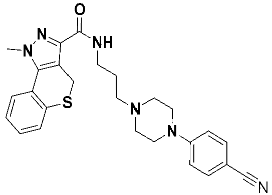 | **41** | **2** |
| **30** | 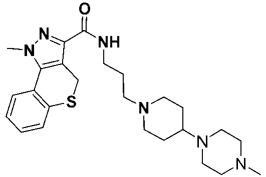 | **9** | **6** |
| **31** | 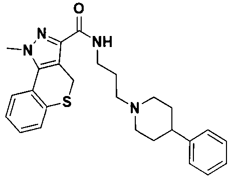 | **21** | **19** |
| **32** | 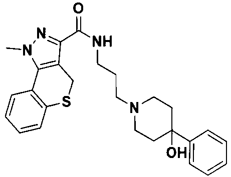 | **17** | **16** |
| **33** | 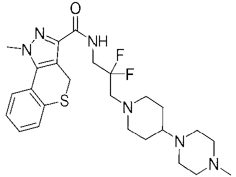 | **19** | **18** |
| **34** | 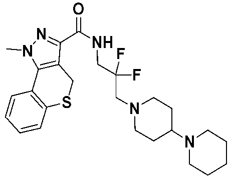 | **12** | **14** |
| **35** | 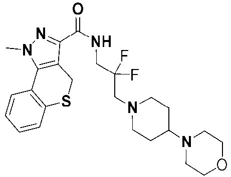 | **28** | **22** |
| **36** | 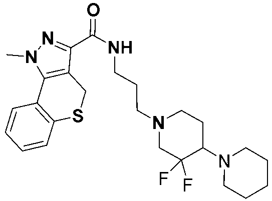 | **62** | **71** |
| **37** | 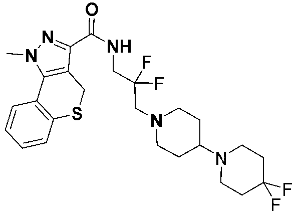 | **19** | **28** |
| **38** | 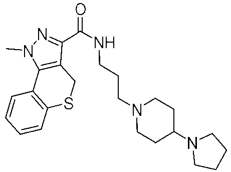 | **3** | **4** |
| **39** | 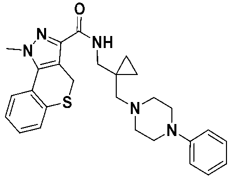 | **/** | **/** |
| **40** | 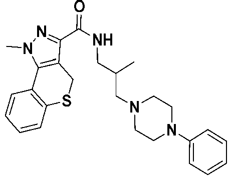 | **21** | **>100** |
| **41** | 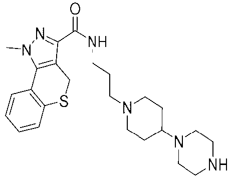 | **8** | **7** |
| **42** | 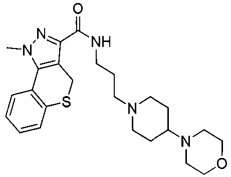 | **43** | **39** |
| **43** | 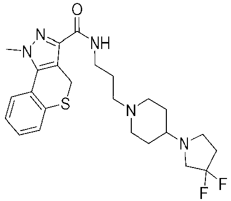 | **41** | **43** |
| **44** | 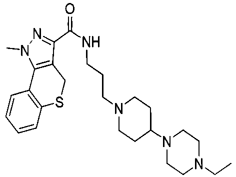 | **2** | **0.2** |
| **45** | 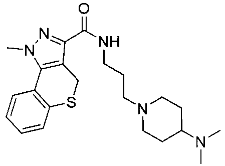 | **5** | **6** |
| **46** | 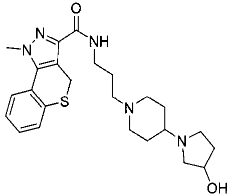 | **2** | **6** |
| **47** | 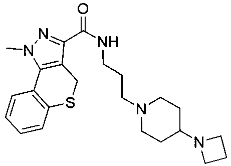 | **2** | **4** |
| **48** | 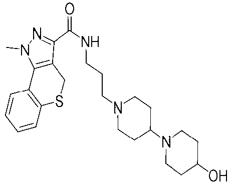 | **5** | **5** |
| **49** | 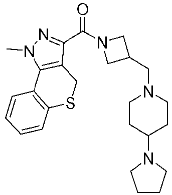 | **7** | **7** |
| **50** | 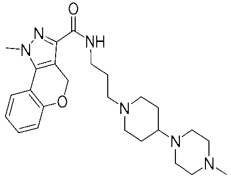 | **7** | **6** |
| **51** | 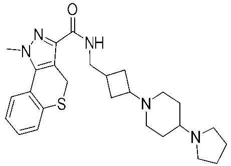 | **1** | **1** |
| **52** | 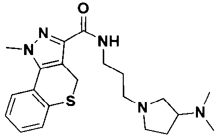 | **59** | **50** |
| **53** | 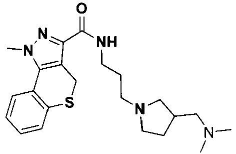 | **92** | **63** |
| **54** | 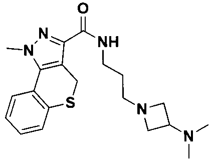 | **6** | **5** |
| **55** | 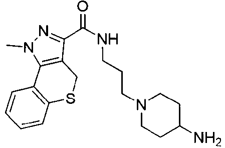 | **4** | **5** |
| **56** | 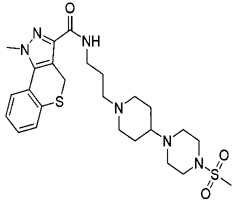 | **50** | **62** |
| **57** | 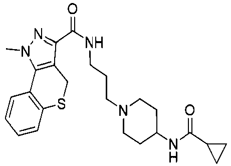 | **2** | **21** |
| **58** | 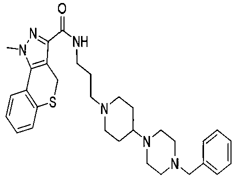 | **2** | **2** |
| **59** | 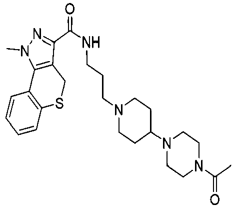 | **11** | **31** |
| **60** | 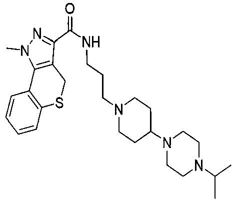 | **2** | **4** |
| **61** | 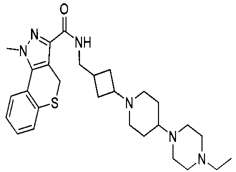 | **0.2** | **1** |
| **62** | 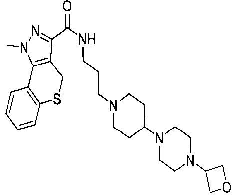 | **/** | **/** |
| **63** | 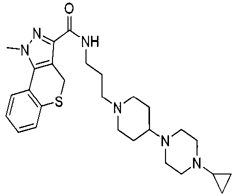 | **12** | **10** |
| **64** | 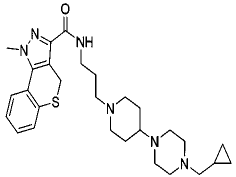 | **5** | **6** |
| **65** | 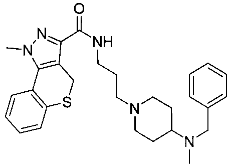 | **8** | **14** |
| **66** | 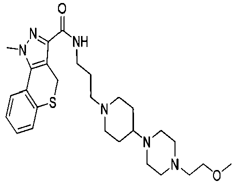 | **19** | **21** |
| **67** | 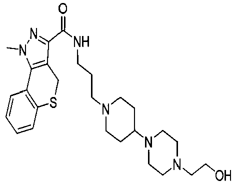 | **20** | **24** |
| **68** | 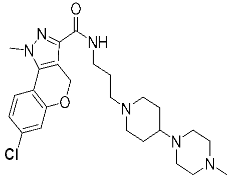 | **0.7** | **2** |
| **69** | 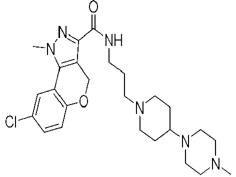 | **0.6** | **5** |
| **70** | 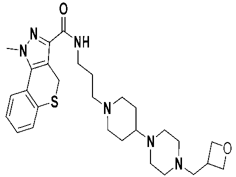 | **39** | **9** |
| **71** | 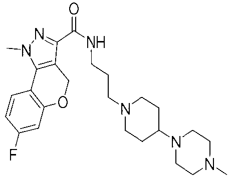 | **8** | **5** |
| **72** | 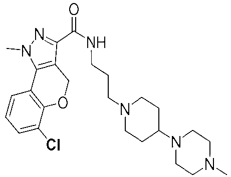 | **2** | **2** |
| **73** | 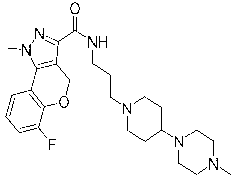 | **12** | **3** |
| **74** | 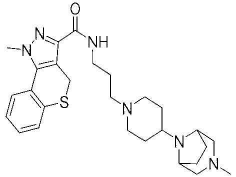 | **10** | **8** |
| **75** | 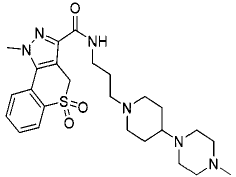 | **18** | **105** |
| **76** | 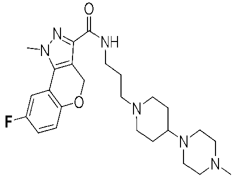 | **18** | **22** |
| **77** | 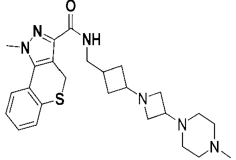 | **21** | **27** |
| **78** | 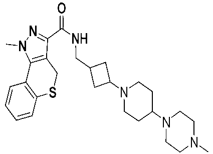 | **12** | **6** |
| **79** | 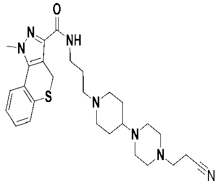 | **57** | **40** |
| **80** | 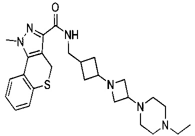 | **28** | **8** |
| **81** | 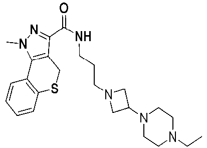 | **26** | **11** |
| **82** | 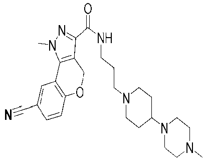 | **8** | **8** |
| **83** | 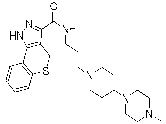 | **18** | **10** |
| **84** | 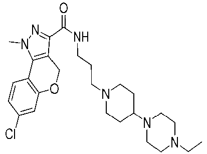 | **2** | **1** |
| **85** | 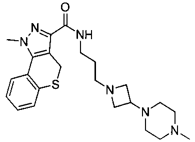 | **25** | **19** |
| **86** | 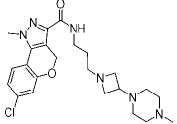 | **6** | **6** |
| **87** | 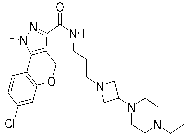 | **6** | **3** |
| **88** | 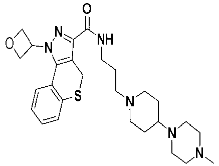 | **22** | **41** |
| **89** | 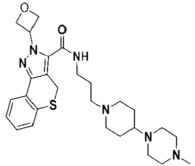 | **15** | **32** |
| **90** | 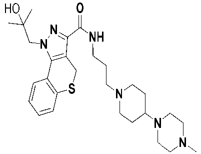 | **21** | **34** |
| **91** | 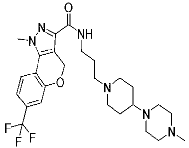 | **2** | **4** |
| **92** | 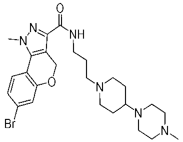 | **4** | **4** |
| **93** | 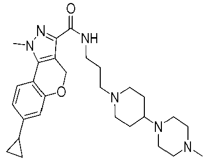 | **3** | **4** |
| **94** | 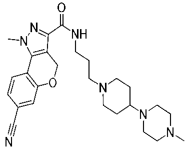 | **7** | **10** |
| **95** | 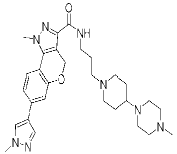 | **2** | **2** |
| **96** | 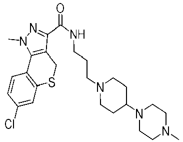 | **4** | **1** |
| **97** | 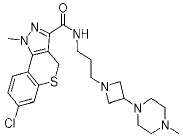 | **7** | **5** |
| **98** | 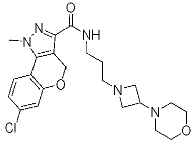 | **18** | **29** |
| **99** | 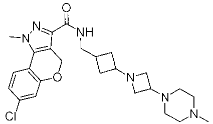 | **9** | **17** |
| **100** | 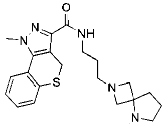 | **61** | **71** |
| **101** | 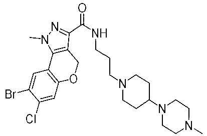 | **1** | **1** |
| **102** | 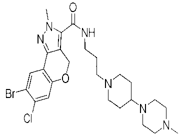 | **1** | **1** |
| **103** | 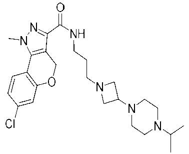 | **5** | **5** |
| **104** | 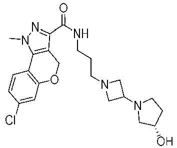 | **30** | **41** |
| **105** | 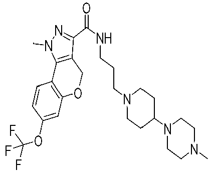 | **4** | **3** |
| **106** | 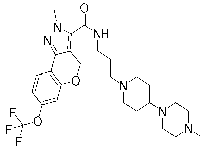 | **4** | **2** |
| **107** | 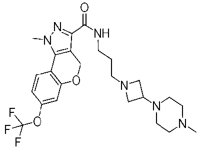 | **2** | **5** |
| **108** | 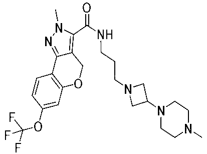 | **5** | **8** |
| **109** | 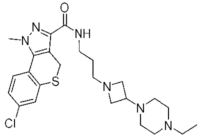 | **7** | **10** |
| **110** | 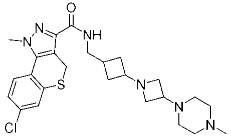 | **4** | **9** |
| **111** | 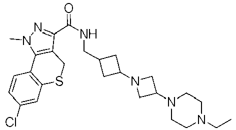 | **6** | **9** |
| **112** | 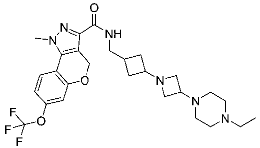 | **3** | **4** |
| **113** | 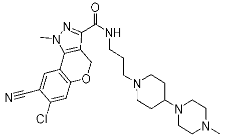 | **1** | **0.8** |
| **114** | 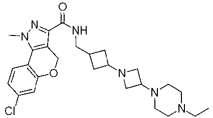 | **4** | **2** |
| **115** | 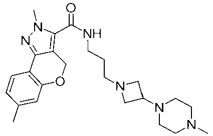 | **43** | **18** |
| **116** | 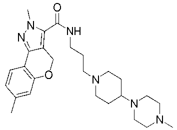 | **18** | **7** |
| **117** | 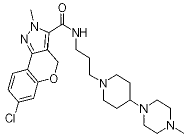 | **6** | **0.7** |
| **118** | 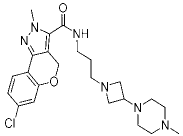 | **10** | **5** |
| **119** | 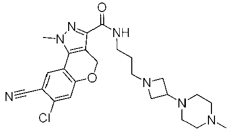 | **5** | **7** |
| **120** | 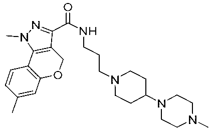 | **8** | **10** |
| **121** | 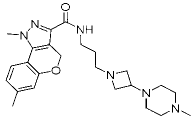 | **18** | **20** |
| **122** | 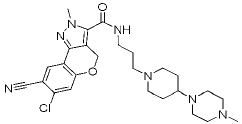 | **4** | **5** |
| **123** | 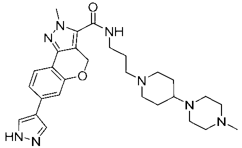 | **114** | **85** |
| **124** | 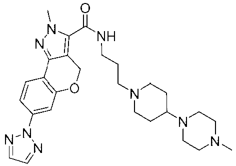 | **2** | **5** |
| **125** | 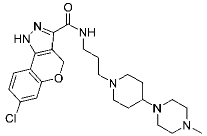 | **1** | **1** |
| **126** | 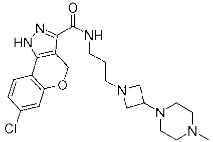 | **9** | **7** |
| **127** | 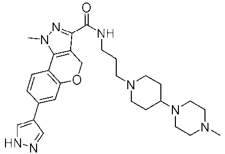 | **8** | **4** |
| **128** | 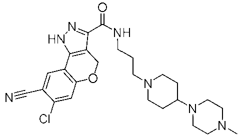 | **4** | **6** |
| **129** | 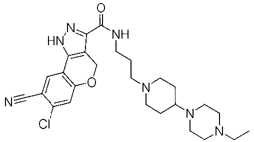 | **5** | **4** |
| **130** | 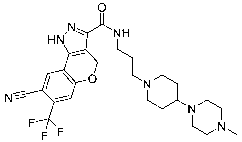 | **10** | **4** |
| **131** | 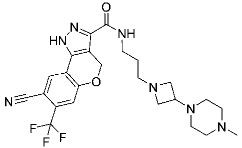 | **14** | **9** |
| **132** | 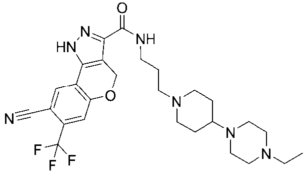 | **4** | **1** |
| **133** | 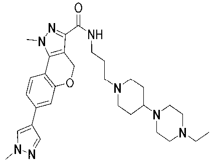 | **2** | **1** |

| | | | |
|---|---|---|---|
| "\" represents undetected. | | | |

Although specific implementations of the present disclosure are described above, those skilled in the art should understand that these are merely examples, and various changes or modifications can be made to these implementations without departing from the principle and essence of the present disclosure. Therefore, the claimed scope of the present disclosure shall be defined by the appended claims.

## Claims

1. A compound of Formula (IA) or a pharmaceutically acceptable salt thereof, 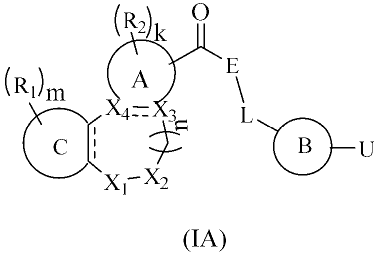
wherein ring C is selected from the group consisting of a benzene ring and "a 5-to 6-membered heteroaromatic ring having 1, 2, or 3 heteroatoms being 1, 2, or 3 selected from a group consisting of N, O, and S";
R₁ is selected from a group consisting of halogen, cyano, C₁₋₆ alkyl, C₃₋₁₀ cycloalkyl, C₁₋₆ alkoxy, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁;
R₁₋₁ is C₁₋₆ alkyl;
m and k are independently 0, 1, or 2;
X₁ is selected from a group consisting of -CH₂-, -O-, -S-, -NH-, and -S(=O)₂-;
X₂ is selected from a group consisting of -C(RₐR_{b})- and -O-;
Rₐ and R_{b} are independently selected from a group consisting of hydrogen, deuterium, and C₁₋₆ alkyl; alternatively, Rₐ, R_{b}, and a shared carbon atom thereof are joined together to form a 3- to 10-membered saturated carbocyclic ring;
n is 0 or 1;
"-̅ -̅ -̅" represents a single bond or a double bond;
X₃ and X₄ are independently selected from a group consisting of C and N;
ring A is a pyrazole ring;
R₂ is selected from a group consisting of H, halogen, C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", C₆₋₁₀ aryl, C₁₋₆ alkyl substituted with one or more R₂₋₁, and "5- to 10-membered heteroaryl having 1, 2, or 3 heteroatoms being 1, 2, or 3 selected from the group consisting of N, O, and S" substituted with one or more R₂₋₂;
R₂₋₁ and R₂₋₂ are independently selected from a group consisting of halogen, hydroxy, C₁₋₆ alkyl, -N(R₂ₐR_{2b}), C₃₋₁₀ cycloalkyl, C₁₋₆ alkoxy, "3- to 10-membered heterocycloalkyl having 1, 2, or 3 heteroatoms being 1, 2, or 3 selected from the group consisting of N, O, and S", C₆₋₁₀ aryl, and "5- to 10-membered heteroaryl having 1, 2, or 3 heteroatoms being 1, 2, or 3 selected from the group consisting of N, O, and S";
R₂ₐ and R_{2b} are independently selected from a group consisting of H and C₁₋₆ alkyl;
E is selected from a group consisting of -N(R₃)- and 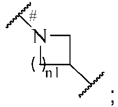 n1 is 1, 2, or 3; # represents an end linked to -C(O)- in Formula (IA);
R₃ is selected from a group consisting of hydrogen, C₁₋₆ alkyl, and C₃₋₁₀ cycloalkyl;
L is selected from a group consisting of C₁₋₆ alkylene, C₁₋₆ alkylene substituted with one or more R_{L}, "-(C₁₋₆ alkylene) -L_{A}-*", and -L_{A}-;
R_{L} is selected from a group consisting of deuterium, C₁₋₆ alkoxy, C₃₋₁₀ cycloalkyl, hydroxy, halogen, and oxo (=O); alternatively, when two R_{L} substituents are on a shared carbon atom, the two R_{L} substituents and the shared carbon atom are joined together to form a 3- to 10-membered saturated carbocyclic ring or "a 3-to 10-membered saturated heterocyclic ring having 1, 2, or 3 heteroatoms being 1, 2, or 3 selected from the group consisting of N, O, and S";
L_{A} is selected from a group consisting of -C(O)NH-, -O-, -N(R_{LA})-, vinylene, ethynylene, a 3- to 10-membered saturated carbocyclic ring, and "a 3- to 10-membered saturated heterocyclic ring having 1, 2, or 3 heteroatoms being 1, 2, or 3 selected from the group consisting of N, O, and S";
R_{LA} is selected from a group consisting of H, C₁₋₆ alkyl, C₃₋₁₀ cycloalkyl, and "3-to 10-membered heterocycloalkyl having 1, 2, or 3 heteroatoms being 1, 2, or 3 selected from the group consisting of N, O, and S";
ring B is selected from a group consisting of a 3- to 10-membered saturated or unsaturated carbocyclic ring, a 3- to 10-membered saturated or unsaturated carbocyclic ring substituted with one or more R_{B-1}, "a 3- to 10-membered saturated or unsaturated heterocyclic ring having 1, 2, or 3 heteroatoms being 1, 2, or 3 selected from the group consisting of N, O, and S", and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-1} and R_{B-2} are independently selected from a group consisting of hydroxy, halogen, oxo (=O), C₁₋₆ alkyl, C₁₋₆ alkoxy, and C₁₋₆ alkyl substituted with one or more hydroxy groups;
when U is absent, the ring B is 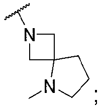
U is absent or selected from a group consisting of -N(R')(R"), C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", "5- to 10-membered heteroaryl having 1, 2, or 3 heteroatoms being 1, 2, or 3 selected from the group consisting of N, O, and S" substituted with one or more R_{U-5}, and -X-R_{U-6};
X is selected from a group consisting of -C(O)-, -SO₂-, and -O-;
R' and R" are independently selected from a group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl;
R_{U-1} is selected from a group consisting of -N(R')(R"), halogen, C₃₋₁₀ cycloalkyl, C₆₋₁₀ aryl, "5- to 10-membered heteroaryl having 1, 2, or 3 heteroatoms being 1, 2, or 3 selected from the group consisting of N, O, and S", or "5- to 10-membered heteroaryl having 1, 2, or 3 heteroatoms being 1, 2, or 3 selected from the group consisting of N, O, and S" substituted with one or more R_{U-1-1}; R_{U-1-1} is C₁₋₆ alkyl;
R_{U-2}, R_{U-3}, R_{U-4}, and R_{U-5} are independently selected from a group consisting of halogen, cyano, hydroxy, -SO₂-C₁₋₆ alkyl, -C(O)-C₁₋₆ alkyl, -C(O)O-C₁₋₆ alkyl, -C(O)NH-C₁₋₆ alkyl, C₁₋₆ alkyl, C₁₋₆ alkyl substituted with one or more R_{U-3-1}, C₁₋₆ alkoxy, C₁₋₆ alkoxy substituted with one or more R_{U-3-2}, C₃₋₁₀ cycloalkyl, "3- to 10-membered heterocycloalkyl having 1, 2, or 3 heteroatoms being 1, 2, or 3 selected from the group consisting of N, O, and S", and -O-"3- to 10-membered heterocycloalkyl having 1, 2, or 3 heteroatoms being 1, 2, or 3 selected from the group consisting of N, O, and S";
R_{U-3-1} and R_{U-3-2} are independently selected from a group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S";
R_{U-6} is selected from a group consisting of C₆₋₁₀ aryl and "5- to 10-membered heteroaryl having 1, 2, or 3 heteroatoms being 1, 2, or 3 selected from the group consisting of N, O, and S"; and
a compound of Formula (I) is not any one selected from a group consisting of following compounds:

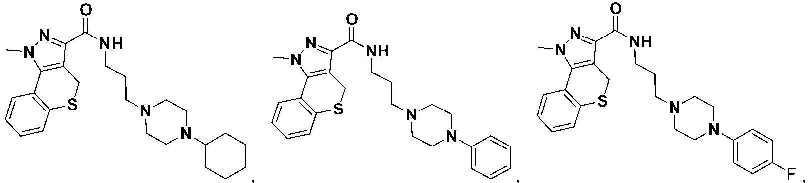

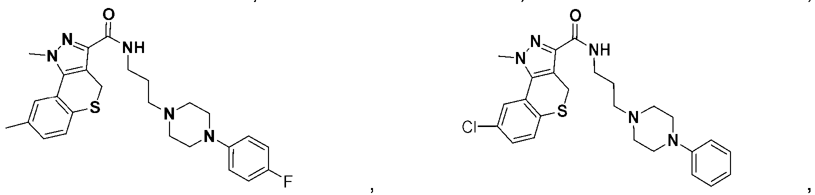

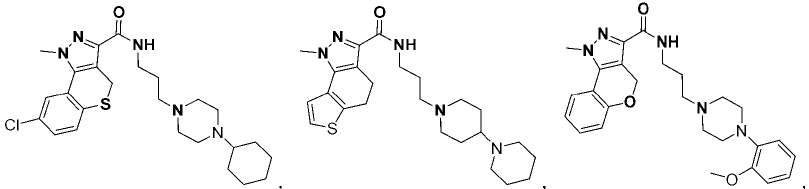

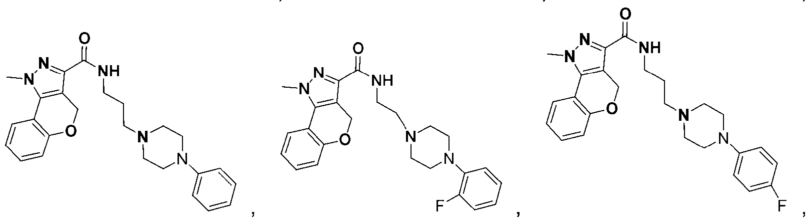

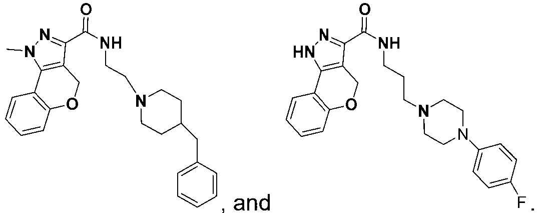

2. The compound of Formula (IA) or the pharmaceutically acceptable salt thereof according to claim 1, wherein the compound of Formula (IA) is a compound of Formula (IIA): 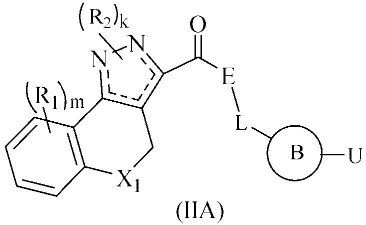
R₁ is selected from the group consisting of halogen, cyano, C₁₋₆ alkyl, C₃₋₁₀ cycloalkyl, C₁₋₆ alkoxy, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁;
R₁₋₁ is C₁₋₆ alkyl;
m and k are independently 0, 1, or 2;
X₁ is selected from a group consisting of -CH₂-, -O-, -S-, and -S(=O)₂-;
R₂ is selected from a group consisting of H, C₁₋₆ alkyl, C₁₋₆ alkyl substituted with one or more R₂₋₁, and "3- to 10-membered heterocycloalkyl having 1, 2, or 3 heteroatoms being 1, 2, or 3 selected from the group consisting of N, O, and S"; R₂₋₁ is selected from a group consisting of hydroxy and C₃₋₁₀ cycloalkyl;
"-̅ -̅ -̅" represents a single bond or a double bond;
E is selected from the group consisting of -N(R₃)- and 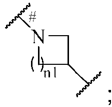 n1 is 1, 2, or 3; # represents an end linked to -C(O)- in Formula (IA);
R₃ is selected from a group consisting of hydrogen and C₁₋₆ alkyl;
L is selected from a group consisting of C₁₋₆ alkylene, C₁₋₆ alkylene substituted with one or more R_{L}, and "-(C₁₋₆ alkylene) -L_{A}-*";
R_{L} is selected from a group consisting of hydroxy and halogen; alternatively, when two R_{L} substituents are on a shared carbon atom, the two R_{L} substituents and the shared carbon atom are joined together to form a 3- to 10-membered saturated carbocyclic ring;
L_{A} is selected from a group consisting of -NH-, -N(C₁₋₆ alkyl)-, and a 3- to 10-membered saturated carbocyclic ring;
the ring B is selected from a group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is selected from a group consisting of hydroxy, halogen, C₁₋₆ alkyl, C₁₋₆ alkoxy, and C₁₋₆ alkyl substituted with one or more hydroxy groups;
when U is absent, the ring B is 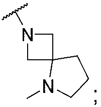
U is absent or selected from a group consisting of -N(R')(R"), C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R_{U-5};
R' and R" are independently selected from a group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl;
R_{U-1} is selected from a group consisting of -N(R')(R"), C₃₋₁₀ cycloalkyl, and C₆₋₁₀ aryl;
R_{U-2}, R_{U-3}, R_{U-4}, and R_{U-5} are independently selected from a group consisting of halogen, -SO₂-C₁₋₆ alkyl, -C(O)-C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", cyano, hydroxy, C₁₋₆ alkyl, C₁₋₆ alkyl substituted with one or more R_{U-3-1}, C₁₋₆ alkoxy, and C₁₋₆ alkoxy substituted with one or more R_{U-3-2}; and
R_{U-3-1} and R_{U-3-2} are independently selected from a group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S".

3. The compound of Formula (IA) or the pharmaceutically acceptable salt thereof according to claim 1, wherein the compound of Formula (IA) is selected from a group consisting of a compound of Formula (IIIA) and a compound of Formula (IIIB): 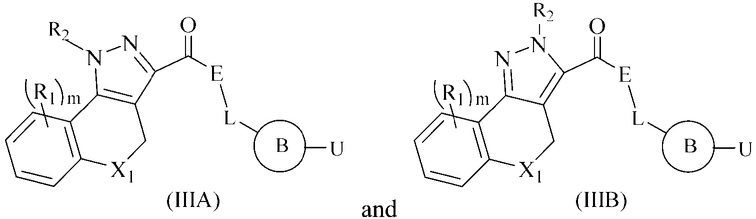
R₁ is selected from the group consisting of halogen, cyano, C₁₋₆ alkyl, C₃₋₁₀ cycloalkyl, C₁₋₆ alkoxy, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁;
R₁₋₁ is C₁₋₆ alkyl;
m is 0, 1, or 2;
X₁ is selected from a group consisting of -CH₂-, -O-, -S-, and -S(=O)₂-;
R₂ is selected from a group consisting of H, C₁₋₆ alkyl, C₁₋₆ alkyl substituted with one or more R₂₋₁, and "3- to 10-membered heterocycloalkyl having 1, 2, or 3 heteroatoms being 1, 2, or 3 selected from the group consisting of N, O, and S"; R₂₋₁ is selected from a group consisting of hydroxy and C₃₋₁₀ cycloalkyl;
E is selected from the group consisting of -N(R₃)- and 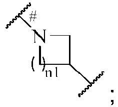 n1 is 1, 2, or 3; # represents an end linked to -C(O)- in Formula (IA);
R₃ is selected from a group consisting of hydrogen and C₁₋₆ alkyl;
L is selected from a group consisting of C₁₋₆ alkylene, C₁₋₆ alkylene substituted with one or more R_{L}, and "-(C₁₋₆ alkylene) -L_{A}-*";
R_{L} is selected from a group consisting of hydroxy and halogen; alternatively, when two R_{L} substituents are on a shared carbon atom, the two R_{L} substituents and the shared carbon atom are joined together to form a 3- to 10-membered saturated carbocyclic ring;
L_{A} is a 3- to 10-membered saturated carbocyclic ring;
the ring B is selected from a group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is selected from a group consisting of halogen and C₁₋₆ alkyl;
when U is absent, the ring B is 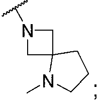
U is absent or selected from a group consisting of -N(R')(R"), C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, and "5- to 10-membered heteroaryl having 1, 2, or 3 heteroatoms being 1, 2, or 3 selected from the group consisting of N, O, and S";
R' and R" are independently selected from the group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl;
R_{U-1} is selected from a group consisting of C₃₋₁₀ cycloalkyl and C₆₋₁₀ aryl;
R_{U-2}, R_{U-3}, and R_{U-4} are independently selected from a group consisting of halogen, hydroxy, -SO₂-C₁₋₆ alkyl, -C(O)-C₁₋₆ alkyl, C₁₋₆ alkyl, C₃₋₁₀ cycloalkyl, "3-to 10-membered heterocycloalkyl having 1, 2, or 3 heteroatoms being 1, 2, or 3 selected from the group consisting of N, O, and S", C₁₋₆ alkoxy, and C₁₋₆ alkyl substituted with one or more R_{U-3-1}; and
R_{U-3-1} is selected from a group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S".

4. The compound of Formula (IA) or the pharmaceutically acceptable salt thereof according to claim 1, wherein one or more of following conditions are satisfied:
(1) R₁ is selected from a group consisting of halogen, cyano, C₁₋₆ alkyl, C₃₋₁₀ cycloalkyl, C₁₋₆ alkyl substituted with one or more halogens, C₁₋₆ alkoxy substituted with one or more halogens, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R₁₋₁;
R₁₋₁ is C₁₋₆ alkyl;
(2) R₂ is selected from the group consisting of H, C₁₋₆ alkyl, C₁₋₆ alkyl substituted with one or more R₂₋₁, and "3- to 10-membered heterocycloalkyl having 1, 2, or 3 heteroatoms being 1, 2, or 3 selected from the group consisting of N, O, and S"; R₂₋₁ is selected from a group consisting of hydroxy and C₃₋₁₀ cycloalkyl;
(3) E is -N(R₃)-;
(4) L is selected from a group consisting of C₁₋₆ alkylene, C₁₋₆ alkylene substituted with one or more R_{L}, and "-(C₁₋₆ alkylene) -L_{A}-*";
R_{L} is selected from a group consisting of hydroxy and halogen; alternatively, when two R_{L} substituents are on a shared carbon atom, the two R_{L} substituents and the shared carbon atom are joined together to form a 3- to 10-membered saturated carbocyclic ring;
L_{A} is selected from a group consisting of -NH-, -N(C₁₋₆ alkyl)-, and a 3- to 10-membered saturated carbocyclic ring, preferably the 3- to 10-membered saturated carbocyclic ring;
(5) the ring B is selected from a group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is selected from a group consisting of hydroxy, halogen, C₁₋₆ alkyl, C₁₋₆ alkoxy, and C₁₋₆ alkyl substituted with one or more hydroxy groups, preferably the halogen or the C₁₋₆ alkyl;
(6) U is absent or selected from a group consisting of -N(R')(R"), C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5-to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R_{U-5};
R' and R" are independently selected from the group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl;
R_{U-1} is selected from a group consisting of -N(R')(R"), C₃₋₁₀ cycloalkyl, and C₆₋₁₀ aryl;
R_{U-2}, R_{U-3}, R_{U-4}, and R_{U-5} are independently selected from a group consisting of halogen, -SO₂-C₁₋₆ alkyl, -C(O)-C₁₋₆ alkyl, C₃₋₁₀ cycloalkyl, "3- to 10-membered heterocycloalkyl having 1, 2, or 3 heteroatoms being 1, 2, or 3 selected from the group consisting of N, O, and S", cyano, hydroxy, C₁₋₆ alkyl, C₁₋₆ alkyl substituted with one or more R_{U-3-1}, C₁₋₆ alkoxy, and C₁₋₆ alkoxy substituted with one or more R_{U-3-2}; and
R_{U-3-1} and R_{U-3-2} are independently selected from a group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S";
preferably, U is absent or selected from a group consisting of -N(R')(R"), C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, and "5- to 10-membered heteroaryl having 1, 2, or 3 heteroatoms being 1, 2, or 3 selected from the group consisting of N, O, and S";
R' and R" are independently selected from the group consisting of hydrogen, C₁₋₆ alkyl, -C(O)-C₃₋₁₀ cycloalkyl, and C₁₋₆ alkyl substituted with one or more R‴; R‴ is C₆₋₁₀ aryl;
R_{U-1} is selected from a group consisting of C₃₋₁₀ cycloalkyl and C₆₋₁₀ aryl;
R_{U-2}, R_{U-3}, and R_{U-4} are independently selected from a group consisting of halogen, hydroxy, -SO₂-C₁₋₆ alkyl, -C(O)-C₁₋₆ alkyl, C₁₋₆ alkyl, C₃₋₁₀ cycloalkyl, "3-to 10-membered heterocycloalkyl having 1, 2, or 3 heteroatoms being 1, 2, or 3 selected from the group consisting of N, O, and S", C₁₋₆ alkoxy, and C₁₋₆ alkyl substituted with one or more R_{U-3-1}; and
R_{U-3-1} is selected from a group consisting of halogen, C₃₋₁₀ cycloalkyl, C₆₋₁₀ aryl, C₁₋₆ alkoxy, hydroxy, cyano, and "3- to 10-membered heterocycloalkyl having 1, 2, or 3 heteroatoms being 1, 2, or 3 selected from the group consisting of N, O, and S".

5. The compound of Formula (IA) or the pharmaceutically acceptable salt thereof according to claim 1, wherein one or more of following conditions are satisfied:
(1) R₁ is halogen;
(2) m and k are independently 0 or 1;
(3) R₂ is selected from a group consisting of C₁₋₆ alkyl and C₁₋₆ alkyl substituted with one or more R₂₋₁;
R₂₋₁ is C₃₋₁₀ cycloalkyl;
(4) R₃ is selected from a group consisting of hydrogen and C₁₋₆ alkyl;
(5) L is selected from a group consisting of C₁₋₆ alkylene, C₁₋₆ alkylene substituted with one or more R_{L}, and "-(C₁₋₆ alkylene) -L_{A}-*";
R_{L} is selected from a group consisting of hydroxy and halogen; alternatively, when two R_{L} substituents are on a shared carbon atom, the two R_{L} substituents are joined together to form a 3- to 10-membered saturated carbocyclic ring;
L_{A} is selected from a group consisting of -NH- and -N(C₁₋₆ alkyl)-, preferably the -NH-;
(6) the ring B is selected from a group consisting of "a 3- to 10-membered saturated or unsaturated heterocyclic ring having 1, 2, or 3 heteroatoms being 1, 2, or 3 selected from the group consisting of N, O, and S" and "a 3- to 10-membered saturated or unsaturated heterocyclic ring having 1, 2, or 3 heteroatoms being 1, 2, or 3 selected from the group consisting of N, O, and S" substituted with one or more R_{B-2};
R_{B-2} is selected from a group consisting of hydroxy, halogen, C₁₋₆ alkoxy, and C₁₋₆ alkyl substituted with one or more hydroxy groups, preferably the halogen; and
(7) U is selected from a group consisting of C₃₋₆ alkyl, C₁₋₆ alkyl substituted with one or more R_{U-1}, C₃₋₁₀ cycloalkyl, C₃₋₁₀ cycloalkyl substituted with one or more R_{U-2}, "3- to 10-membered heterocycloalkyl having 1, 2, or 3 heteroatoms being 1, 2, or 3 selected from the group consisting of N, O, and S", "3- to 10-membered heterocycloalkyl having 1, 2, or 3 heteroatoms being 1, 2, or 3 selected from the group consisting of N, O, and S" substituted with one or more R_{U-3}, C₆₋₁₀ aryl, C₆₋₁₀ aryl substituted with one or more R_{U-4}, "5- to 10-membered heteroaryl having 1, 2, or 3 heteroatoms being 1, 2, or 3 selected from the group consisting of N, O, and S", and "5- to 10-membered heteroaryl having 1, 2, or 3 heteroatoms being 1, 2, or 3 selected from the group consisting of N, O, and S" substituted with one or more R_{U-5};
R_{U-1} is selected from a group consisting of C₃₋₁₀ cycloalkyl and C₆₋₁₀ aryl;
R_{U-2}, R_{U-3}, R_{U-4}, and R_{U-5} are independently selected from a group consisting of halogen, C₃₋₁₀ cycloalkyl, "3- to 10-membered heterocycloalkyl having 1, 2, or 3 heteroatoms being 1, 2, or 3 selected from the group consisting of N, O, and S", cyano, hydroxy, C₁₋₆ alkyl, C₁₋₆ alkyl substituted with one or more R_{U-3-1}, C₁₋₆ alkoxy, and C₁₋₆ alkoxy substituted with one or more R_{U-3-2}; and
R_{U-3-1} and R_{U-3-2} are independently selected from a group consisting of halogen and C₃₋₁₀ cycloalkyl.

6. The compound of Formula (IA) or the pharmaceutically acceptable salt thereof according to claim 1, wherein one or more of following conditions are satisfied:
(1) R₃ is hydrogen;
(2) L is selected from a group consisting of C₁₋₆ alkylene and C₁₋₆ alkylene substituted with one or more R_{L};
R_{L} is selected from a group consisting of hydroxy and halogen, preferably the hydroxy;
(3) the ring B is "a 3- to 10-membered saturated or unsaturated heterocyclic ring having 1, 2, or 3 heteroatoms being 1, 2, or 3 selected from the group consisting of N, O, and S"; and
(4) U is selected from a group consisting of C₁₋₆ alkyl substituted with one or more R_{U-1}, C₃₋₁₀ cycloalkyl, C₃₋₁₀ cycloalkyl substituted with one or more R_{U-2}, "3-to 10-membered heterocycloalkyl having 1, 2, or 3 heteroatoms being 1, 2, or 3 selected from the group consisting of N, O, and S", C₆₋₁₀ aryl, C₆₋₁₀ aryl substituted with one or more R_{U-4}, and "5- to 10-membered heteroaryl having 1, 2, or 3 heteroatoms being 1, 2, or 3 selected from the group consisting of N, O, and S";
R_{U-1} is selected from a group consisting of C₃₋₁₀ cycloalkyl and C₆₋₁₀ aryl;
R_{U-2} and R_{U-4} are independently selected from a group consisting of halogen, C₁₋₆ alkoxy, and C₁₋₆ alkyl substituted with one or more R_{U-3-1}; and
R_{U-3-1} is halogen.

7. The compound of Formula (IA) or the pharmaceutically acceptable salt thereof according to any one of claims 1 to 6, wherein one or more of following conditions are satisfied:
(1) in the ring C, the "5- to 6-membered heteroaromatic ring having 1, 2, or 3 heteroatoms being 1, 2, or 3 selected from the group consisting of N, O, and S" is "a 5- to 6-membered heteroaromatic ring having 1 or 2 heteroatoms being N";
(2) the "C₁₋₆ alkyl" is selected from a group consisting of methyl, ethyl, n-propyl, isopropyl, n-butyl, tert-butyl, isobutyl, and sec-butyl;
(3) the "C₁₋₆ alkoxy" is selected from a group consisting of methoxy, ethoxy, n-propoxy, isopropoxy, n-butoxy, tert-butoxy, isobutoxy, and sec-butoxy;
(4) the "halogen" is selected from a group consisting of fluorine, chlorine, bromine, and iodine;
(5) when Rₐ, R_{b}, and the shared carbon atom thereof are joined together to form a 3- to 10-membered saturated carbocyclic ring, the "3- to 10-membered saturated carbocyclic ring" is a 3-membered saturated carbocyclic ring;
(6) the "C₃₋₁₀ cycloalkyl" is selected from a group consisting of C₃₋₆ monocyclic cycloalkyl and C₅₋₁₀ polycyclic cycloalkyl (comprising spiro cycloalkyl, fused cycloalkyl, or bridged cycloalkyl);
(7) the "3- to 10-membered heterocycloalkyl having 1, 2, or 3 heteroatoms being 1, 2, or 3 selected from the group consisting of N, O, and S" is selected from a group consisting of 4- to 6-membered monocyclic heterocycloalkyl and 5- to 10-membered polycyclic heterocycloalkyl (comprising spiro heterocycloalkyl, fused heterocycloalkyl, or bridged heterocycloalkyl);
(8) the "C₆₋₁₀ aryl" is selected from a group consisting of phenyl and naphthyl;
(9) the "5- to 10-membered heteroaryl having 1, 2, or 3 heteroatoms being 1, 2, or 3 selected from the group consisting of N, O, and S" is 5- to 6-membered monocyclic heteroaryl, preferably 5- to 6-membered monocyclic heteroaryl having 1, 2, or 3 heteroatoms being N;
(10) the "C₁₋₆ alkylene" is C₁₋₄ alkylene;
(11) when two R_{L} substituents are on a shared carbon atom, the "3- to 10-membered saturated carbocyclic ring" formed by the two R_{L} substituents and the shared carbon atom being joined together is a 3-membered saturated carbocyclic ring;
(12) when L_{A} is a 3- to 10-membered saturated carbocyclic ring, the "3- to 10-membered saturated carbocyclic ring" is a 4- to 6-membered saturated carbocyclic ring, comprising 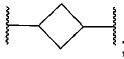 ; and (12) the "3- to 10-membered saturated or unsaturated heterocyclic ring having 1, 2, or 3 heteroatoms being 1, 2, or 3 selected from the group consisting of N, O, and S" is "a 4- to 8-membered saturated or unsaturated heterocyclic ring having 1 or 2 heteroatoms being 1 or 2 selected from a group consisting of N and O", preferably "a 5- to 6-membered saturated or unsaturated heterocyclic ring having 1 or 2 heteroatoms being N".

8. The compound of Formula (IA) or the pharmaceutically acceptable salt thereof according to claim 7, wherein one or more of following conditions are satisfied:
(1) in the ring C, the "5- to 6-membered heteroaromatic ring having 1, 2, or 3 heteroatoms being 1, 2, or 3 selected from the group consisting of N, O, and S" is selected from a group consisting of a pyridine ring and a pyrimidine ring;
(2) the "C₁₋₆ alkyl" is selected from a group consisting of methyl and ethyl;
(3) the "C₁₋₆ alkoxy" is methoxy;
(4) the "halogen" is selected from a group consisting of fluorine, chlorine, and bromine, preferably the fluorine or the chlorine, more preferably the fluorine;
(5) when Rₐ, R_{b}, and the shared carbon atom thereof are joined together to form a 3- to 10-membered saturated carbocyclic ring, the "3- to 10-membered saturated carbocyclic ring" is 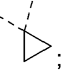
(6) the "C₃₋₆ monocyclic cycloalkyl" is selected from a group consisting of cyclopropyl, cyclobutyl, cyclopentyl, and cyclohexyl;
(7) the "C₅₋₁₀ polycyclic cycloalkyl" is C₅₋₁₀ spiro cycloalkyl, preferably 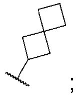
(8) the "4- to 6-membered monocyclic heterocycloalkyl" is selected from a group consisting of "4- to 6-membered monocyclic heterocycloalkyl having 1 or 2 heteroatoms being 1 or 2 selected from a group consisting of N and O" (preferably 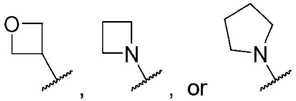 ) and "6-membered heterocycloalkyl having 1 or 2 heteroatoms being 1 or 2 selected from a group consisting of N and O" (preferably 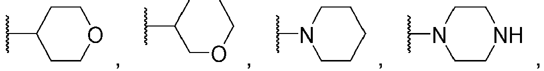 or 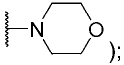
(9) the "5- to 10-membered polycyclic heterocycloalkyl" is selected from a group consisting of 5- to 10-membered spiro heterocycloalkyl and bridged heterocycloalkyl, preferably 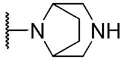 or 6-membered spiro 3-membered heterocycloalkyl, more preferably 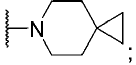
(10) the "C₆₋₁₀ aryl" is phenyl;
(11) the "5- to 10-membered heteroaryl having 1, 2, or 3 heteroatoms being 1, 2, or 3 selected from the group consisting of N, O, and S" is 5- to 6-membered monocyclic heteroaryl, the 5- to 6-membered monocyclic heteroaryl is "5- to 6-membered monocyclic heteroaryl having 1 or 2 heteroatoms being N, preferably 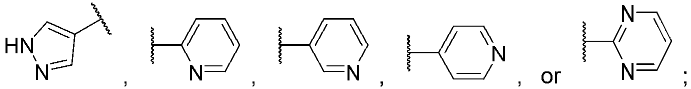 alternatively, the 5- to 6-membered monocyclic heteroaryl is 5- to 6-membered monocyclic heteroaryl having 3 heteroatoms being N, preferably 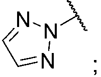
(12) the "C₁₋₆ alkylene" is selected from a group consisting of methylene, 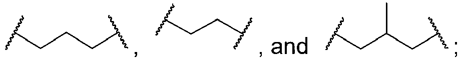
(13) when two R_{L} substituents are on a shared carbon atom, the "3- to 10-membered saturated carbocyclic ring" formed by the two R_{L} substituents and the shared carbon atom being joined together is 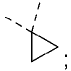 and
(14) the "3- to 10-membered saturated or unsaturated heterocyclic ring having 1, 2, or 3 heteroatoms being 1, 2, or 3 selected from the group consisting of N, O, and S" is selected from a group consisting of

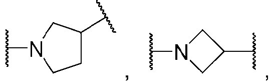

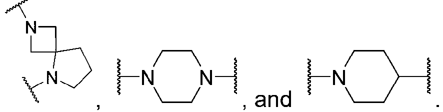

9. The compound of Formula (IA) or the pharmaceutically acceptable salt thereof according to claim 1, wherein one or more of following conditions are satisfied:
(1) the ring C is a benzene ring;
(2) R₁ is selected from a group consisting of fluorine, chlorine, bromine, cyano, trifluoromethyl, cyclopropyl, trifluoromethoxy, methyl, 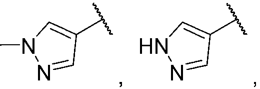 and 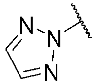 ; m is 0, 1, or 2;
(3) X₁ is selected from a group consisting of -CH₂-, -O-, -S-, and -S(=O)₂-; X₂ is -CH₂-; n is 0; X₃ and X₄ are C;
(4) R₂ is selected from a group consisting of hydrogen, methyl, ethyl, 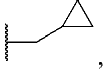 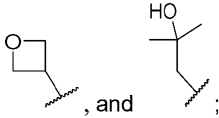 k is 0 or 1;
(5) a structural moiety 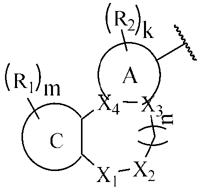 is selected from a group consisting of 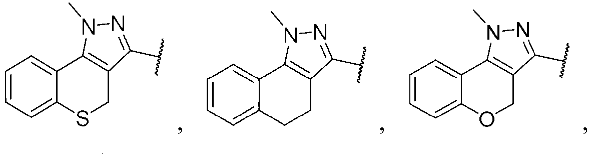 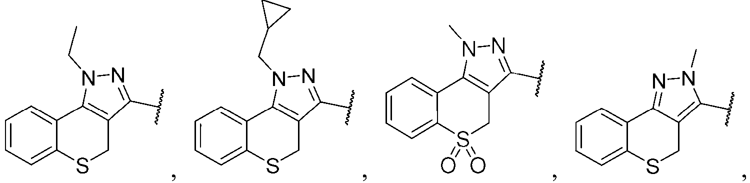 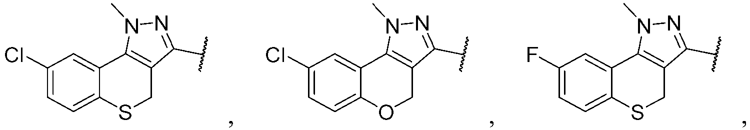 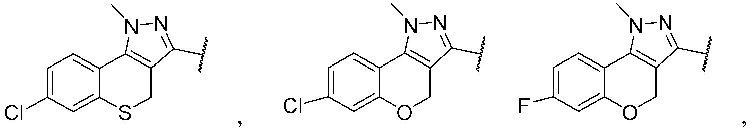 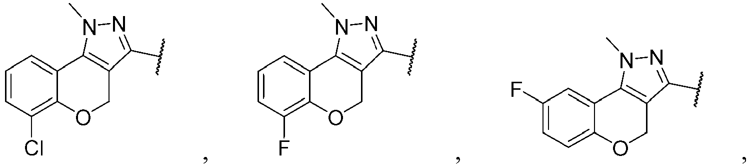 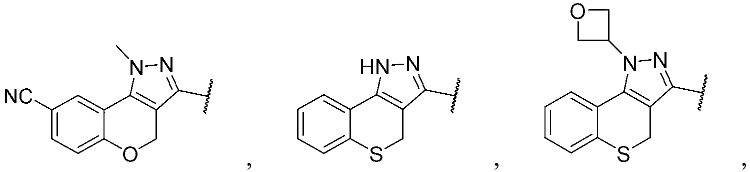 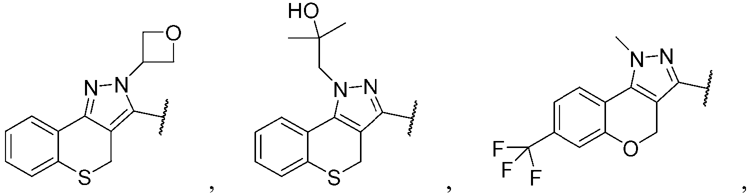 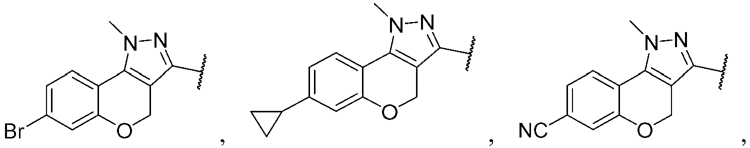 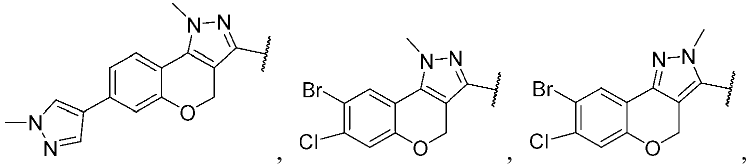 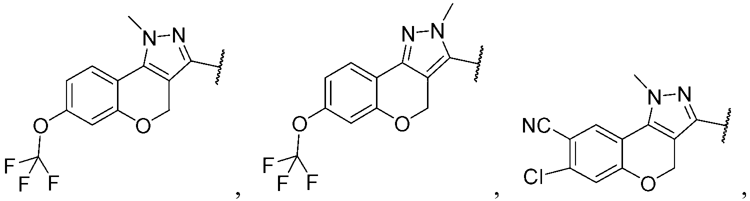 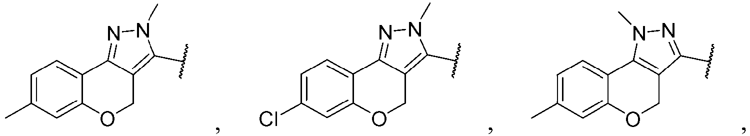 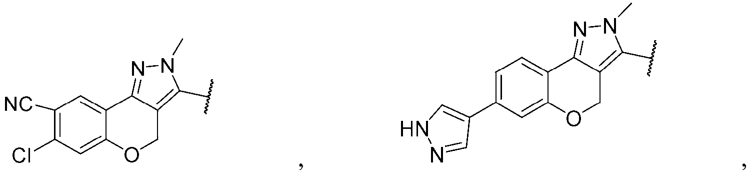 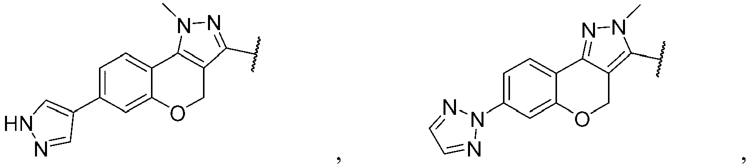 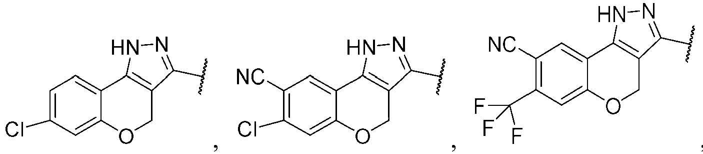 , and 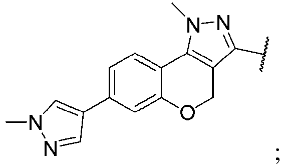
(6) R₃ is selected from a group consisting of hydrogen and methyl;
(7) L is selected from a group consisting of methylene, 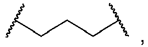 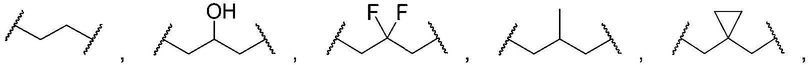 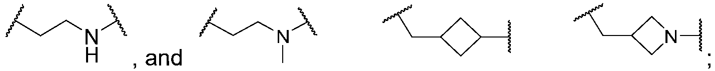
(8) the ring B is selected from a group consisting of 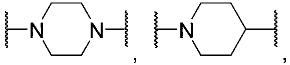 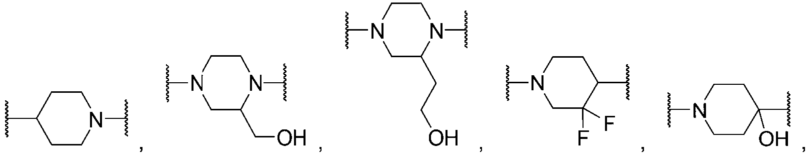 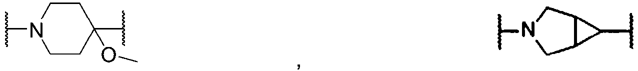 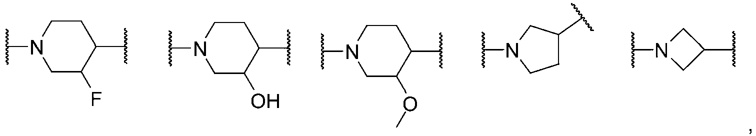 and 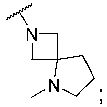
(9) U is selected from a group consisting of 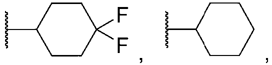 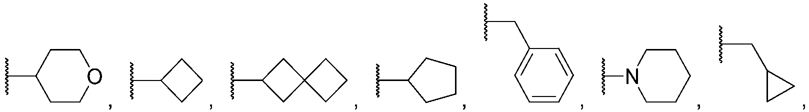 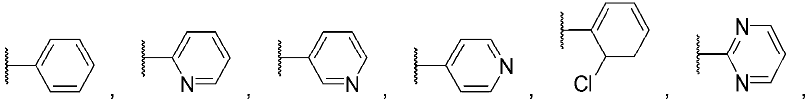 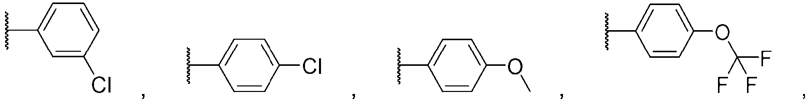 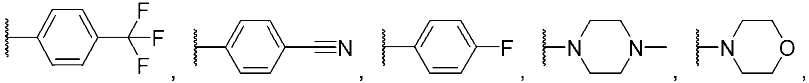 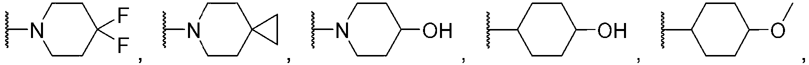 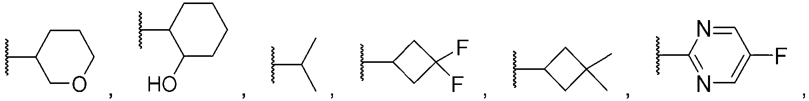 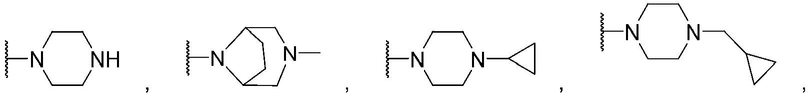 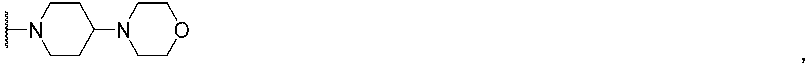 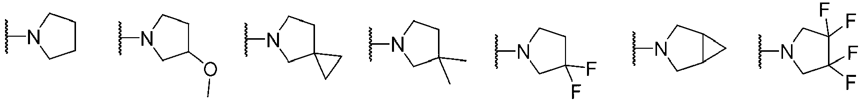 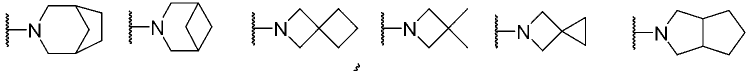 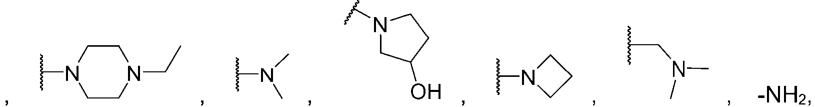 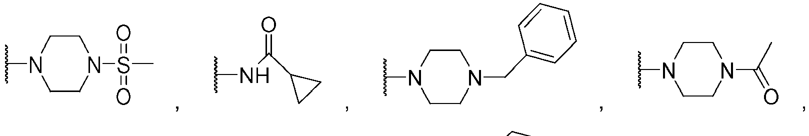 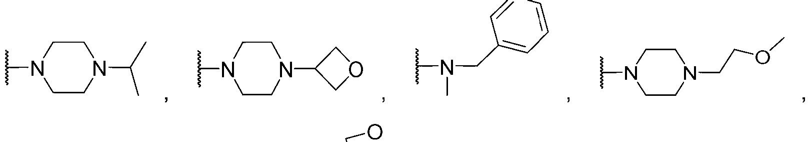 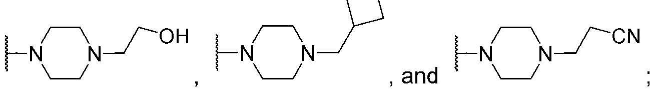 and
(10) E is selected from a group consisting of -NH-, -N(CH₃)-, and

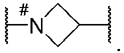

10. The compound of Formula (IA) or the pharmaceutically acceptable salt thereof according to claim 1, wherein the compound of Formula (IA) is any one selected from a group consisting of following compounds:

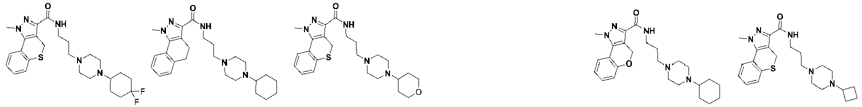

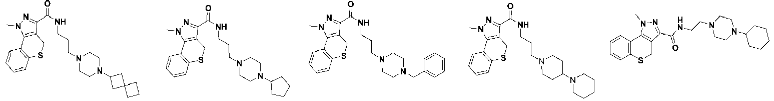

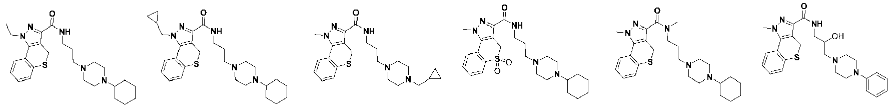

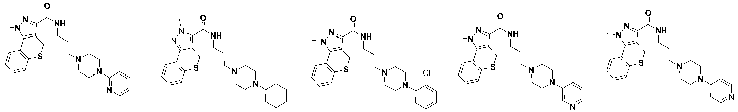

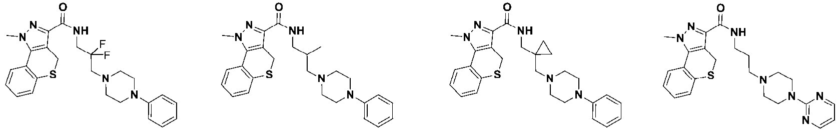

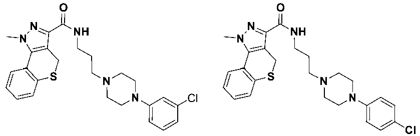

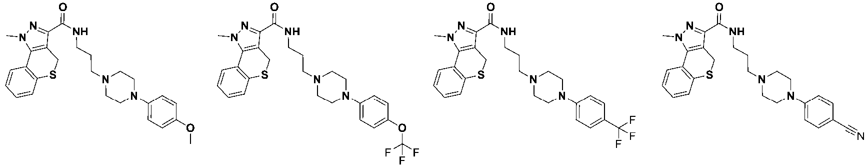

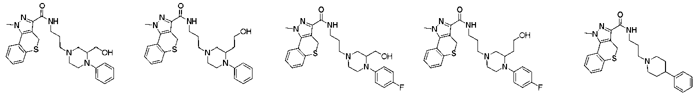

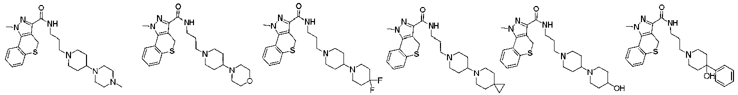

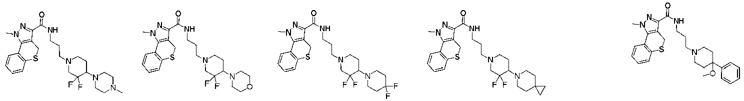

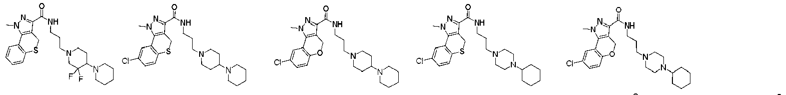

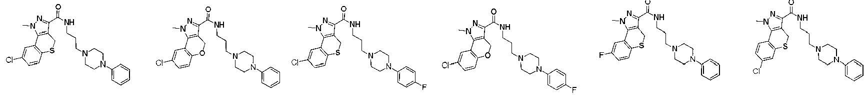

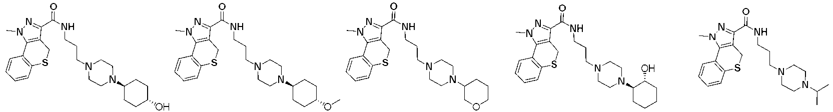

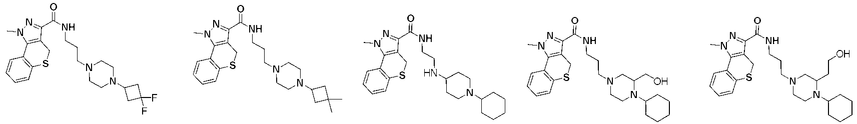

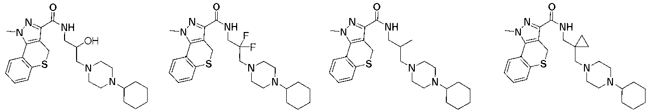

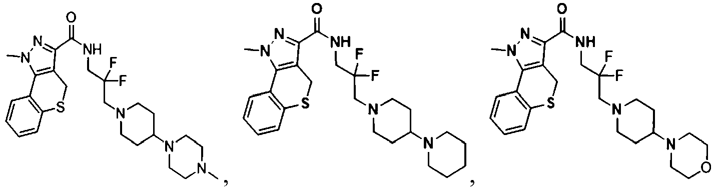

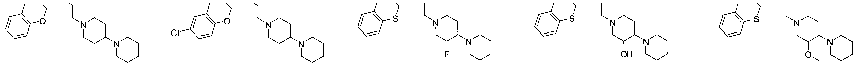

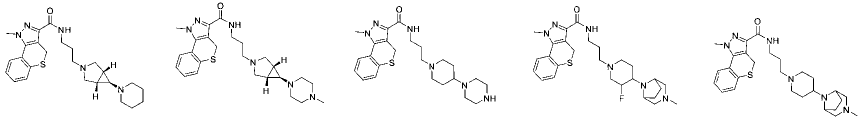

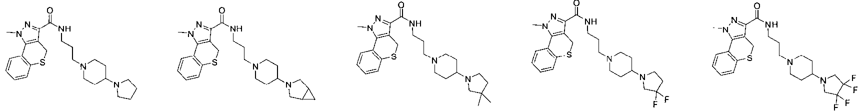

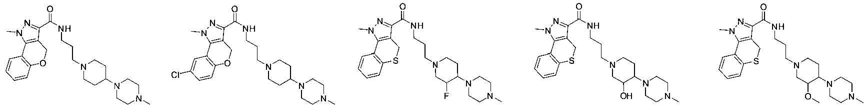

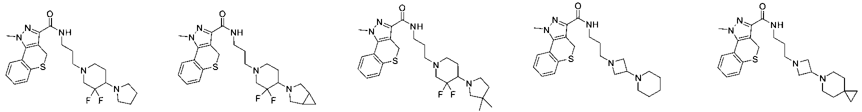

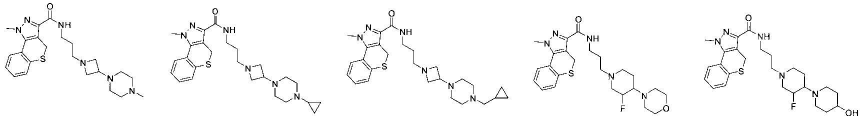

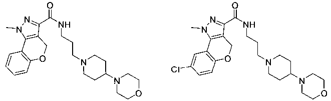

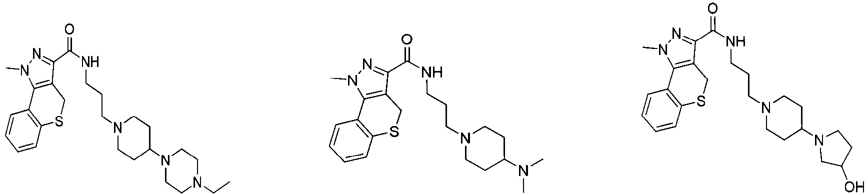

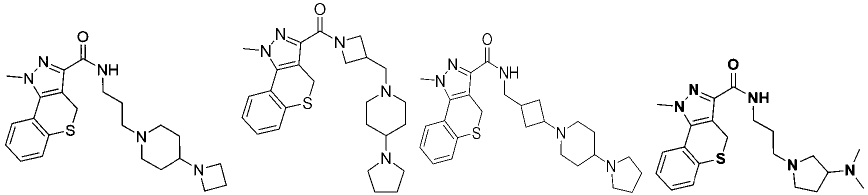

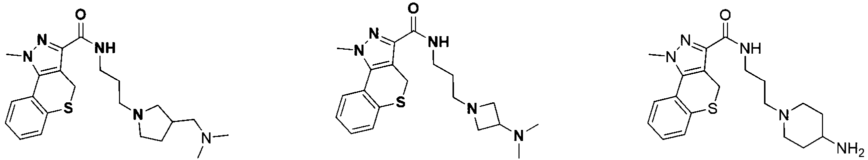

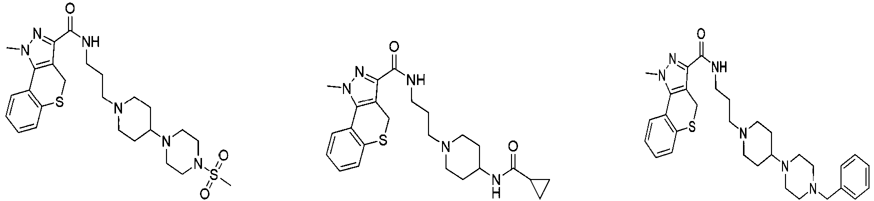

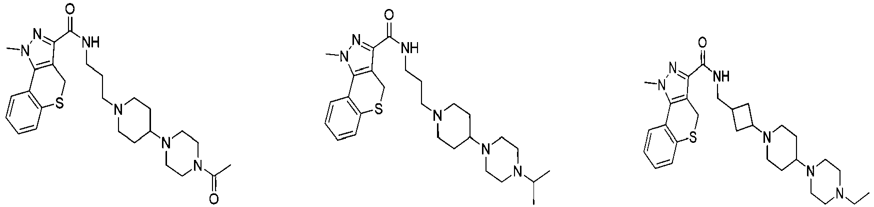

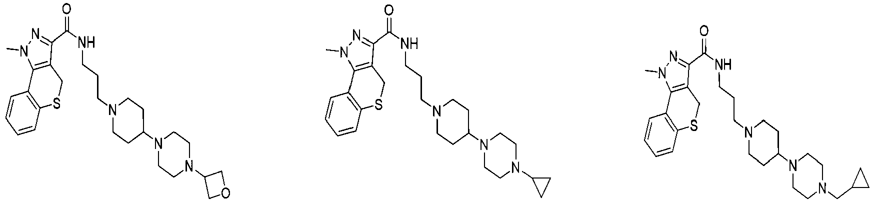

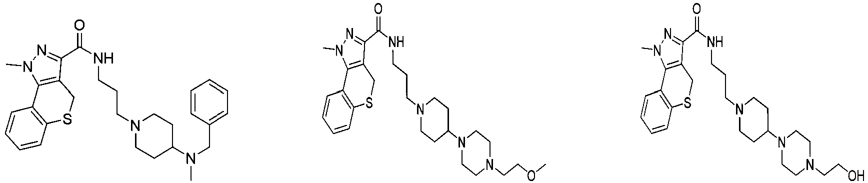

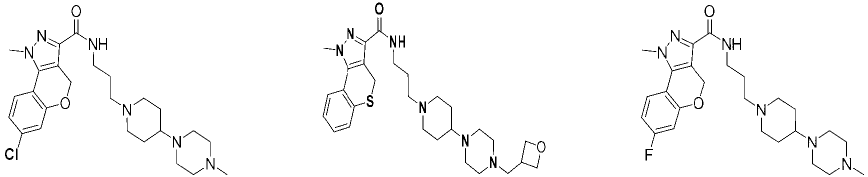

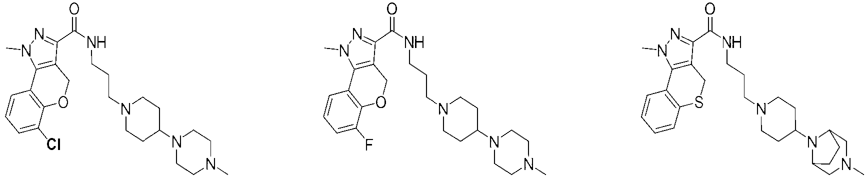

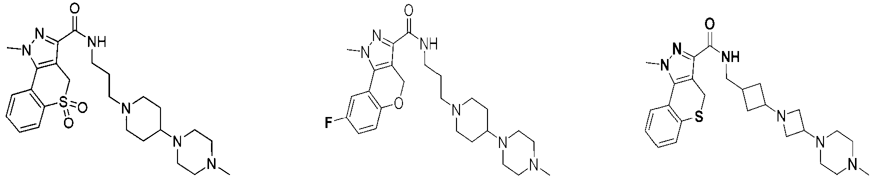

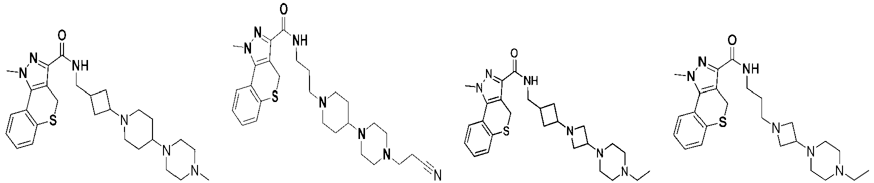

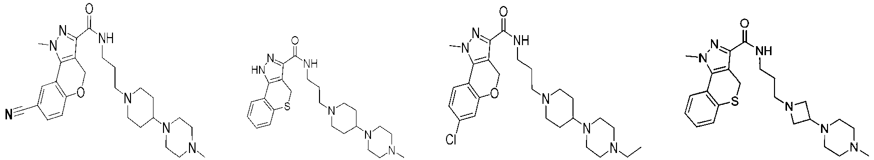

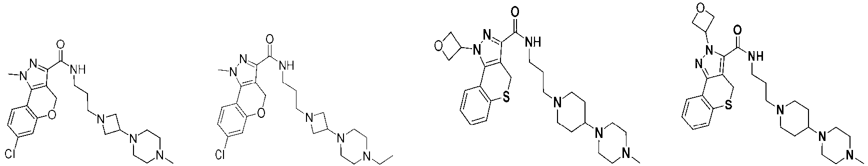

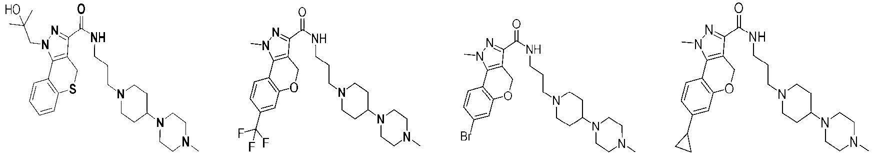

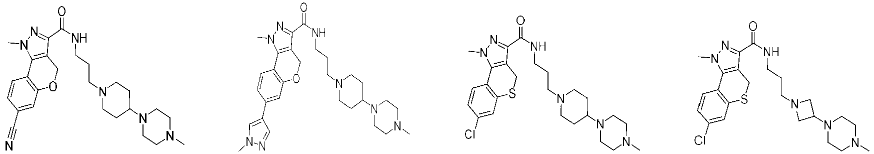

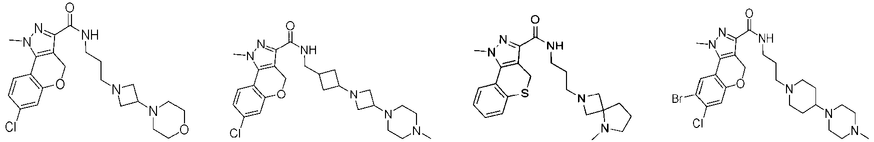

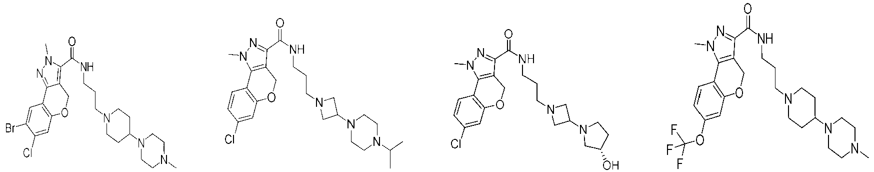

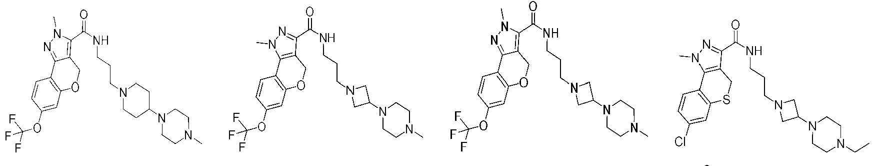

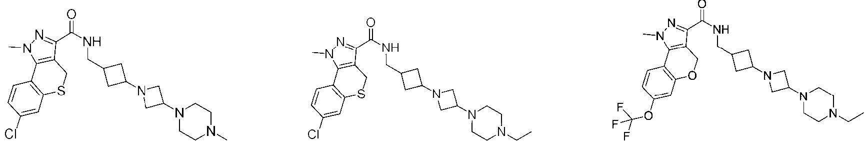

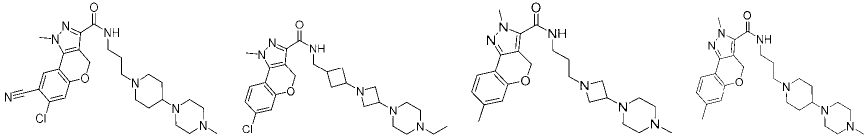

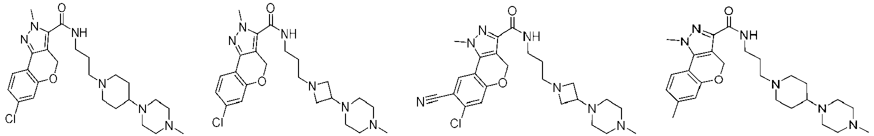

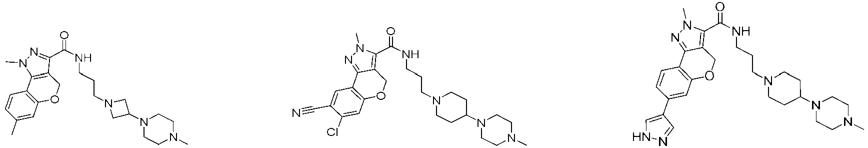

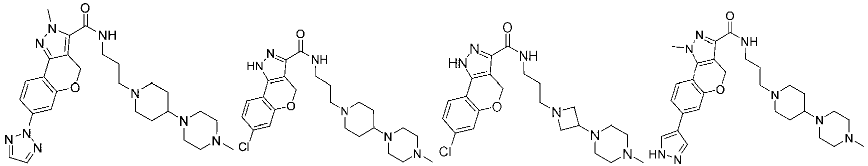

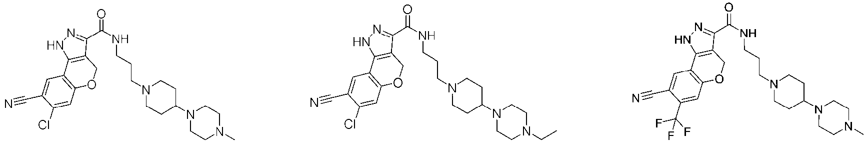

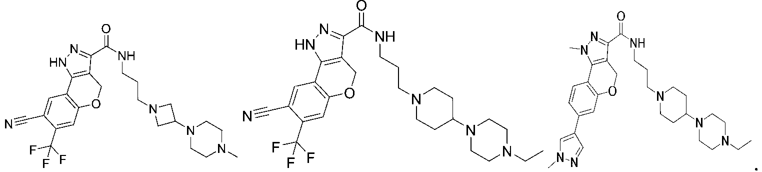

11. A pharmaceutical composition, comprising: a substance A and a pharmaceutically acceptable excipient, wherein the substance A is selected from a group consisting of a compound of Formula (X-A) and a pharmaceutically acceptable salt thereof; 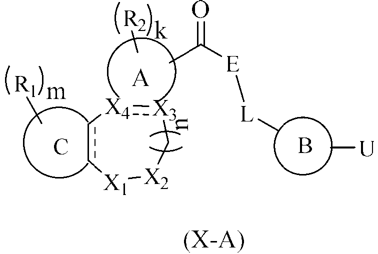
,
wherein ring A, ring B, ring C, X₁, X₂, X₃, X₄, R₁, R₂, E, m, n, k, L, B, and U are as defined according to claim 1; and
preferably, the pharmaceutical composition is used for treating and/or preventing a Kinase Suppressor of Ras 2-Adenosine 5'-Monophosphate-activated Protein Kinase (KSR2-AMPK)-related disease or disorder comprising cancer, wherein the cancer comprises liver cancer.

12. Use of the substance A or the pharmaceutical composition according to claim 11 in preparation of a KSR2-AMPK inhibitor, wherein the KSR2-AMPK inhibitor is used *in vivo* in a mammalian organism; the KSR2-AMPK inhibitor is also used *in vitro* primarily for an experimental purpose; for example, the KSR2-AMPK inhibitor is provided as a standard or a control for comparison, or made into a kit according to conventional methods in the prior art to provide rapid detection of an effect of inhibiting KSR2-AMPK.

13. Use of the substance A or the pharmaceutical composition according to claim 11 in preparation of a drug, wherein the drug is for treating and/or preventing a KSR2-AMPK-related disease or disorder; the substance A is in a therapeutically effective amount; and the KSR2-AMPK-related disease or disorder is preferably a cancer, comprising liver cancer.

14. Use of the substance A or the pharmaceutical composition according to claim 11 in preparation of a drug, wherein the drug is for treating and/or preventing a cancer, comprising liver cancer; and the substance A is in a therapeutically effective amount.
